# EUROPEAN PATENT APPLICATION

(11) **EP 4 520 765 A1**
(43) Date of publication of application: **12.03.2025**
(21) Application number: 23462003.7
(22) Date of filing: 08.09.2023
(51) Int. Cl.: C07J 51/00, C07F 7/18, A61K 9/51, A61K 47/24, C12N 15/88, A61K 9/127

(54) **IONIZABLE CATIONIC LIPIDS INCORPORATING SILICON**

(71) Applicant: AldexChem Kft., 2030 Érd (HU)
(72) Inventor: Répási, József, 2030 Érd (HU); Szilvágyi, Gábor, 2017 Pócsmegyer (HU)
(74) Representative: Danubia Patent & Law Office LLC

(57) **Abstract**

The present invention belongs to the field of biomedicine and drug delivery as well as pest and vector controls.

The invention relates to a novel ionizable cationic lipid family incorporating silicon, which belongs to the trademark LipexSil^{™} 2^{nd} generation lipids, wherein the tail is connected to the headgroup with biodegradable silyl acetal linker. Lipids containing silyl acetal linker(s) are state-of-the-art and are effective as ionizable cationic lipids in the formulation of empty or loaded lipid nanoparticles (LNPs). The novel linkers according to the invention are designed by means of proprietary borane catalysts [WO 2022/129966]. The invention describes the synthesis of the lipids of formula (I), formation and characterization of nanoparticles and biological experiments demonstrating that the lipid nanoparticles prepared with these novel lipids can efficiently deliver their cargo (e.g. RNA, DNA, mRNA, siRNA, dsRNA, pDNA, circular DNA, small biologically active molecules) into the cells.

## Description

### TECHNICAL FIELD

The present invention belongs to the field of biomedicine and drug delivery as well as pest and vector controls.

The invention relates to a novel ionizable cationic lipid family incorporating silicon, which belongs to the trademark LipexSil^{™} 2^{nd} generation lipids, wherein the tail is connected to the headgroup with biodegradable silyl acetal linker. Lipids containing silyl acetal linker(s) are state-of-the-art and are effective as ionizable cationic lipids in the formulation of empty or loaded lipid nanoparticles (LNPs). The novel linkers according to the invention are designed by means of proprietary borane catalysts [WO 2022/129966]. The invention describes the synthesis of the lipids of formula (I), formation and characterization of nanoparticles and biological experiments demonstrating that the lipid nanoparticles prepared with these novel lipids can efficiently deliver their cargo (e.g. RNA, DNA, mRNA, siRNA, dsRNA, pDNA, circular DNA, small biologically active molecules) into the cells.

### BACKGROUND

There are many challenges associated with the transfection of nucleic acids-based agents e.g. messenger RNA, antisense oligonucleotides, ribozymes, plasmids and of small molecules etc. to trigger a desired response in a biological system. There is a need to design and synthesize novel second generation lipids (biodegradable ionizable amine lipids) in order to improve the delivery of biologically active agents, for example DNA, RNA, mRNA and various small molecules to cells.

In the last decade, application fields of nanocarriers have been gradually expanding thanks to the improvement of gene and RNA technology and getting more attention on the lipid-based nanoparticle mediated delivery. These application fields wherein lipid-based nanoparticles are involved: viral infection treatment, gene therapy, cancer therapy, mammalian transient protein expression, pests control, vector control etc.

In the field of medicine those treatments are the most effective which can selectively and directly target affected cells or tissues with the appropriate active pharmaceutical agents. With this in mind, the efficiency of treatment shall be increased, and undesirable toxic side effects shall be avoided or reduced. Loaded lipid nanoparticles (LNPs), which contain lipid components have been proven to be an effective carrier system which can be functionalized to protect and deliver the payload to its targeted site. It is to be emphasized that LNPs formed from ionizable cationic lipids also have proven to be outstanding lipid-based carriers in gene therapy.

LNPs also contain synthetic lipids which may be toxic to human cells and the cytotoxicity of synthetic lipids depends on certain motifs (e.g. type of headgroup, linker) relating to their biodegradability and path of their metabolism in the human body.

In the art few examples can be found demonstrating that silicon atom containing lipids are also suitable components of LNPs [WO 2011134675, WO 2021055835] which are effective in the delivery of DNA, RNA, siRNA and nucleotides etc. Silicon is a carbon isostere in drug research and the incorporation of silicon atom could provide innovative solutions to medicinal chemistry problems. The incorporated silicon atom in the lipids can enhance the lipophilicity thereby improving the membrane permeability. Nevertheless, there is still growing demand in the art of efficient, biodegradable, less toxic delivery platforms, since in the next decade the gene therapy is going to become part of the routine treatments.

### DETAILED DESCRIPTION

The present invention provides a series of novel lipids incorporating silicon having the structure of formula (I), details of synthetic methods, details of LNPs' formation and characterisation as well as toxicity and transfection experiments of loaded LNPs.

In one embodiment, the invention provides an ionizable cationic lipid of formula (I) 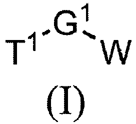 or its salt or stereoisomer,
wherein:
G¹ is
   unsubstituted C₂-C₁₂ alkylene,
   -(CH₂)ₓ-CH=CH-(CH₂)_{y}-, wherein x is an integer selected from 1 to 9, y is an integer selected from 1 to 9, and the sum of x+y is an integer selected from 2 to 10, or
   -(CH₂)_{w}-X-(CH₂)_{z}-, wherein w is an integer selected from 1 to 10, z is an integer selected from 2 to 10, the sum of w+z is an integer selected from 3 to 11, wherein -(CH₂)_{z}- is attached to N, and X is selected from O, S, -S-S-, SO and SO₂;
T¹ is 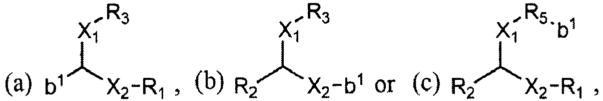
   wherein b¹ is a bond to G¹,
   X₁ and X₂ are the same or different and each independently represents O or S, R₁ is linear C₁-C₃₂ alkyl, branched C₃-C₃₂ alkyl, in both cases the chain optionally containing one S, SO, SO₂, -S-S-, O, or Si(R^{a})₂, wherein R^{a} is C₁-C₆ alkyl, at any position in the carbon chain with the proviso that the heteroatom is not in the alpha or omega position of the carbon chain, linear or branched C₃-C₃₂ alkenyl containing one or more double bonds with the proviso that there is at least one -CH₂- group between the double bond and X₂,
   R₁ is CH₃-(CH₂)_{d}-V₁-(CH₂)ₑ-, wherein d is an integer selected from 1 to 20, e is an integer selected from 1 to 20, and the sum of d+e is an integer selected from 2 to 29, and V₁ is a C₃-C₆ cycloalkylene, or
   R₁ is V₂-(CH₂)_{f}-, wherein f is an integer selected from 1 to 30, and V₂ is a C₃-C₆ cycloalkyl, or
   R₁ is 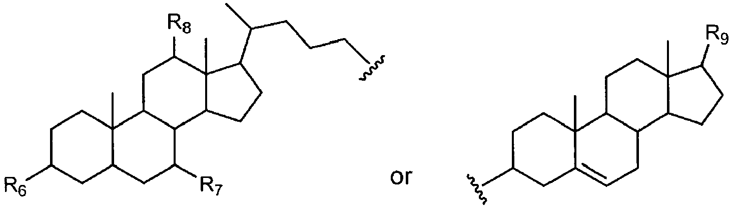
      wherein R₆, R₇, R₈ are the same or different and each is independently H, OH, -C₁-C₆ alkoxy, -O-C(O)-C₁-C₆ alkyl or fluorine,
      R₉ is linear or branched C₃-C₁₂ alkyl or C₃-C₁₂ alkenyl containing one double bond, or
   R₁ is 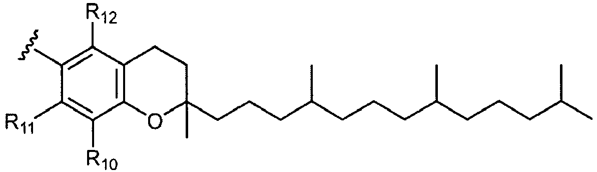 wherein R₁₀, R₁₁, R₁₂ are the same or different and each is independently H, F or methyl, or
   R₁ is 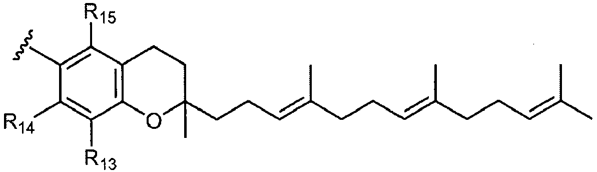 wherein R₁₃, R₁₄, R₁₅ are the same or different and each is independently H, F or methyl, or
   R₁ is 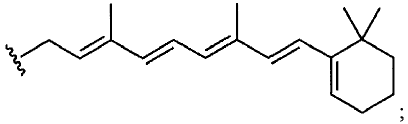
   R₂ is linear C₁-C₃₂ alkyl, branched C₃-C₃₂ alkyl, in both cases optionally containing one S, SO, SO₂, -S-S-, O, or Si(R^{b})₂, wherein R^{b} is C₁-C₆ alkyl, at any position in the carbon chain with the proviso that the heteroatom is not in the alpha or omega position of the carbon chain, linear or branched C₃-C₃₂ alkenyl containing one or more double bonds with the proviso that there is at least one -CH₂- group between the double bond and the carbon linking X₁ and X₂, or
   R₂ is CH₃-(CH₂)_{g}-V₃-(CH₂)ₕ-, wherein g is an integer selected from 1 to 20, h is an integer selected from 1 to 20, and the sum of g+h is an integer selected from 2 to 28, wherein V₃ is a C₃-C₆ cycloalkylene, or
   R₂ is V₄-(CH₂)ₜ-, wherein t is an integer selected from 1 to 29, wherein V₃ is a C₃-C₆ cycloalkyl, or
   R₂ is 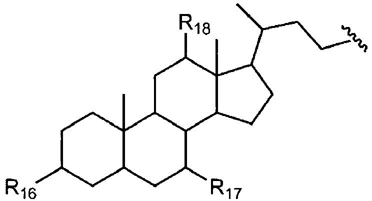 wherein R₁₆, R₁₇, R₁₈ are the same or different and each is independently H, OH, -C₁-C₆ alkoxy, -O-C(O)-C₁-C₆ alkyl or fluorine; or
   R₁ is connected to R₃, wherein R₃ has the meaning as defined above, wherein Y₁, X₃ and b₂ are as defined above and R₄ forms together with R₁ an alkylene or alkenylene containing one double bond, and thus R₁ and R₃ form together with the intervening atoms of (a) a 5-32-membered ring, or
   R₂ is connected to R₃, wherein R₃ has the meaning as defined above, wherein Y₁, X₃ and b₂ are as defined above and R₄ forms together with R₂ an alkylene or alkenylene containing one double bond, and thus R₂ and R₃ form together with the intervening atoms of (b) a 5-32-membered ring, wherein the carbon atom of R₂ at the first or second position numbered from that carbon atom which is attached to the carbon linking X₁ and X₂, can be optionally replaced by O or S heteroatom, or
   R₂ is connected to R₁ and R₂ forms together with R₁ an alkylene or alkenylene containing one double bond and thus R₂ and R₁ form together with the intervening atoms of (c) a 5-32-membered ring, wherein the carbon atom of R₂ at the first or second position numbered from that carbon atom which is attached to the carbon linking X₁ and X₂, can be optionally replaced by O or S heteroatom;
   R₃ is 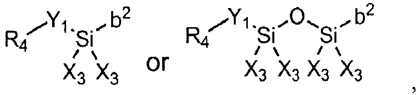
   R₅-b' is 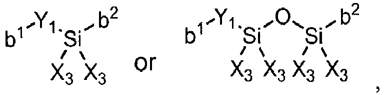 wherein
      b² is a link to X₁,
      Y₁ is -O- or -CH₂- or -O-CH₂-CH₂- wherein -CH₂- is attached to Si,
      each X₃ is independently C₁-C₄ alkyl or C₁-C₄ alkoxy,
      R₄ is linear C₁-C₃₂ alkyl, branched C₃-C₃₂ alkyl, in both cases the chain optionally containing one S, SO, SO₂, -S-S-, O, or Si(R^{c})₂, wherein R^{c} is C₁-C₆ alkyl, at any position in the carbon chain with the proviso that the heteroatom is not in the alpha or omega position of the carbon chain, linear or branched C₃-C₃₂ alkenyl containing one or more double bonds with the proviso that there is at least one -CH₂- group between the double bond and Y₁, or
      R₄is 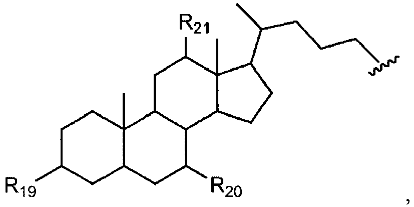 wherein R₁₉. R₂₀, R₂₁ are the same or different and each is independently H, OH, -C₁-C₆ alkoxy, -O-C(O)-C,-C₆ alkyl or fluorine, or
      R₄ is CH₃-(CH₂)_{U}-V₅-(CH₂)_{S}-, wherein u is an integer selected from 1 to 20, s is an integer selected from 1 to 20, and the sum of u+s is an integer selected from 2 to 29, and V₅ is a C₃-C₆ carbocycle, or
      R₄ is V₆-(CH₂)ᵢ-, wherein i is an integer selected from 1 to 30, and V₆ is a C₃-C₆ carbocycle, or
      R₄ is 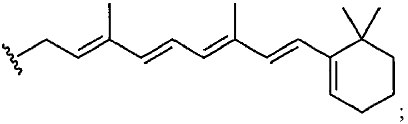
W is 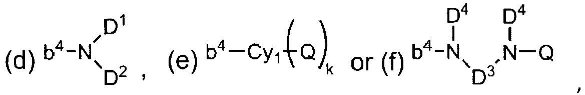 wherein
   b⁴ is a bond to G¹;
   D¹ and D² are independently selected from the following:
      linear C₁-C₈ alkyl, branched C₃-C₈ alkyl, 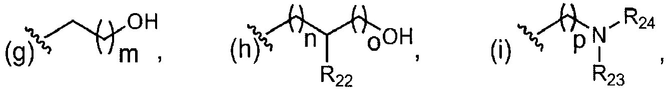 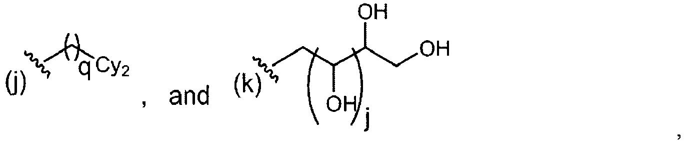
      wherein
         R₂₂ is C₁-C₆ alkyl, cyclopentyl, cyclohexyl, hydroxyl, hydroxymethyl, hydroxyethyl, phenyl, benzyl, 4-hydroxy benzyl, 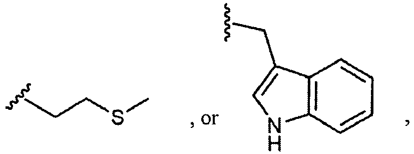
         R₂₃ and R₂₄ are independently selected from H and C₁-C₆ alkyl, or one of R₂₃ and R₂₄ is H and the other forms together with the joint N atom a guanidyl group;
         m is an integer selected from 1 to 6,
         n is an integer selected from 0 to 6,
         o is an integer selected from 0 to 6,
         p is an integer selected from 2 to 6,
         q is an integer selected from 0 to 6,
         j is an integer selected from 1 to 4,
         Cy₂ is a C₃-C₆ cycloalkyl optionally substituted by one or more -OH, or
         Cy₂ is a pyranose, furanose ring, which can be linked via any of its OH group, wherein the pyranose or furanose ring is optionally substituted by a -NH-CO-CH₃ group, adenine, guanine, uracil, cytosine, thymine, a mono- or oligosaccharide, or by a 4-, 5-, 6- or 7-membered heterocyclic ring containing 1, 2, 3 or 4 heteroatoms selected from N, O or S, or
         Cy₂ is a 4-, 5-, 6- or 7-membered heterocyclic ring containing 1, 2, 3 or 4 heteroatoms selected from O, N or S, wherein the heterocyclic ring is optionally substituted by R₂₅, wherein R₂₅ represents C₁-C₆ alkyl, an arginine containing peptide, a pyranose or furanose ring attached by any of their ring-carbon atoms to the 4-, 5-, 6- or 7-membered heterocyclic ring, wherein the pyranose or furanose ring is optionally substituted by a -NH-CO-CH₃ group, adenine, guanine, uracil, cytosine, thymine or a mono- or oligosaccharide,
         R₂₅ can be a group selected from (g) or (h), wherein m, n, o and R₂₂ are as defined above,
            or
         R₂₅ can be a 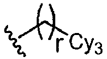 group wherein r is an integer selected from 1 to 4, and Cy₃ is a 4-, 5-, 6- or 7-membered heterocyclic ring containing 1, 2, 3 or 4 heteroatoms selected from O, N and S, optionally substituted by C₁-C₆ alkyl;
   Cy₁ is a 4-, 5-, 6- or 7-membered heterocyclic ring containing at least one N atom which is attached to G¹ via b⁴, and optionally containing 1, 2 or 3 further heteroatoms selected from O, N and S,
   wherein the heterocyclic ring is optionally substituted with Q, which is linked to the heterocyclic ring via any N or C atom of the heterocyclic ring, with the proviso that the N atom which is substituted with Q is not adjacent to that N atom that is attached to G' via b⁴,
   k is an integer selected from 0 and 1;
   Q is T²-G²
   wherein
      G² is defined as G' above, where G¹ and G² may be identical or different;
      T² is defined as T¹ above and wherein T¹ and T² may be identical or different; or
   Q is defined as D¹ above;
   D³ is C₂-C₈ alkylene;
   D⁴ is selected from (g) above, wherein m is defined as above.

By unsubstituted C₂-C₁₂ alkylene we mean ethylene, propylene, butylene, pentylene, hexylene, heptylene, octylene, nonylene, decylene, undecylene, dodecylene.

By C₂-C₈ alkylene we mean ethylene, propylene, butylene, pentylene, hexylene, heptylene, octylene.

By C₁-C₆ alkyl group we mean a linear or branched alkyl group containing 1 to 6 carbon atoms, e.g. methyl, ethyl, propyl, butyl, pentyl, hexyl, isopropyl, isobutyl, *tert*-butyl, neopentyl, isopentyl, neohexyl or isohexyl, preferably methyl or ethyl.

By C₁-C₄ alkyl group we mean a linear or branched alkyl group containing 1 to 4 carbon atoms, preferably methyl.

By linear C₁-C₈ alkyl group we mean a linear alkyl group containing 1 to 8 carbon atoms, preferably methyl.

By branched C₃-C₈ alkyl group we mean a branched alkyl group containing 3 to 8 carbon atoms.

By linear C₁-C₃₂ alkyl, branched C₃-C₃₂ alkyl, in both cases the chain optionally containing one S, SO, SO₂, -S-S-, O, or Si(R)₂, wherein R is C₁-C₆ alkyl, at any position in the carbon chain with the proviso that the is not in the alpha or omega position of the carbon chain, we mean a linear C₁-C₃₂ alkyl or branched C₃-C₃₂ alkyl group containing 1-32 or 3-32 carbon atoms respectively, wherein one -CH₂- of the alkyl chain, that is not in the terminal position at any side of the chain, is optionally replaced by one of the following groups: -O- , -S-, -SS-, -SO-, SO₂-, -Si(R)₂, e.g. one of the following groups in case of R₁: 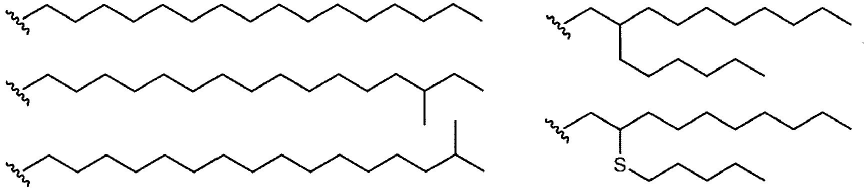 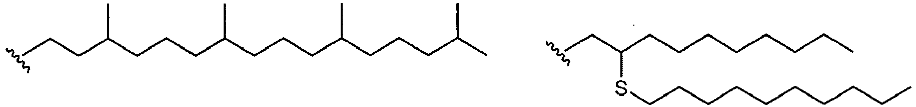 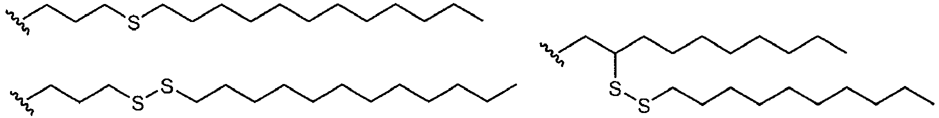 e.g. one of the following groups in case of R₂:

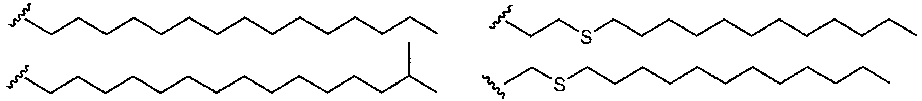

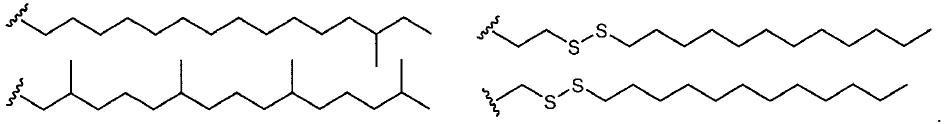

By CH₃-(CH₂)_{d}-V₁-(CH₂)ₑ-, wherein d is an integer selected from 1 to 20, e is an integer selected from 1 to 20, and the sum of d+e is an integer selected from 2 to 29, and V₁ is a C₃-C₆ cycloalkylene, we mean a linear alkyl, which contains in the interchain a 3-, 4-, 5- or 6-membered saturated cycloalkylene, e.g. R₁ is the following group:

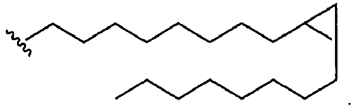

By V₂-(CH₂)ᵣ-, wherein f is an integer selected from 1 to 30, and V₂ is a C₃-C₆ cycloalkyl, we mean a linear alkyl which contains a 3-, 4-, 5- or 6-membered saturated cycloalkyl at the terminal of alkyl chain, e.g. R₁ is the following group:

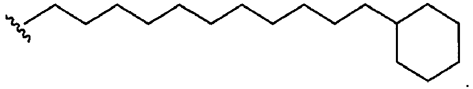

By CH₃-(CH₂)_{g}-V₃-(CH₂)ₕ-, wherein g is an integer selected from 1 to 20, h is an integer selected from 1 to 20, and the sum of g+h is an integer selected from 2 to 28 and V₃ is a C₃-C₆ cycloalkylene, we mean a linear alkyl, wherein -(CH₂)ₕ- is attached to the carbon linking X₁ and X₂ and which contains in the interchain 3-, 4-, 5- or 6-membered saturated cycloalkylene, e.g. R₂ is the following group:

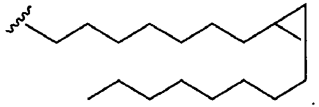

By V₄-(CH₂)ₜ-, wherein t is an integer selected from 1 to 29, and V₃ is a C₃-C₆ cycloalkyl, we mean a linear alkyl wherein -(CH₂)ₜ- is attached to the carbon linking X₁ and X₂ and which contains in the interchain 3-, 4-, 5- or 6-membered saturated cycloalkyl, e.g. R₂ is the following group:

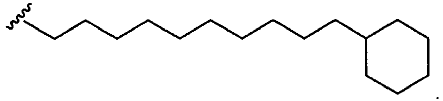

By linear or branched C₃-C₃₂ alkenyl containing one or more double bonds with the proviso that there is at least one -CH₂- group between the double bond and X₂ or the carbon linking X₁ and X₂ or Y₁ respectively we mean a mono- or polyunsaturated linear or branched C₃-C₃₂ alkyl group, in which the -CH₂- group in the terminal position of the alkyl chain that is the link to the other part of the compound is saturated, e.g. one of the following groups in case of R₁: 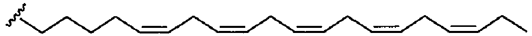 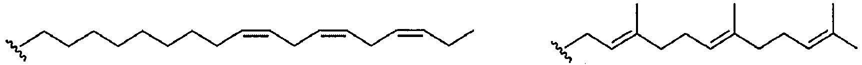 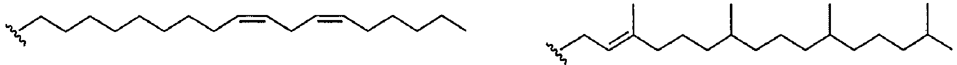 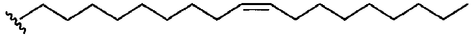 ,
e.g. one of the following groups in case of R₂:

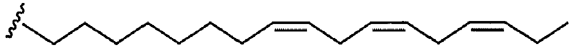

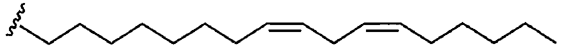

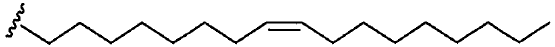

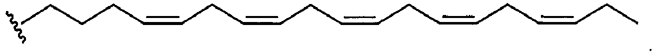

By C₁-C₆ alkoxy we mean -O-C₁-C₆ alkyl group wherein the C₁-C₆ alkyl group is as defined above.

By C₁-C₄ alkoxy we mean -O-C₁-C₄ alkyl group wherein the C₁-C₄ alkyl group is as defined above, like methoxy, ethoxy, propoxy, butoxy, isopropoxy, *sec*-butoxy or *tert*-butoxy.

By C₃-C₆ cycloalkyl we mean cyclopropyl, cyclobutyl, cyclopentyl, cyclohexyl.

By -O-C(O)-C₁-C₆ alkyl we mean C₁-C₆ acylate group e.g.: isobutanoate, 2,2-dimethylpropanoate

By the formula 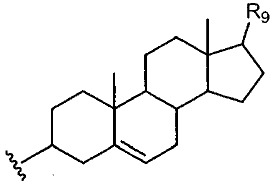 wherein R₉ is linear or branched C₃-C₁₂ alkyl or C₃-C₁₂ alkenyl with one double bond, we mean specifically cholesterol, ergosterol, brassicasterol, avenasterol, sitosterol, campesterol, stigmasterol, isofucosterol.

By R₁ connected to R₃, wherein R₃ has the meaning as defined above, wherein Y₁, X₃ and b₂ are as defined above and R₄ forms together with R₁ an alkylene or alkenylene containing one double bond, and thus R₁ and R₃ form together with the intervening atoms of (a) a 5-32-membered ring we mean e.g.:

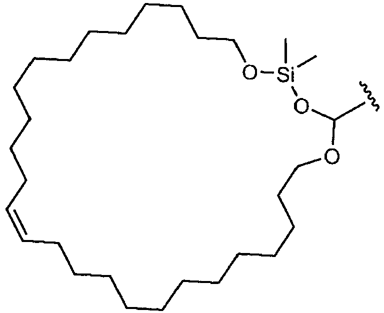

By R₂ connected to R₃, wherein R₃ has the meaning as defined above, wherein Y₁, X₃ and b₂ are as defined above and R₄ forms together with R₂ an alkylene or alkenylene containing one double bond, and thus R₂ and R₃ form together with the intervening atoms of (b) a 5-32-membered ring, wherein the carbon atom of R₂ at the first or second position numbered from that carbon atom which is attached to the carbon linking X₁ and X₂, can be optionally replaced by O or S heteroatom we mean e.g.:

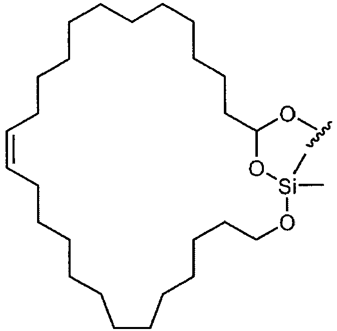

R₂ connected to R₁ and R₂ forms together with R₁ an alkylene or alkenylene containing one double bond and thus R₂ and R₁ form together with the intervening atoms of (c) a 5-32-membered ring, wherein the carbon atom of R₂ at the first or second position numbered from that carbon atom which is attached to the carbon linking X₁ and X₂, can be optionally replaced by O or S heteroatom we mean e.g.:

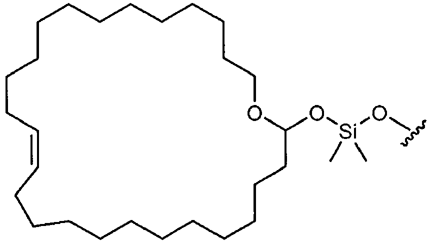

By the group (i) where one of R₂₃ and R₂₄ is H and the other form together with the joint N atom a guanidyl group (i.e. -C(=NH)-NH₂) we mean e.g. the following:

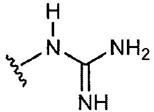

By 4-, 5-, 6- or 7-membered heterocyclic ring containing at least one N atom and optionally containing 1, 2 or 3 further heteroatoms selected from O, N and S, we mean an aromatic ring or an unsaturated, partly saturated or fully saturated heterocyclic ring containing at least 1 N atom, e.g.: azetine, azetidine, pyrrolidine, oxazolidine, piperidine, piperazine, morpholine, pyrrole, imidazole, pyrazole, 1,2,3-triazole, 1,2,4-triazole, tetrazole, pyridine, pyrimidine, pyridazine, pyrazine, 1,2,4-triazine, 1,3,5-triazine, 1,2,4,5 tetrazine, oxazole, isoxazole, thiazole, isothiazole, 1,2-oxazine, 1,3-oxazine, 1,4-oxazine, 1,2-thiazine, 1,3-thiazine, 1,4-thiazine, azepane, azepine, 1,2-diazepane, 1,3-diazepane, 1,4-diazepane, 1,2-diazepine, 1,3-diazepine, 1,4-diazepine, 1,2-oxazepane, 1,3-oxazepane, 1,4-oxazepane, 1,2-oxazepine, 1,3-oxazepine, 1,4-oxazepine, 1,2-thiazepane, 1,3-thiazepane, 1,4-thiazepane, 1,2-thiazepine, 1,3-thiazepine, or 1,4-thiazepine ring, preferably pyrrolidine, imidazole, 1,2,3-triazol, piperidine, piperazine or morpholine.

By a 4-, 5-, 6- or 7-membered heterocyclic ring containing 1, 2, 3 or 4 heteroatoms selected from O, N or S mean an aromatic ring or an unsaturated, partly saturated or fully saturated heterocyclic ring, in addition to the above definition e.g. furane, thiophene, pyrane, thiopyran, oxetane, tetrahydrofuran, dioxane.

By furanose we mean all stereoisomers of ribofuranose, deoxyribofuranose, xylofuranose, fructofuranose, glucofuranose, galactofuranose, mannofuranose, allofuranose, arabinofuranose, or altrofuranose which can be also protected with e.g. acetyl, benzyl, benzoyl, isopropylidene or benzylidene groups. For example: 2,3,5,6-tetra-O-acetyl-galactofuranose.

By pyranose we mean all stereoisomers of glucopyranose, galactopyranose, mannopyranose, allopyranose, fructopyranose, arabinopyranose or altropyranose which can also be protected with e.g. acetyl, benzyl, benzoyl, isopropylidene, or benzylidene groups. For example: 2,3,4,6-tetra-0-acetyl-glucopyranose.

By mono-, oligosaccharide we mean monosaccharide as we defined above for furanose and pyranose and we mean by oligosaccharide e.g. sucrose, lactose, maltose, isomaltulose, cellobiose, trehalose.

By arginine containing peptide we mean a peptide with 6 amino acids or less comprising at least one arginine residue. The peptide is linked by its C or N terminus.

By salts of the compounds of formula (I) we mean salts of the compounds of formula (I) with inorganic or organic acids. Preferred salts are those with pharmaceutically acceptable acids. The salts are e.g. chloride, sulfate, phosphate, formate, acetate, fumarate, maleate, oxalate, citrate or tartrate. The salts formed during purification or isolation are also subject of the invention.

By stereoisomers we mean optical and geometric isomers. The compounds of formula (1) may contain one or more asymmetric carbon atoms thus they can exist in the form of optical isomers, enantiomers or diastereomers. The compounds of formula (I) may contain double bounds and the groups attached to the double bond can have different (cis or trans) confirmations, e.g. cis or trans fatty acid moieties.

A narrower group of compounds of formula (1) is, wherein G' is linear C₂-C₁₂ alkylene, preferably C₄-C₉ alkylene.

An further narrower group of compounds of formula (I) is wherein T¹ is (c) or (a), preferably (c).

A specific embodiment of this group is, wherein R₁ is selected from the following structures:

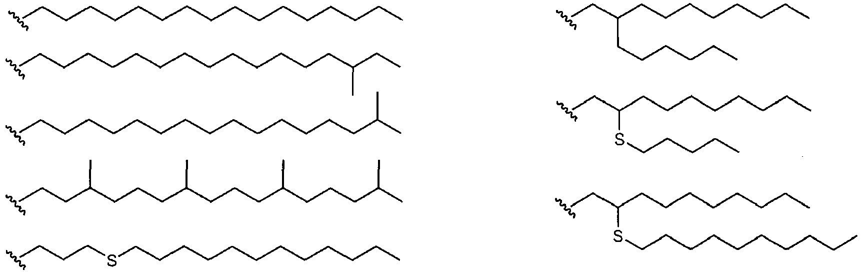

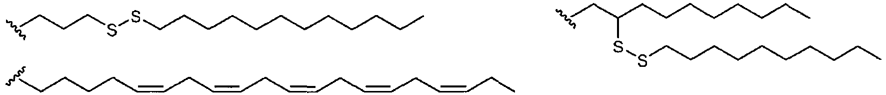

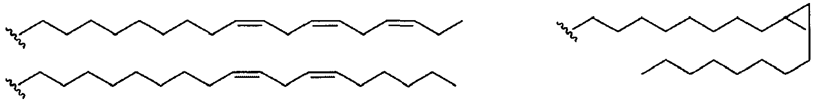

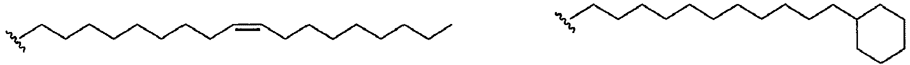

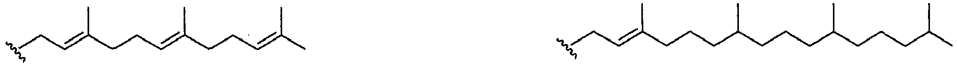

A further specific embodiment of this narrower group is wherein R₁ is selected from the following structures:

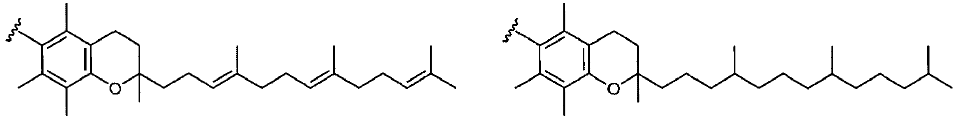

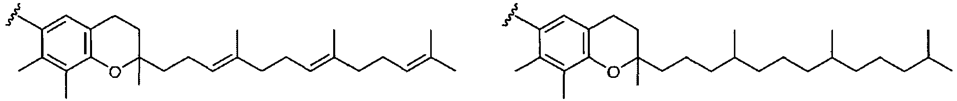

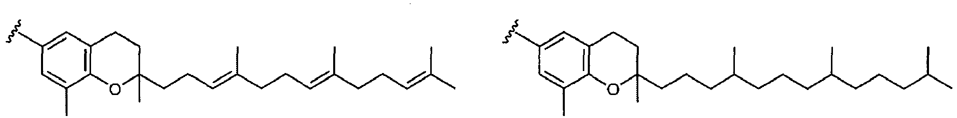

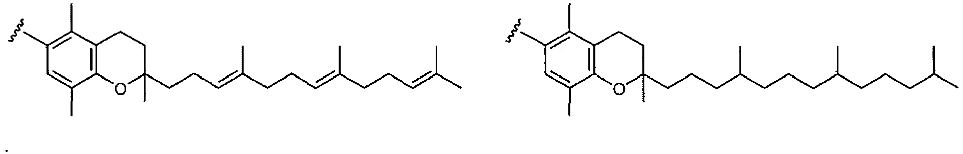

Another specific embodiment of this group is, wherein R₁ is selected from the following structures:

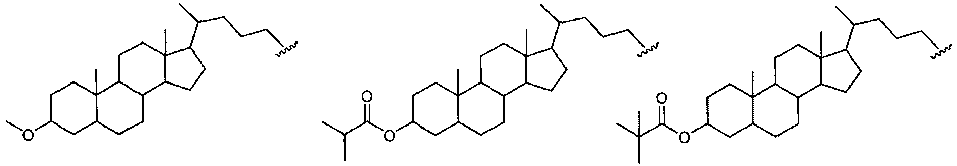

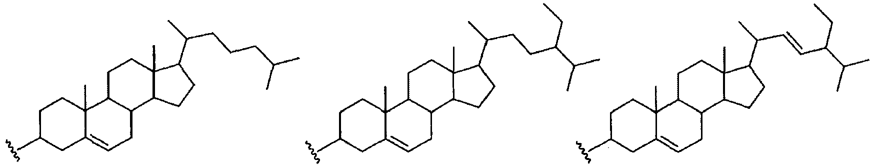

A narrower group of compounds of formula (I) is wherein T¹ is (b) or (c), preferably (c).

A specific embodiment of this group is, wherein R₂ is selected from the following structures:

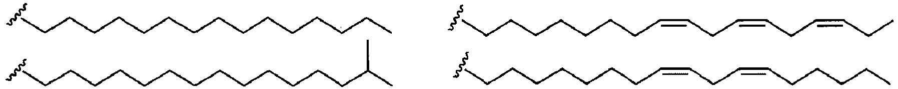

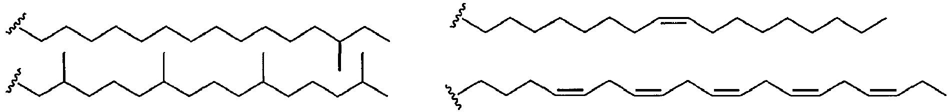

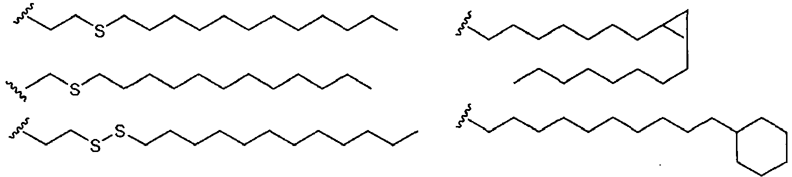

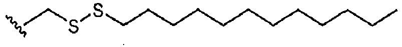

Another specific embodiment of this group is, wherein R₂ is selected from the following structures:

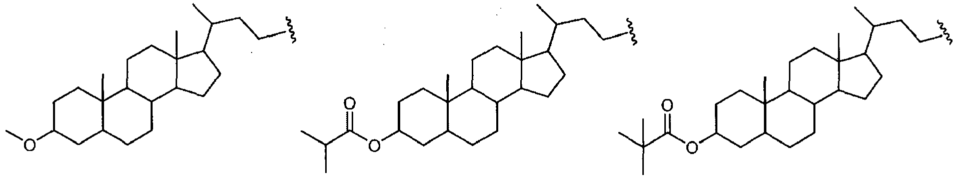

Another specific embodiment of this group is, wherein T¹ is selected from the following structures:

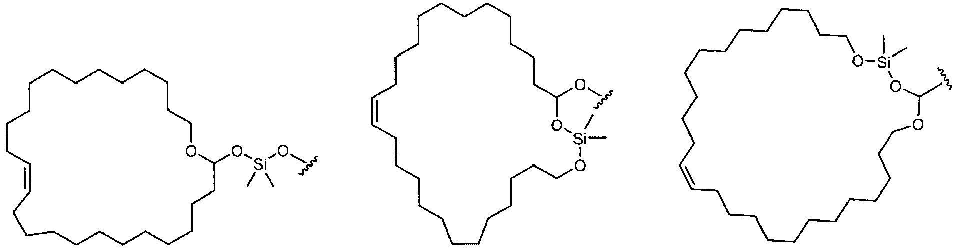

Another group of the compounds of formula (I) is, wherein W is (d).

A specific embodiment of this group is, wherein D¹ and/or D² has one of the following structures:
Methyl, ethyl, propyl, butyl, pentyl, hexyl, *sec-*propyl, *sec*-butyl, *tert*-butyl, neopentyl.

Another specific embodiment of this group is wherein D¹ and D² are the same or different group and D¹, D² are linear C₁-C₈ alkyl and/or branched C₃-C₈ alkyl.

Another specific embodiment of this group is wherein D¹ and D² are the same group and D¹ and D² are selected from any of (g), (h), or (k).

Another specific embodiment of this group is wherein D¹ is selected from any of (h), (i), (j) or (k) and D² is (g).

Another specific embodiment of this group is wherein D¹ is selected from any of (i), (j) or (k) and D² is (h).

Another specific embodiment of this group is D¹ is selected from any of (g), (h), (i), (j) or (k) and D² is linear C₁-C₈ alkyl or branched C₃-C₈ alkyl.

A further specific embodiment of this group is, wherein is, wherein D¹ and/or D² is selected from the following structures:

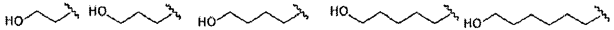

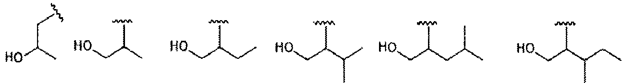

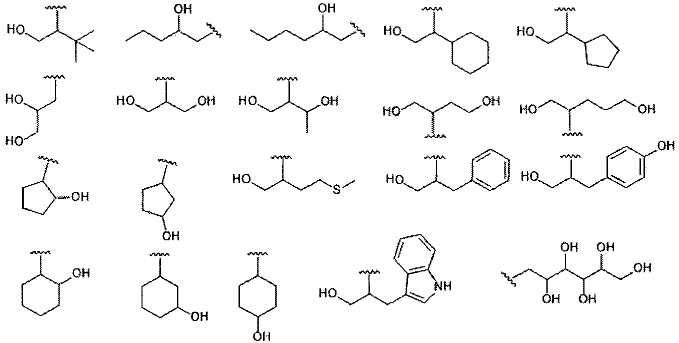

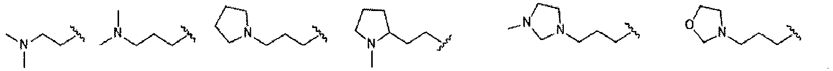

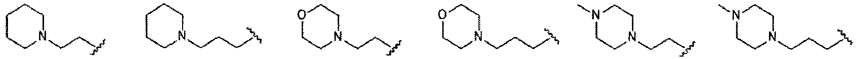

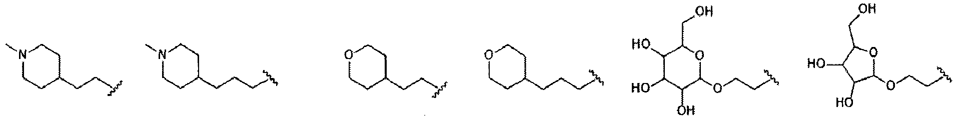

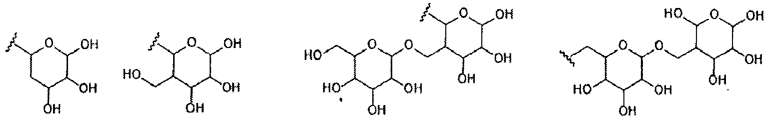

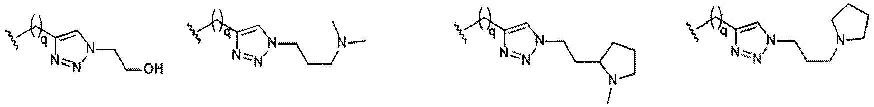

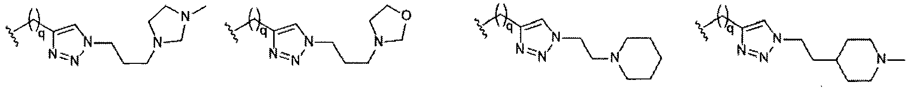

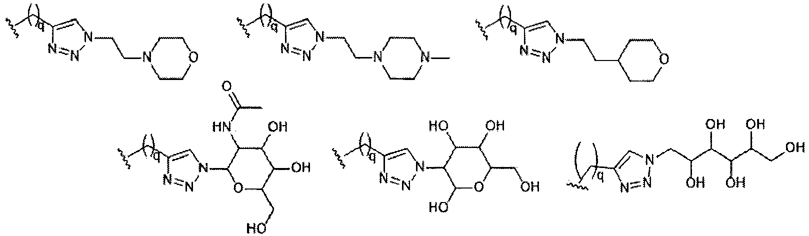

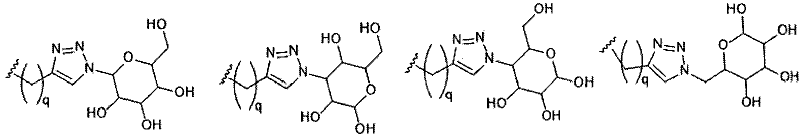

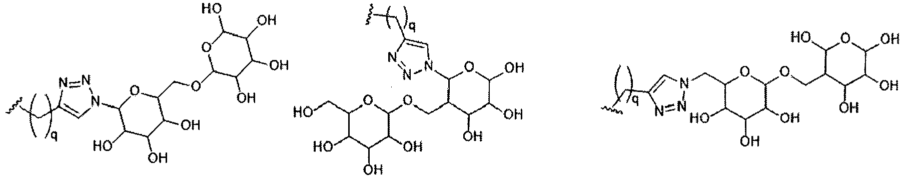

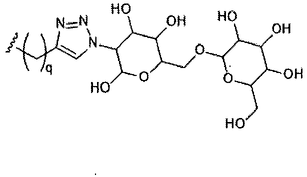

A further specific embodiment of this group is, wherein is, wherein D¹ and/or D² has one of the following structures:

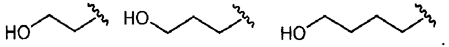

An other narrower group of compounds of formula (1), wherein W is (e).

A specific embodiment of this group is wherein W is selected from the following structures:

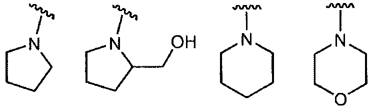

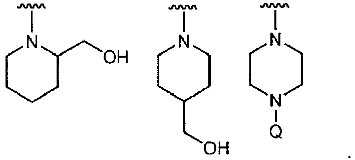

A further narrower group of compounds is wherein W if (f).

A specific embodiment of this group is wherein W is

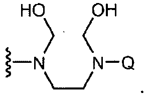

The invention also provides a lipid nanoparticle (LNP) comprising compound of formula (I) as defined above and a therapeutic agent (e.g. RNA, DNA, mRNA, siRNA, dsRNA, pDNA, circular DNA, or small biologically active molecules).

In a particular embodiment the invention provides a lipid nanoparticle comprising compound formula (I) and green fluorescence protein (GFP) mRNA.

The invention also provides a method for delivering GFP mRNA into a cell.

The present invention opens a new chemical space via silicon containing linkers that results in novel biodegradable lipids of formula (I). The mentioned linkers are very difficult to synthesize with the traditional chemical toolbox, but recently published borane catalysts [WO2022/129966] enabled their synthetic feasibility. The mixed acetal building blocks of the present invention were prepared via similar way as described in WO2022/129966. These novel mixed acetals are structurally different from that ones are described in WO2022/129966, moreover further difference is that the mixed acetal moiety is retained and incorporated into the lipids according to the present invention.

The novel intermediates and building blocks used for the synthesis of the compounds of formula (I) are also subject of the present invention. The claimed compounds are structurally different from the compounds disclosed in the prior art.

Another significant feature of present invention is the modularity. Thanks to the nature of the designed silicon-based linker a large number of compounds and diverse lipid library can be designed and synthesized from simple and readily available starting materials.

Additionally, the chemical stability (e.g. sensitivity to hydrolysis) of the silicon containing ionizable lipids can be fine-tuned with the chemical properties of the substituents and with the number of O and Si atoms on the mixed silyl acetal group (linker), which is manifested in the biodegradability of the lipids.

Another aspect of the invention is the preparation of compounds of formula (I) and the intermediates and building blocks used therein.

The compounds of the formula (I) can be prepared according to the following reaction schemes.

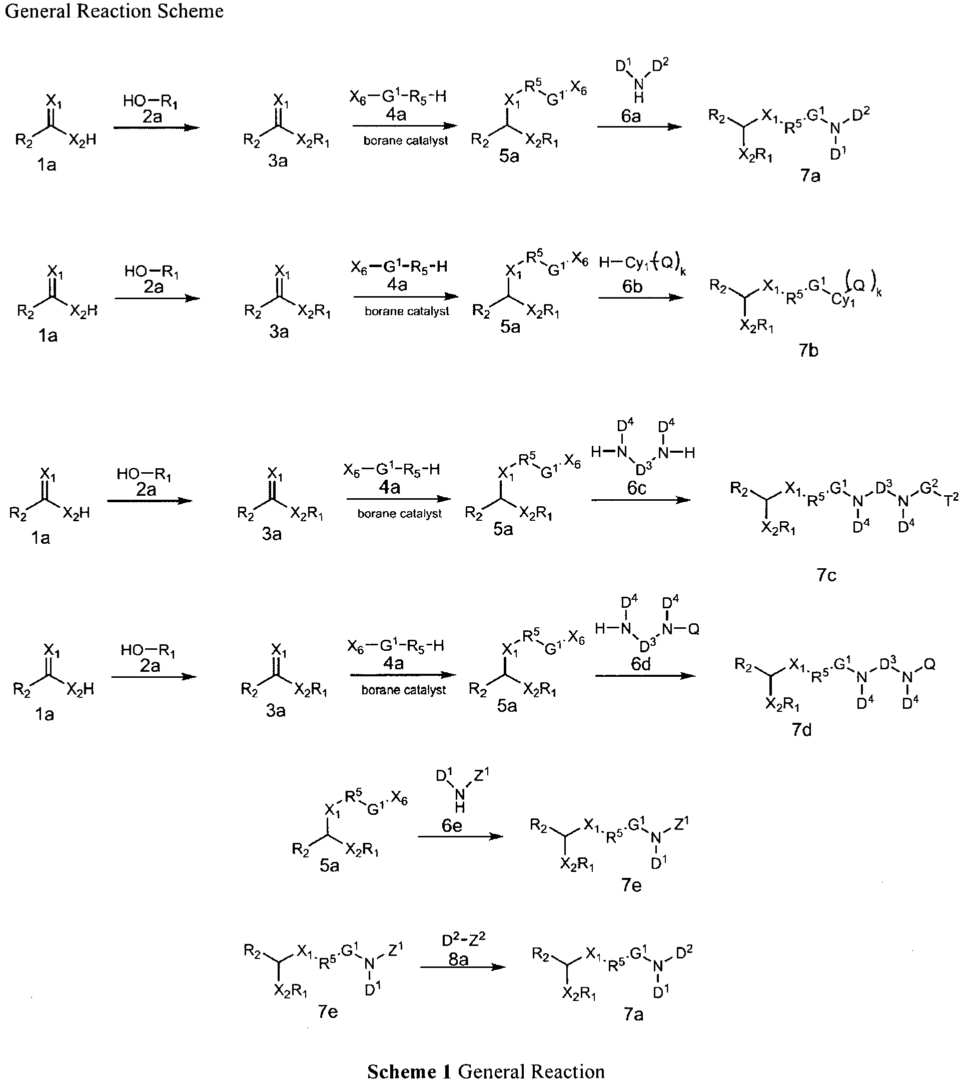

**Scheme 1** illustrates the synthesis of the lipids having the structure of formula (1) wherein X₆ is halogen or pseudohalogen, T¹ is (c) as defined above, G¹, G², T² X₁, X₂, R₁, R₂, R₅, D¹, D², D³, D⁴, Q, Z¹ are as defined above and Z² is N₃ or ethynyl depending on the meaning of Z¹. G¹-T¹, G²-T² can be identical of different. The synthetic strategy is demonstrated through one case wherein T¹ is (c), further cases wherein T¹ is (a) or (b) as defined above can be easily derived from this by analogy to the earlier application PCT/EP2023/070748. Compounds of structure 1a, 2a, 3a, 4a, 6a, 6b, 6c, 6d, 6e, 8a can be purchased or prepared according to methods known in the art. Reaction of the carboxylic acid derivative 1a with alcohol 2a under appropriate esterification conditions (e.g. oxalyl chloride, Et₃N) yields ester 3a which can be transformed to mixed silyl acetal 5a with the appropriate silane derivative 4a and borane catalyst [borane catalysts are disclosed in WO2022/129966]. The resulting mixed silyl acetal 5a which corresponds to the formula (III) that contains halogen or pseudohalogen atom is reacted with the amine derivative 6a, 6b, 6c, 6d under appropriate alkylation conditions (e.g. KI, K₂CO₃ in CPME/MeCN) to yield a compound of 7a or 7b or 7c or 7d respectively. In that case if the amine contains Z¹ substituent the intermediate can be synthesized under appropriate alkylation conditions (e.g. KI, K₂CO₃ in CPME/MeCN) to yield 7e which can be transformed with 8a under "click" reaction condition (e.g. azidosugar, CuBr, PMDT in DMF) to the desired product 7a.

### Brief Description of the Drawings

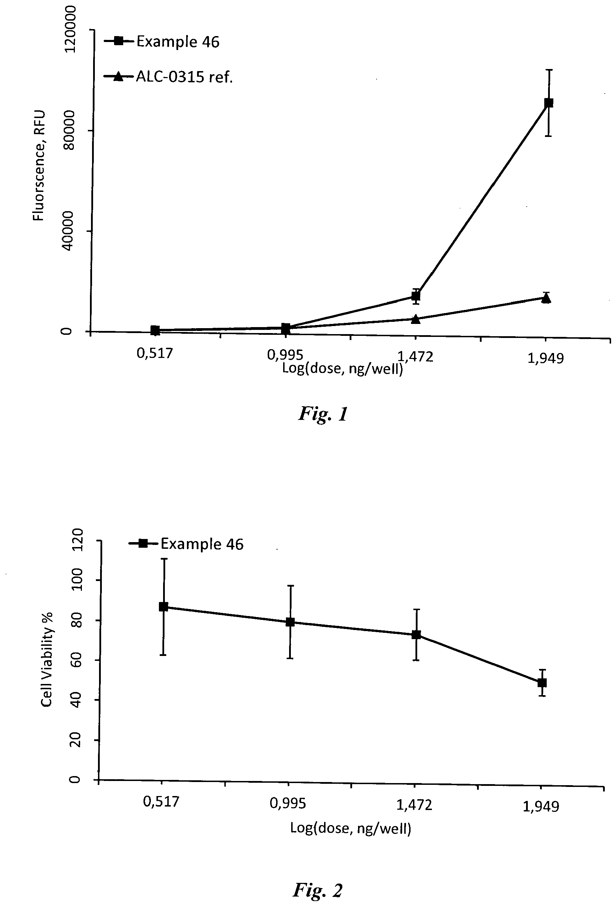

**Figure 1****:** Transfection efficacy of **Example 46 (■)** and **ALC-0315 ref. (▲)** at different doses of GFP mRNA (3.3-89 ng mRNA/well)
**Figure 2****:** Cell viability of **Example 46 (■)** at different doses of GFP mRNA (3.3-89 ng mRNA/well)

### Purification Method A

Eluent A: EtOAc, eluent B: petroleum ether +1% Et₃N; full gradient A:B 0:100 -> 100:0. Sample loading ~200 mg; column size: 25 g of SiO₂. Column conditioned with ~400 mL of B prior loading the sample.

### Purification Method B

Eluent A: DCM:MeOH:aq. NH₃ 80:20:1, eluent B: DCM; full gradient A:B 0:100 -> 100:0. Sample loading ∼200 mg; column size: 25 g of SiOz. Column conditioned with ~400 mL of A and then with ~400 mL of B prior loading the sample.

### Purification Method C

Eluent A: MeOH, eluent B: water; full gradient A:B 0:100 -> 100:0. Sample loading ∼100 mg; column: Biotage SNAP KP-C18-HS 12g.

### General Procedure A

A three neck round bottom flask was equipped with dropping funnel, drying tube (with CaCl₂) and thermometer and charged with 1.25 eq. oxalyl chloride and abs. dichloromethane (concentration: 1.0 mmol oxalyl chloride in 0.41 mL abs. dichloromethane). The solution in the flask was cooled down to 0°C and 1.0 eq. acid was added dropwise. The reaction mixture was left to warm to room temperature and stirred for overnight. After this time, it was concentrated under reduced pressure to give an oil which was used in the ester synthesis without further purification.

A three neck round bottom flask was equipped with dropping funnel, drying tube (with CaCl₂) and thermometer and charged with 1.0 eq. alcohol, 1.2 eq. triethylamine and dichloromethane (concentration: 1.0 mmol alcohol in 4 ml dichloromethane). The mixture in the flask was cooled down to 0°C and the dichloromethane solution of 1.0 eq. acyl chloride (concentration: 1.0 mmol acyl chloride in 1.0 ml dichloromethane) was added dropwise. The reaction mixture was left to warm to room temperature and stirred for overnight. The reaction was monitored by TLC (hexane/ethyl acetate 9/1). After overnight the volatiles were removed under reduced pressure and filtered the triethylamine hydrochloride salt. The filtrate was evaporated onto silica gel under reduced pressure, and it was purified by flash column chromatography.

### General Procedure B

A two neck round bottom flask was equipped with septum, drying tube (with CaCl₂) and charged with carboxylic acid (1.1 eq.), alcohol (1.0 eq.) and abs. dichloromethane (concentration: 1.0 mmol carboxylic acid in 3.0 mL abs. dichloromethane). To the solution, 2.0 eq. HBTU was added and followed by the addition of 5.0 eq. TEA. After that, the reaction mixture was stirred for overnight at room temperature. Then reaction mixture was quenched with NaHCO₃ solution and the layers were separated, the organic phase was washed with water and dried over Na₂SO₄ and concentrated. The crude material was purified by flash column chromatography (0-50 % EtOAc in petroleum ether).

### General Procedure C

A flame dried three necked round bottom flask was purged with the stream of argon. The flask was equipped with septum, thermometer and argon balloon and charged with the mixture of abs. toluene (1.0 ml toluene/1.0 mmol ester) and ester. To the reaction mixture the catalyst solution was added in one portion, followed by the dropwise addition of silane, maintaining the internal temperature between 20-30°C. The reaction mixture was stirred for overnight. The reaction was monitored by TLC (hexane/ethyl acetate 9/1, PMA visualization and if different, it is specified in the example). After reaction completion 1 ml EtOAc, 0.6 ml MeCN and silica gel were added to the reaction mixture and stirred for further 10 mins and filtered off the silica gel and washed with 2×25 ml n-pentane. Volatiles were removed under reduced pressure to obtain the crude product that was purified by flash column chromatography or simple filtration via a short pad of silica to give the product.

### General Procedure D

A MW reaction vial was charged with ω-bromo alkyl silyl mixed acetal, amine, abs. acetonitrile (1.0 mmol ω-bromo alkyl silyl mixed acetal/4.0 ml of abs. acetonitrile) and abs. cyclopentyl methyl ether (ω-bromo alkyl silyl mixed acetal/1.33 ml of abs. cyclopentyl methyl ether). To the stirred reaction mixture K₂CO₃ (2.0 eq.) and KI (0.2 eq.) were added and purged with argon and sealed. The resulting mixture was stirred for 18 hours at 62°C. Then the reaction mixture was cooled down to room temperature and partitioned between ethyl acetate and brine and stirred vigorously for 15 minutes. The organic phase was separated and dried over Na₂SO₄, filtered and concentrated. The residue was purified by flash column chromatography

### General Procedure E

A MW reaction vial was charged with 1.0 eq. propargyl amine derivative, 1.5 eq. azide derivative, 0.33 eq. CuBr, 0.33 eq. 1,1,4,7,7-Pentamethyldiethylenetriamine and abs. DMF (0.1 mmol propargyl amine derivative/3.0 ml of abs. DMF) and purged with argon and sealed. The resulting mixture was stirred for 20 h at 50°C. Then the reaction mixture was concentrated to dryness under vacuum and the residue was purified by flash column chromatography.

### Examples of the ester synthesis

### Example 1

### Synthesis of (9Z,12Z)-octadeca-9,12-dien-1-yl 6-bromohexanoate

(9Z,12Z)-octadeca-9,12-dien-1-yl 6-bromohexanoate was prepared from 6-bromohexanoic acid and linoleyl alcohol according to general procedure **A.** The product was obtained as a yellowish oil: 1.25 g, 2.81 mmol, 100% yield.
¹H NMR (500 MHz, CDCl₃) δ 5.42-5.29 (m, 4H), δ 4.06 (t, 2H), δ 3.40 (t, 2H), δ 2.77 (m, 2H), δ 2.31 (t, 2H), δ 2.10-2.00 (q, 4H), δ 1.93-1.82 (m, 2H), δ 1.72-1.55 (m, 4H), δ 1.53-1.42 (m, 2H), δ 1.41-1.22 (br.m, 16H), δ 0.89 (t, 3H)

### Example 2

### Synthesis of 6-bromohexyl (9Z,12Z)-octadeca-9,12-dienoate

6-bromohexyl (9Z,12Z)-octadeca-9,12-dienoate was prepared from linoleic acid and 6-bromohexan-l-ol according to general procedure **A.** The product was obtained as a yellowish oil: 1.49 g, 3.35 mmol, 94 % yield.
¹H NMR (500 MHz, CDCl₃) δ 5.42-5.29 (m, 4H), δ 4.06 (t, 2H), δ 3.40 (t, 2H), δ 2.77 (m, 2H), δ 2.29 (t, 2H), δ 2.04 (m, 4H), δ 1.91-1.83 (m, 2H), δ 1.69-1.56 (m, 4H), δ 1.51-1.24 (br. m, 18H), δ 0.89 (t, 3H)

### Example 3

### Synthesis of (Z)-octadec-9-en-1-yl palmitate

(Z)-octadec-9-en-1-yl palmitate was prepared from palmitic acid and oleyl alcohol according to general procedure **A.** The product was obtained as a yellowish oil: 2.77 g, 3.80 mmol, 70 % yield.
¹H NMR (500 MHz, CDCl₃) δ 5.35-5.33 (m, 2H), δ 4.05 (t, 2H), δ 2.28 (t, 2H), δ 2.05-1.99 (m, 4H), δ 1.65-1.56 (m, 4H), δ 1.38-1.21 (br. m, 46H), δ 0.87 (t, 6H)

### Example 4

### Synthesis of hexadecyl oleate

Hexadecyl oleate was prepared from oleic acid and palmityl alcohol according to general procedure A. The product was obtained as a yellowish oil: 0.85 g, 1.68 mmol, 50 % yield.
¹H NMR (500 MHz, CDCl₃) δ 5.39-5.32 (m, 2H), δ 4.05 (t, 2H), δ 2.28 (t, 2H), δ 2.02-1.98 (m, 4H), δ 1.63-1.58 (m, 4H), δ 1.38-1.21 (br. m, 46H), δ 0.88 (t, 6H)

### Example 5

### Synthesis of hexadecyl palmitate

Hexadecyl oleate was prepared from palmitic acid and hexadecanol according to general procedure **A.** The product was obtained as a yellowish oil: 1.37 g, 2.85 mmol, 50 % yield.
¹H NMR (500 MHz, CDCl₃) δ 4.05 (t, 2H), δ 2.30-2.27 (m, 2H), δ 1.66-1.59 (m, 4H), δ 1.38-1.23 (m, 50H), δ 0.88 (t, 6H)

### Example 6

### Synthesis of (9Z,12Z)-octadeca-9,12-dien-1-yl palmitate

(9Z,12Z)-octadeca-9,12-dien-1-yl palmitate was prepared from palmitic acid and linoleyl alcohol according to general procedure **A.** The product was obtained as a yellowish oil: 1.72 g, 3.41 mmol, 62 % yield.
¹H NMR (500 MHz, CDCl₃) δ 5.41-5.28 (m, 4H), δ 4.04 (t, 2H), δ 2.76 (t, 2H), δ 2.27 (t, 2H), δ 2.07-2.02 (m, 4H), δ 1.66-1.57 (m, 4H), δ 1.40-1.19 (br. m, 40H), δ 0.91-0.85 (m, 6H)

### Example 7

### Synthesis of hexadecyl (9Z, 12Z)-octadeca-9, 12-dienoate

Hexadecyl (9Z,12Z)-octadeca-9,12-dienoate was prepared from linoleic acid and palmityl alcohol according to general procedure **A.** The product was obtained as a yellowish oil: 1.59 g, 3.15 mmol, 52 % yield.
'H NMR (500 MHz, CDCl₃) δ 5.41-5.31 (m, 4H), δ 4.04 (t, 2H), δ 2.77 (t, 2H), δ 2.28 (t, 2H), δ 2.07-2.02 (m, 4H), δ 1.65-1.59 (m, 4H), δ 1.40-1.19 (br. m, 40H), δ 0.91-0.85 (m, 6H)

### Example 8

Synthesis of (Z)-octadec-9-en-1-yl (9Z,12Z)-octadeca-9,12-dienoate was prepared from linoleic acid and oleyl alcohol according to general procedure **A.** The product was obtained as a yellowish oil: 2.06 g, 3.873 mmol, 79 % yield.
¹H NMR (500 MHz, CDCl₃) δ 5.43-5.29 (m, 6H), δ 4.05 (t, 2H), δ 2.77 (m, 2H), δ 2.29 (t, 2H), δ 2.07-1.97 (m, 8H), δ 1.65-1.58 (m, 4H), δ 1.42-1.21 (br. m, 36H), δ 0.91-0.86 (m, 6H)

### Example 9

### Synthesis of (9Z, 12Z)-octadeca-9, 12-dien-l-yl oleate

(9Z,12Z)-octadeca-9,12-dien-1-yl oleate was prepared from oleic acid and linoleyl alcohol according to general procedure **A.** The product was obtained as a yellowish oil: 1.47 g, 2.77 mmol, 83 % yield.
¹H NMR (500 MHz, CDCl₃) δ 5.42-5.30 (m, 6H), δ 4.05 (t, 2H), δ 2.77 (m, 2H), δ 2.29 (t, 2H), δ 2.10-1.96 (m, 8H), δ 1.65-1.58 (m, 4H), δ 1.41-1.22 (br. m, 36H), δ 0.91-0.86 (m, 6H)

### Example 10

### Synthesis of (Z)-octadec-9-en-1-yl oleate

(Z)-octadec-9-en-1-yl oleate was prepared from oleic acid and oleyl alcohol according to general procedure **A.** The product was obtained as a yellowish oil: 0.62 g, 1.16 mmol, 35 % yield.
¹H NMR (500 MHz, Benzene-d₆) δ 5.30-5.20 (m, 4H), δ 3.95 (t, 2H), δ 2.19 (t, 2H), δ 1.94-1.89 (m, 8H), δ 1.53-1.49 (m, 4H), δ 1.26-1.13 (br. m, 42H), δ 0.80-0.76 (t, 6H)

### Example 11

### Synthesis of (9Z,12Z)-octadeca-9,12-dien-1-yl (9Z,12Z)-octadeca-9,12-dienoate

(9Z,12Z)-octadeca-9,12-dien-1-yl (9Z,12Z)-octadeca-9,12-dienoate was prepared from linoleic acid and linoleyl alcohol according to general procedure **A.** The product was obtained as a yellowish oil: 3.53 g, 6.67 mmol, 71 % yield.
¹H NMR (500 MHz, CDCl₃) δ 5.42-5.29 (m, 8H), δ 4.05 (t, 2H), δ 2.77 (m, 4H), δ 2.29 (t, 2H), δ 2.06 (m, 8H), δ 1.66-1.56 (m, 4H), δ 1.40-1.24 (br. m, 30H), δ 0.89 (t, 6H)

### Example 12

### Synthesis of 2-hexyldecyl oleate

2-hexyldecyl oleate was prepared from oleic acid and 2-hexyldecyl alcohol according to general procedure **A.** The product was obtained as a yellowish oil: 2.53 g, 2.12 mmol, 42 % yield.
¹H NMR (500 MHz, CDCl₃) δ 5.38-5.30 (m, 2H), δ 3.97 (d, 2H), δ 2.29 (t, 2H), δ 2.05-1.98 (m, 4H), δ 1.65-1.58 (m, 3H), δ 1.36-1.21 (br. m, 44H), δ 0.88 (t, 9H)

### Example 13

### Synthesis of (Z)-octadec-9-en-1-yl (4R)-4-((3R,10S,13R)-3-methoxy-10,13-dimethylhexadecahydro-1H-cyclopenta[a]phenanthren-17-yl)pentanoate

(Z)-octadec-9-en-1-yl (4R)-4-((3R,10S,13R)-3-methoxy-10,13-dimethylhexadecahydro- 1H-cyclopenta[a]phenanthren-17-yl)pentanoate was prepared from (4R)-4-((3R,10S,13R)-3-methoxy-10,13-dimethylhexadecahydro-1H-cyclopenta[a]phenanthren-17-yl)pentanoic acid and oleyl alcohol according to general procedure **A.** The product was obtained as a yellowish oil: 1.43 g, 2.24 mmol, 75 % yield.
¹H NMR (500 MHz, CDCl₃) δ 5.38-5.32 (m, 2H), δ 4.04 (t, 2H), δ 3.34 (s, 3H), δ 3.19-3.14 (m, 1H), δ 2.37-2.31 (m, 1H), δ 2.25-2.14 (m, 1H), δ 2.04-1.50 (br.m, 16H), δ 1.47-0.98 (br.m, 38H), δ 0.94-0.83 (br. m, 9H), δ 0.60 (s, 3H)

### Example 14

### Synthesis of (9Z,12Z)-octadeca-9,12-dien-1-yl (4R)-4-((3R,10S, 13R)-3-methoxy-10,13-dimethylhexadecahydro-1H-cyclopenta[a]phenanthren-17-yl)pentanoate

(9Z,12Z)-octadeca-9,12-dien-1-yl (4R)-4-((3R,10S,13R)-3-methoxy-10,13-dimethylhexadecahydro-1H-cyclopenta[a]phenanthren-17-yl)pentanoate was prepared from (4R)-4-((3R,10S,13R)-3-methoxy-10,13-dimethylhexadecahydro-1H-cyclopenta(a]phenanthren-17-yl)pentanoic acid and linoleyl alcohol according to general procedure **A.** The product was obtained as a yellowish oil: 0.40 g, 0.626 mmol, 38 % yield.
¹H NMR (500 MHz, Benzene-d₆) δ 5.56-5.44 (t, 4H), δ 4.11 (t, 2H), δ 3.26 (s, 3H), δ 3.05 (m, 1H), δ 2.90 (t, 2H), δ 2.42-2.32 (m, 1H), δ 2.31-2.23 (m, 1H), δ 2.12-2.06 (m, 4H), δ 1.88-1.72 (m, 6H), δ 1.56-1.10 (br. m, 30H), δ 1.08-0.71 (br. m, 17H) δ 0.59 (s, 3H)

### Example 15

### Synthesis of (Z)-octadec-9-en-1-yl (4R)-4-((3R,10S,13R)-3-hydroxy-10,13-dimethylhexadecahydro-1H-cyclopenta[a]phenanthren-17-yl)pentanoate

(Z)-octadec-9-en-1-yl (4R)-4-((3R,10S,13R)-3-hydroxy-10,13-dimethylhexadecahydro-1H-cyclopenta[a]phenanthren-17-yl)pentanoate was prepared from (4R)-4-((3R,10S,13R)-3-hydroxy-10,13-dimethylhexadecahydro-1H-cyclopenta[a]phenanthren-17-yl)pentanoic acid and oleyl alcohol according to general procedure **B.** The product was obtained as a yellowish oil: 1.60 g, 2.55 mmol, 90 % yield.
¹H NMR (500 MHz, CDCl₃) δ 5.38-5.30 (m, 2H), δ 4.05 (t, 2H), δ 3.66-3.58 (m, 1H), δ 2.37-2.30 (m, 1H), δ 2.24-2.16 (m, 1H), δ 2.03-1.93 (m,4H), δ 2.03-1.93 (m, 4H), δ 1.89-1.71 (m, 5H), δ 1.68-1.52 (m, 6H), δ 1.45-1.20 (br. m, 30H), δ 1.16-1.01 (m, 6H), δ 0.91-0.85 (m, 9H), δ 0.63 (s, 3H)

### Example 16

### Synthesis of (Z)-octadec-9-en-1-yl (4R)-4-((3R,10S,13R)-10,13-dimethyl-3-(pivaloyloxy)hexadecahydro-1H-cyclopenta[a]phenanthren-17-yl)pentanoate

(Z)-octadec-9-en-1-yl (4R)-4-((3R,10S,13R)-10,13-dimethyl-3-(pivaloyloxy)hexadecahydro- 1H-cyclopenta[a]phenanthren-17-yl)pentanoate was prepared from pivaloic acid and (Z)-octadec-9-en-1-yl (4R)-4-((3R,10S,13R)-3-hydroxy-10,13-dimethylhexadecahydro-1H-cyclopenta[a]phenanthren-17-yl)pentanoate according to general procedure **A.** The product was obtained as a yellowish oil: 1.74 g, 2.45 mmol, 48 % yield.
¹H NMR (500 MHz, CDCl₃) δ 5.38-5.32 (m, 2H), δ 4.72-4.65 (m, 1H), δ 4.05 (t, 2H), δ 2.37-2.29 (m, 1H), δ 2.24-2.14 (m, 1H), δ 2.05-1.95 (m, 4H), δ 1.90-1.75 (m, 5H), δ 1.66-0.98 (br. m, 54H), δ 0.93-0.86 (m, 9H), δ 0.64 (s, 3H),

### Example 17

### Synthesis of (9Z,12Z)-octadeca-9,12-dien-1-yl (4R)-4-((3R,10S,13R)-3-hydroxy-10,13-dimethylhexadecahydro-1H-cyclopenta[a]phenanthren-17-yl)pentanoate

(9Z,12Z)-octadeca-9,12-dien-1-yl (4R)-4-((3R, 1 0S,13R)-3-hydroxy-10,13-dimethylhexadecahydro-1H-cyclopenta[a]phenanthren-17-yl)pentanoate was prepared from (4R)-4-((3R,10S,13R)-3-hydroxy-10,13-dimethylhexadecahydro-1H-cyclopenta[a]phenanthren-17-yl)pentanoic acid and linoleyl alcohol according to general procedure **B.** The product was obtained as a yellowish oil: 0.98 g, 1.58 mmol, 64 % yield.
¹H NMR (500 MHz, CDCl₃) δ 5.42-5.29 (m, 4H), δ 4.05 (t, 2H), δ 3.66-3.58 (m, 1H), δ 2.79-2.75 (m, 2H), δ 2.39-2.29 (m, 1H), δ 2.26-2.15 (m, 1H), δ 1.97-0.96 (br. m, 48H), δ 0.91-0.87 (m, 9H), δ 0.61 (s, 3H)

### Example 18

### Synthesis of (9Z,12Z)-octadeca-9,12-dien-1-yl (4R)-4-((3R,10S,13R)-10,13-dimethyl-3-(pivaloyloxy)hexadecahydro-1H-cyclopenta[a]phenanthren-l 7-yl)pentanoate

(9Z,12Z)-octadeca-9,12-dien-1-yl (4R)-4-((3R,10S,13R)-10,13-dimethyl-3-(pivaloyloxy)hexadecahydro-1H-cyclopenta[a]phenanthren-17-yl)pentanoate was prepared from pivaloic acid and (9Z, 12Z)-octadeca-9,12-dien-1-yl (4R)-4-((3R,10S,13R)-3-hydroxy-10,13-dimethylhexadecahydro-1H-cyclopenta[a]phenanthren-17-yl)pentanoate according to general procedure **A.** The product was obtained as a yellowish oil: 0.99 g, 1.39 mmol, 89 % yield.
¹H NMR (500 MHz, CDCl₃) δ 5.42-5.29 (m, 4H), δ 4.73-4.61 (m, 1H), δ 4.05 (t, 2H), δ 2.77 (t, 2H), δ 2.37-2.30 (m, 1H), δ 2.24-2.16 (m, 1H), δ 2.07-2.02 (m, 4H), δ 1.98-0.99 (br. m, 53H), δ 0.99-0.87 (m, 9H), δ 0.64 (s, 3H)

### Examples of the silane synthesis

### Example 19

### Synthesis of ((6-bromohexyl)oxy)dimethylsilane

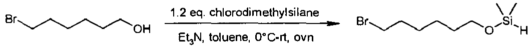

A 250 ml three neck round bottom flask was purged with the stream of argon. The flask was equipped with dropping funnel, thermometer, argon balloon and charged with 6-bromo-1-hexanol (8.00 g, 44.20 mmol), triethylamine (6.70 g, 66.30 mmol) and 44.2 ml abs. toluene. The reaction mixture was cooled down to 0°C and chlorodimethylsilane (5.02 g, 53.04 mmol) was added dropwise maintained the inner temperature between 0°C-5°C. After addition of the silane the reaction was stirred overnight at room temperature. Then the reaction mixture was filtered through on a pad of celite and washed with 2×15 ml pentane and the precipitated triethylamine hydrochloride salt was filtered off. The filtrate was concentrated under reduced pressure. The crude product was purified by vacuum distillation at 0.02-0.05 mbar (63°C-64°C) to give the product as a colourless oil, ((6-bromohexyl)oxy)dimethylsilane (7.27 g, 30.39 mmol, 63 % yield).
¹H NMR (500 MHz, Benzene-d₆) δ 4.85 (m, 1H), δ 3.47 (t, 2H), δ 2.90 (t, 2H), δ 1.45 (m, 2H), δ 1.37 (m, 2H), δ 1.11 (m, 4H) δ 0.14 (d, 6H)

### Example 20

### Synthesis of dimethyl(((9Z, 12Z)-octadeca-9,12-dien- 1 -yl)oxy)silane

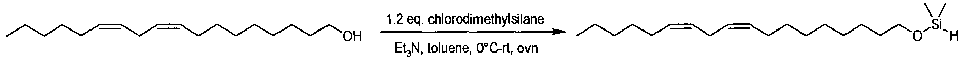

A 50 ml three neck round bottom flask was purged with the stream of argon. The flask was equipped with dropping funnel, thermometer, argon balloon and charged with linoleyl alcohol (2.00 g, 7.50 mmol, 1.0 eq.), triethylamine (1.37 g, 13.51 mmol, 1.8 eq.) and 12 ml abs. toluene. The reaction mixture was cooled down to 0°C and chlorodimethylsilane (994.2 mg, 10.51 mmol, 1.40 eq.) was added dropwise maintained the inner temperature between 0°C-5°C. After addition of the silane the reaction was stirred overnight at room temperature. Then the reaction mixture was concentrated to dryness and resuspended with 20 ml hexane the precipitated triethylamine hydrochloride salt was filtered off and the filtrate was concentrated under reduced pressure to give the product as a colourless oil, dimethyl(((9Z,12Z)-octadeca-9,12-dien-1-yl)oxy)silane (2.43 g, 7.50 mmol, 100 % yield).
¹H NMR (500 MHz, CDCl₃) δ 5.43-5.30 (m, 4H), δ 4.62 (m, 1H), δ 3.63 (t, 2H), δ 2.78 (m, 2H), δ 2.07 (m, 4H), δ 1.61-1.52 (m, 2H), δ 1.41-1.24 (m, 16H), δ 0.9 (t, 3H), δ 0.22 (d, 6H)

### Examples of the mixed silyl acetal synthesis

### Example 21

### Synthesis of (6Z,9Z,32Z,35Z)-22-(5-bromopentyl)-20,20-dimethyl-19,21,23-trioxa-20-silahentetraconta-6,9,32,35-tetraene

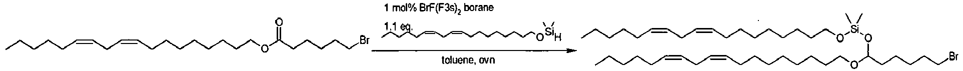

(6Z,9Z,32Z,35Z)-22-(5-bromopentyl)-20,20-dimethyl-19,21,23-trioxa-20-silahentetraconta-6,9,32,35-tetraene was prepared from (9Z,12Z)-octadeca-9,12-dien-1-yl 6-bromohexanoate (1.40 g, 3.157 mmol, 1.0 eq.) and dimethyl(((9Z,12Z)-octadeca-9,12-dien-1-yl)oxy)silane (1.127 g, 3.473 mmol, 1.1 eq.) using 100 µlBrF(F3s)₂ borane catalyst solution (14.11 mg was dissolved in 100 µl abs. toluene) according to **General Procedure C.** The reaction was completed after overnight stirring. The crude product that was purified by flash column chromatography with gradient elution (0-30 % EtOAc in petroleum ether) yielding a transparent oil: 2.20 g, 2.861 mmol, 91 % yield.
¹H NMR (500 MHz, CDCl₃) δ 5.42-5.29 (m, 8H), δ 4.80 (m, 1H), δ 3.70-3.63 (m, 3H), δ 3.40 (m, 2H), δ 3.34-3.28 (m, 1H) δ 2.79-2.76 (m, 4H), δ 2.07-2.02 (m, 8H), δ 1.91-1.83 (m, 2H), δ 1.69-1.24 (m, 42H), δ 0.89 (t, 6H), δ 0.16 (d, 6H)

### Example 22

### Synthesis of (20Z,23Z)-1-bromo-8-((8Z,11Z)-heptadeca-8,11-dien-1-yl)-10,10-dimethyl-7,9,11-trioxa-10-silanonacosa-20,23-diene

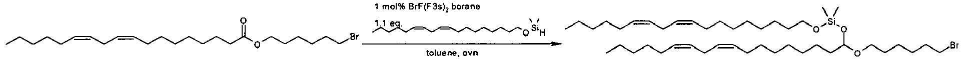

(20Z,23Z)-1-bromo-8-((8Z,11Z)-heptadeca-8,11-dien-1-yl)-10,10-dimethyl-7,9,11-trioxa-10-silanonacosa-20,23-diene was prepared from 6-bromohexyl (9Z,12Z)-octadeca-9,12-dienoate (1.49 g, 3.353 mmol, 1.0 eq.) and dimethyl(((9Z,12Z)-octadeca-9,12-dien-1-yl)oxy)silane (1.20 g, 3.688 mmol, 1.1 eq.) using 100 _{II}I BrF(F3s)₂ borane catalyst solution (14.99 mg was dissolved in 100 µl abs. toluene) according to **General Procedure C.** The reaction was completed after overnight stirring. The crude product that was purified by flash column chromatography with gradient elution (0-30 % EtOAc in petroleum ether) yielding a transparent oil: 2.330 g, 3.03 mmol, 90 % yield.
¹H NMR (500 MHz, CDCl₃) δ 5.42-5.29 (m, 8H), δ 4.80 (m, 1H), δ 3.73-3.63 (m, 3H), δ 3.40 (m, 2H), δ 3.36-3.29 (m, 1H) δ 2.79-2.76 (m, 4H), δ 2.07-2.02 (m, 8H), δ 1.90-1.83 (m, 2H), δ 1.68-1.19 (m, 42H), δ 0.89 (m, 6H), δ 0.16 (d, 6H)

### Example 23

### Synthesis of (Z)-1-bromo-8,8-dimethyl-10-pentadecyl-7,9,11-trioxa-8-silanonacos-20-ene

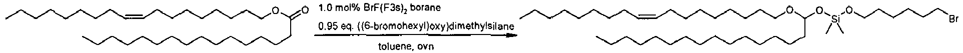

(Z)-1-bromo-8,8-dimethyl-10-pentadecyl-7,9,11-trioxa-8-silanonacos-20-ene was prepared from (Z)-octadec-9-en-1-ylpalmitate (1.92 g, 3.79 mmol, 1.0 eq.) and ((6-bromohexyl)oxy)dimethylsilane (860.8 mg, 3.60 mmol, 0.95 eq.) using 100 _{II}I BrF(F3s)₂ borane catalyst solution (16.93 mg was dissolved in 100 _{II}I abs. toluene) according to **General Procedure C.** The reaction was completed after overnight stirring. The crude product that was purified by flash column chromatography (30 % DCM in petroleum ether with 2 % TEA) yielding a transparent oil: 1.25 g, 0.84 mmol, 22 % yield.
¹H NMR (500 MHz, Benzene-d₆) δ 5.51-5.44 (m, 2H), δ 4.96 (m, 1H), δ 3.85-3.80 (m, 1H), δ 3.68 (t, 2H), δ 3.41-3.37 (m, 1H), δ 2.98 (t, 2H), δ 2.14-2.08 (m, 4H) δ 1.90-1.77 (m, 2H), δ 1.73-1.12 (br.m, 58H), δ 0.94-0.90 (m, 6H), δ 0.33 (s, 6H)

### Example 24

### Synthesis of (Z)-i-bromo-10-(heptadec-8-en-1-yl)-8,8-dimethyl-7,9,11-trioxa-8-silaheptacosane

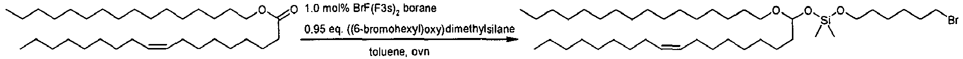

(Z)-1-bromo-10-(heptadec-8-en-1-yl)-8,8-dimethyl-7,9,11-trioxa-8-silaheptacosane was prepared from hexadecyl oleate (840 mg, 1.65 mmol, 1.0 eq.) and ((6-bromohexyl)oxy)dimethylsilane (376.6 mg, 1.57 mmol, 0.95 eq.) using 100 µl BrF(F3s)z borane catalyst solution (741 mg was dissolved in 100 _{II}I abs. toluene) according to **General Procedure C.** The reaction was completed after overnight stirring. The crude product that was purified by flash column chromatography (30 % EtOAc in petroleum ether with 2 % TEA) yielding a transparent oil: 1.15 g, 1.18 mmol, 71 % yield.
¹H NMR (500 MHz, Benzene-d₆) δ 5.55-548 (m, 2H), δ 5.00-4.97 (m, 1H), δ 3.85-3.80 (m, 1H), δ 3.68 (t, 2H), δ 3.45-3.38 (m, 1H), δ 2.98 (t, 2H), δ 2.14-2.08 (m, 4H) δ 1.96-1.75 (m, 2H), δ 1.73-1.12 (br.m, 58H), δ 0.94-0.90 (m, 6H), δ 0.27 (d, 6H)

### Example 25

### Synthesis of (20Z,23Z)-1-bromo-8,8-dimethyl-10-pentadecyl-7,9,11-trioxa-8-silanonacosa-20,23-diene

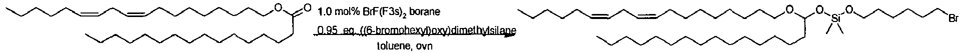

(20Z,23Z)-1-bromo-8,8-dimethyl-10-pentadecyl-7,9,1 1-trioxa-8-silanonacosa-20,23-diene was prepared from (9Z,12Z)-octadeca-9,12-dien-1-yl palmitate (1.71 g, 3.39 mmol, 1.0 eq.) and ((6-bromohexyl)oxy)dimethylsilane (769.7 mg, 3.22 mmol, 0.95 eq.) using 100 µl BrF(F3s)₂ borane catalyst solution (15.14 mg was dissolved in 100 µl abs. toluene) according to **General Procedure C.** The reaction was completed after overnight stirring. The crude product that was purified by flash column chromatography (30 % EtOAc in petroleum ether with 2 % TEA) yielding a transparent oil: 2.42 g, 2.44 mmol, 72 % yield.
¹H NMR (500 MHz, Benzene-d₆) δ 5.54-5.42 (m, 4H), δ 4.99-4.96 (m, 1H), δ 3.86-3.80 (m, 1H), δ 3.67 (t, 2H), δ 3.43-3.37 (m, 1H), δ 2.97 (t, 2H), δ 2.88 (t, 2H), δ 2.12-2.06 (m, 4H), δ 1.92-1.76 (m, 2H), δ 1.70-1.14 (br.m, 52H), δ 0.93-0.87 (m, 6H), δ 0.25 (d, 6H)

### Example 26

### Synthesis of 1-bromo-10-((8Z,11Z)-heptadeca-8,11-dien-1-yl)-8,8-dimethyl-7,9,11-trioxa-8-silaheptacosane

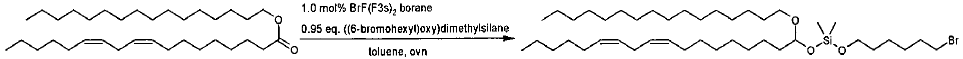

1-bromo-10-((8Z,11Z)-heptadeca-8,11-dien-1 -yl)-8,8-dimethyl-7,9,1 1-trioxa-8-silaheptacosane was prepared from hexadecyl (9Z,12Z)-octadeca-9,12-dienoate (1.59 g, 3.15 mmol, 1.0 eq.) and ((6-bromohexyl)oxy)dimethylsilane (715.7 mg, 2.99 mmol, 0.95 eq.) using 100 µl BrF(F3s)₂ borane catalyst solution (14.08 mg was dissolved in 100 µl abs. toluene) according to **General Procedure C.** The reaction was completed after overnight stirring. The crude product that was purified by flash column chromatography (30 % EtOAc in petroleum ether with 2 % TEA) yielding a transparent oil: 1.51 g, 1.93 mmol, 61 % yield.
¹H NMR (500 MHz, Benzen-d₆) δ 5.55-5.45 (m, 4H), δ 5.00-4.98 (m, 1H), δ 3.89-3.83 (m, 1H), δ 3.69 (t, 2H), δ 3.45-3.39 (m, 1H), δ 2.97 (t, 2H), δ 2.89 (t, 2H), δ 2.11-2.06 (m, 4H), δ 1.94-1.77 (m, 2H), δ 1.62-1.17 (br.m, 52H), δ 0.94-0.88 (m, 6H), δ 0.27 (d, 6H)

### Example 27

### Synthesis of (Z)-1-bromo-10-((8Z,11Z)-heptadeca-8,11-dien-1-yl)-8,8-dimethyl-7,9,11-trioxa-8-silanonacos-20-ene

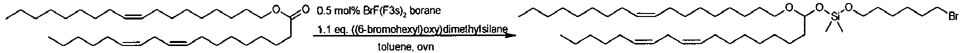

(Z)-1-bromo-10-((8Z,11Z)-heptadeca-8,11-dien-1-yl)-8,8-dimethyl-7,9,11-trioxa-8-silanonacos-20-ene was prepared from (Z)-octadec-9-en-l-yl (9Z,12Z)-octadeca-9,12-dienoate (1.00 g, 1.884 mmol, 1.0 eq.) and ((6-bromohexyl)oxy)dimethylsilane (450.7 mg, 1.884 mmol, 1.0 eq.) using 100 µl BrF(F3s)₂ borane catalyst solution (4.21 mg was dissolved in 100 µl abs. toluene) according to **General Procedure C.** The reaction was completed after overnight stirring. The crude product that was purified by flash column chromatography with gradient elution (0-30 % EtOAc in n-hexane) yielding a transparent oil: 1.42 g, 1.844 mmol, 98 % yield.
¹H NMR (500 MHz, CDCl₃) δ 5.42-5.29 (m, 6H), δ 4.79 (m, 1H), δ 3.68 (t, 3H), δ 3.40 (t, 2H), δ 3.34-3.28 (m, 1H) δ 2.77 (m, 2H), δ 2.10-1.94 (m, 8H), δ 1.90-1.83 (m, 2H), δ 1.68-1.21 (m, 48H), δ 0.89 (m, 6H), δ 0.16 (d, 6H)

### Example 28

### Synthesis of (20Z,23Z)-1-bromo-10-((Z)-heptadec-8-en-1-yl)-8,8-dimethyl-7,9,11-trioxa-8-silanonacosa-20,23-diene

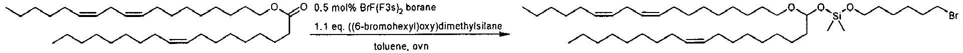

(Z)-1-bromo-10-((8Z,11Z)-heptadeca-8,11-dien-1-yl)-8,8-dimethyl-7,9,11-trioxa-8-silanonacos-20-ene was prepared from (9Z,12Z)-octadeca-9,12-dien-1-yl oleate (1.47 g, 2.773 mmol, 1.0 eq.) and ((6-bromohexyl)oxy)dimethylsilane ( 663.4 mg, 2.773 mmol, 1.0 eq.) using 100 µl BrF(F3s)₂ borane catalyst solution (6.2 mg was dissolved in 100 µl abs. toluene) according to **General Procedure C.** The reaction was completed after overnight stirring. The crude product that was purified by flash column chromatography with gradient elution (0-30 % EtOAc in n-hexane) yielding a transparent oil: 2.13 g, 2.77 mmol, 100 % yield.
¹H NMR (500 MHz, CDCl₃) δ 5.42-5.29 (m, 6H), δ 4.79 (m, 1H), δ 3.68 (t, 3H), δ 3.40 (t, 2H), δ 3.38-3.29 (m, 1H) δ 2.78 (m, 2H), δ 2.10-1.94 (m, 8H), δ 1.90-1.83 (m, 2H), δ 1.68-1.21 (m, 48H), δ 0.89 (m, 6H), δ 0.16 (d, 6H)

### Example 29

### Synthesis of (Z)-1-bromo-10-((Z)-heptadec-8-en-1-yl)-8,8-dimethyl-7,9,11-trioxa-8-silanonacos-20-ene

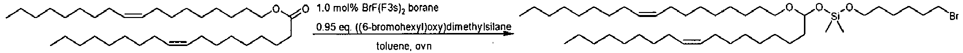

(Z)-1-bromo-10-((Z)-heptadec-8-en-1-yl)-8,8-dimethyl-7,9,1 1-trioxa-8-silanonacos-20-ene was prepared from (Z)-octadec-9-en-l-yl oleate (610 mg, 1.145 mmol, 1.0 eq.) and ((6-bromohexyl)oxy)dimethylsilane (260.13 mg, 1.087 mmol, 0.95 eq.) using 100 µl BrF(F3s)₂ borane catalyst solution (5.12 mg was dissolved in 100 µl abs. toluene) according to **General Procedure C.** The reaction was completed after overnight stirring. The crude product that was purified by flash column chromatography (30 % EtOAc in petroleum ether with 2 % TEA) yielding a transparent oil: 0.85 g, 0.86 mmol, 75 % yield.
¹H NMR (500 MHz, CDCl₃) δ 5.39-5.31 (m, 4H), δ 4.80 (m, 1H), δ 3.70-3.65 (m, 3H), δ 3.41-3.39 (m, 2H), δ 3.35-3.29 (m, 1H), δ 2.03-1.99 (m, 8H), δ 1.91-1.87 (m, 2H), δ 1.67-1.20 (m, 54H), δ 0.88 (m, 6H), δ 0.16 (d, 6H)

### Example 30

### Synthesis of (20Z,23Z)-1-bromo-10-((8Z,11Z)-heptadeca-8,11-dien-1-yl)-8,8-dimethyl-7,9,11-trioxa-8-silanonacosa-20,23-diene

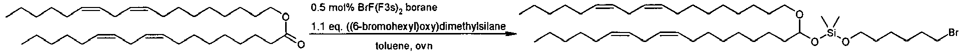

(20Z,23Z)-1-bromo-10-((8Z,11Z)-heptadeca-8,11-dien-1-yl)-8,8-dimethyl-7,9,11-trioxa-8-silanonacosa-20,23-diene was prepared from (9Z,12Z)-octadeca-9,12-dien-1-yl (9Z,12Z)-octadeca-9,12-dienoate (1.00 g, 1.891 mmol, 1.0 eq.) and ((6-bromohexyl)oxy)dimethylsilane (452.3 mg, 1.891 mmol, 1.0 eq.) using 100 µl BrF(F3s)₂ borane catalyst solution (4.23 mg was dissolved in 100 µl abs. toluene) according to **General Procedure C.** The reaction was completed after overnight stirring. The crude product that was purified by flash column chromatography with gradient elution (0-30 % EtOAc in n-hexane) yielding a transparent oil: 1.311 g, 1.707 mmol, 90 % yield.
¹H NMR (500 MHz, CDCl₃) δ 5.41-5.29 (m, 8H), δ 4.79 (m, 1H), δ 3.74-3.63 (m, 3H), δ 3.42-3.38 (m, 2H), δ 3.34-3.28 (m, 1H), δ 2.79-2.75 (m, 4H), δ 2.07-2.02 (m, 8H), δ 1.90-1.83 (m, 2H), δ 1.68-1.23 (m, 42H), δ 0.89 (m, 6H), δ 0.16 (d, 6H)

### Example 31

### Synthesis of 1-bromo-8,8-dimethyl-10-pentadecyl-7,9,11-trioxa-8-silaheptacosane

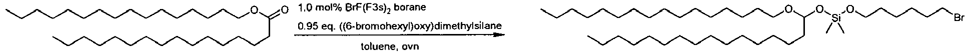

1-bromo-8,8-dimethyl-10-pentadecyl-7,9,11-trioxa-8-silaheptacosane was prepared from hexadecyl palmitate (1.34 g, 2.80 mmol, 1.0 eq.) and ((6-bromohexyl)oxy)dimethylsilane (635.7 mg, 2.66 mmol, 0.95 eq.) using 100 µl BrF(F3s)₂ borane catalyst solution (12.5 mg was dissolved in 100 µl abs. toluene) according to **General Procedure C.** The reaction was completed after overnight stirring. The crude product that was purified by flash column chromatography (30 % EtOAc in petroleum ether with 2 % TEA) yielding a transparent oil: 1.94 mg, 2.54 mmol, 91 % yield.
¹H NMR (500 MHz, Benzene-d₆) δ 4.99 (dt, 1H), δ 3.89-3.81 (m, 1H), δ 3.68 (t, 2H), δ 3.45-3.38 (m, 1H), δ 2.96 (t, 2H), δ 1.97-1.78 (m, 2H), δ 1.72-1.04 (br.m, 62H), δ 0.93-0.88 (m, 6H), δ 0.25 (d, 6H)

### Example 32

### Synthesis of (Z)-1-bromo-10-(heptadec-8-en-1-yl)-13-hexyl-8,8-dimethyl-7,9,11-trioxa-8-silahenicosane

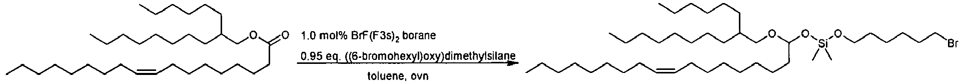

(Z)-1-bromo-10-(heptadec-8-en-1-yl)-13-hexyl-8,8-dimethyl-7,9,11-trioxa-8-silahenicosane was prepared from 2-hexyldecyl oleate (1.064 g, 2.10 mmol, 1.0 eq.) and ((6-bromohexyl)oxy)dimethylsilane (477 mg, 1.99 mmol, 0.95 eq.) using 100 µl BrF(F3s)₂ borane catalyst solution (9.38 mg was dissolved in 100 µl abs. toluene) according to **General Procedure C.** The reaction was completed after overnight stirring. The crude product that was purified by flash column chromatography (30 % EtOAc in petroleum ether with 2 % TEA) yielding a transparent oil: 1.81 g, 1.80 mmol, 86 % yield.
¹H NMR (500 MHz, Benzene-d₆) δ 5.52-5.49 (m, 2H), δ 5.00 (dt, 1H), δ 3.88-3.85 (m, 1H), δ 3.70 (t, 2H), δ 3.42-3.36 (m, 1H), δ 2.98 (t, 2H), δ 2.22 (t, 1H), δ 2.13-2.06 (m, 4H), δ 1.97-1.88 (m, 1H), δ 1.86-1.78 (m, 1H), δ 1.77-1.69 (m, 1H), δ 1.65-1.16 (br. m, 53H), δ 0.93-0.90 (m, 9H), δ 0.28 (d, 6H)

### Example 33

### Synthesis of (Z)-1-bromo-10-((3R)-3-((3R,10S,13R)-3-methoxy-10,13-dimethylhexadecahydro-1H-cyclopenta[a]phenanthren-17-yl)butyl)-8,8-dimethyl-7,9,11-trioxa-8-silanonacos-20-ene

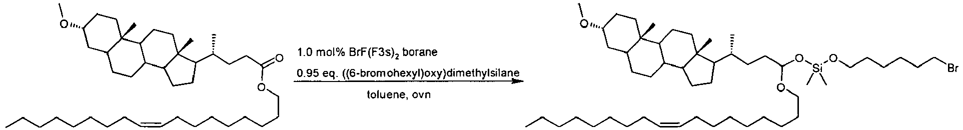

(Z)-1-bromo-10-((3R)-3-((3R,10S,13R)-3-methoxy-10,13-dimethylhexadecahydro-1H-cyclopenta[a]phenanthren-17-yl)butyl)-8,8-dimethyl-7,9, I-trioxa-8-silanonacos-20-ene was prepared from (Z)-octadec-9-en-1-yl (4R)-4-((3R,10S,13R)-3-methoxy-10,13-dimethylhexadecahydro-1H-cyclopenta[a]phenanthren-17-yl)pentanoate (1.40 g, 2.18 mmol, 1.0 eq.) and ((6-bromohexyl)oxy)dimethylsilane (496.3 mg, 2.07 mmol, 0.95 eq.) using 100 µl BrF(F3s)₂ borane catalyst solution (9.76 mg was dissolved in 100 µl abs. toluene) according to **General Procedure C.** The reaction was completed after overnight stirring. The crude product that was purified by flash column chromatography (30 % EtOAc in petroleum ether with 2 % TEA) yielding a transparent oil: 1.83 g, 1.70 mmol, 78 % yield.
¹H NMR (500 MHz, Benzene-d₆) δ 5.51 (m, 2H), δ 5.03 (dt, 1H), δ 3.92-3.85 (m, 1H), δ 3.72 (t, 2H), δ 3.48-3.42 (m, 1H), δ 3.25 (s, 3H), δ 3.09-2.91 (m, 2H), δ 2.18-0.50 (br. m, 77H), δ 0.30 (d, 6H)

### Example 34

### Synthesis of (20Z,23Z)-l-bromo-10-((3R)-3-((3R, 10S, 13R)-3-methoxy-10,13-dimethylhexadecahydro-1H-cyclopenta[a]phenanthren-17-yl)butyl)-8,8-dimethyl-7,9,11-trioxa-8-silanonacosa-20,23-diene

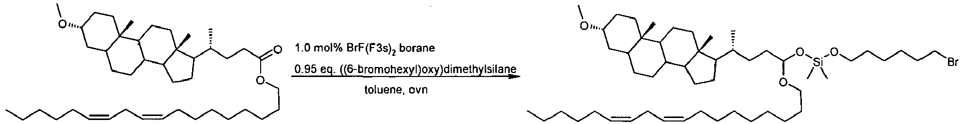

(20Z,23Z)-1-bromo-10-((3R)-3-((3R,10S,13R)-3-methoxy-10,13-dimethylhexadecahydro-1H-cyclopenta[a]phenanthren-17-yl)butyl)-8,8-dimethyl-7,9,11-trioxa-8-silanonacosa-20,23-diene was prepared from (9Z,12Z)-octadeca-9,12-dien-1 -yl (4R)-4-((3R,10S,1 3R)-3-methoxy-10,13-dimethylhexadecahydro-1H-cyclopenta[a]phenanthren-17-yl)pentanoate (400 mg, 0.626 mmol, 1.0 eq.) and ((6-bromohexyl)oxy)dimethylsilane (142.25 mg, 0.595 mmol, 0.95 eq.) using 100 µl BrF(F3s)₂ borane catalyst solution (2.8 mg was dissolved in 100 µl abs. toluene) according to **General Procedure C.** The reaction was completed after overnight stirring. The crude product that was purified by flash column chromatography (30 % EtOAc in petroleum ether with 2 % TEA) yielding a transparent oil: 523 mg, 0.417 mmol, 67 % yield.
¹H NMR (500 MHz, Benzene-d₆) δ 5.55-5.45 (m, 4H), δ 5.02 (dt, 1H), δ 3.92-3.84 (m, 1H), δ 3.72 (t, 2H), δ 3.48-3.42 (m, 1H), δ 3.25 (s, 3H), δ 3.09-2.94 (m, 2H), δ 2.90 (t, 2H), δ 2.18-0.50 (br. m, 71H), δ 0.30 (d, 6H)

### Example 35 GS-1060

### Synthesis of (3R, 10S, 13R)-17-((2R)-5-((((6-bromohexyl)oxy)dimethylsilyl)oxy)-5-(((Z)-octadec-9-en-1 - yl)oxy)pentan-2-yl)-10,13-dimethylhexadecahydro-1H-cyclopenta[a]phenanthren-3-yl pivalate

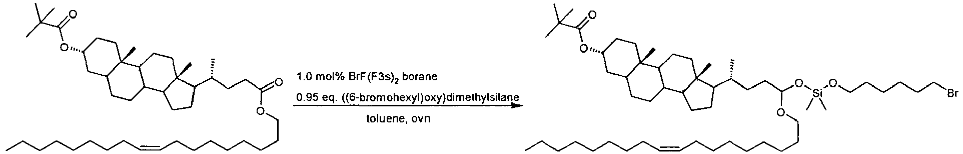

(3R,10S,13R)-17-((2R)-5-((((6-bromohexyl)oxy)dimethylsilyl)oxy)-5-(((Z)-octadec-9-en-1- yl)oxy)pentan-2-yl)-10,13-dimethylhexadecahydro-1H-cyclopenta[a]phenanthren-3-yl pivalate was prepared from (Z)-octadec-9-en-1-yl (4R)-4-((3R,10S,13R)-10,13-dimethyl-3-(pivaloyloxy)hexadecahydro-1H-cyclopenta[a]phenanthren-17-yl)pentanoate (1.74 g, 2.45 mmol) and ((6-bromohexyl)oxy)dimethylsilane (556 mg, 2.32 mmol, 0.95 eq.) using 100 µl BrF(F3s)₂ borane catalyst solution (10.94 mg was dissolved in 100 µl abs. toluene) according to **General Procedure C.** The reaction was completed after overnight stirring. The crude product was filtered through a silica pad rising with MTBE and concentrated and yielding a transparent oil: 2.24 g, 2.40 mmol, 98 % yield.
¹H NMR (500 MHz, Benzene-d₆) δ 5.51-5.48 (m, 2H), δ 5.02 (m, 1H), δ 4.93-4.85 (m, 1H), δ 3.90-3.83 (m, 1H), δ 3.71 (t, 2H), δ 3.49-3.39 (m, 1H), δ 2.99 (t, 2H), δ 2.15-0.50 (br. m, 85H), δ 0.28 (d, 6H)

### Example 36

### Synthesis of (3R,10S,13R)-17-((2R)-5-((((6-bromohexyl)oxy)dimethylsilyl)oxy)-5-(((9Z,12Z)-octadeca-9,12-dien-1-yl)oxy)pentan-2-yl)-10,13-dimethylhexadecahydro-1H-cyclopenta[a]phenanthren-3-yl pivalate

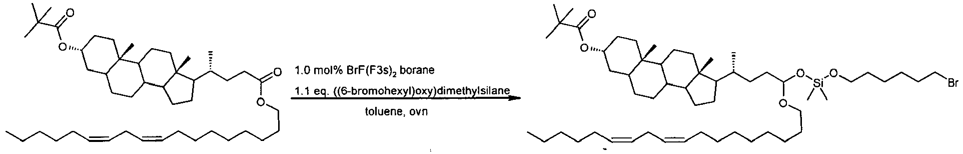

(3R,10S,13R)-17-((2R)-5-((((6-bromohexyl)oxy)dimethylsilyl)oxy)-5-(((9Z,12Z)-octadeca-9,12-dien-l-yl)oxy)pentan-2-yl)-10,13-dimethylhexadecahydro-1H-cyclopenta[a]phenanthren-3-yl pivalate was prepared from (9Z,12Z)-octadeca-9,12-dien-1-yl (4R)-4-((3R,10S, 13R)-10, 13-dimethyl-3-(pivaloyloxy)hexadecahydro-1H-cyclopenta[a]phenanthren-17-yl)pentanoate (990.0 mg, 1.40 mmol, 1.0 eq.) and ((6-bromohexyl)oxy)dimethylsilane (317.3 mg, 1.326 mmol, 0.95 eq.) using 100 µl BrF(F3s)₂ borane catalyst solution (6.24 mg was dissolved in 100 µl abs. toluene) according to **General Procedure C.** The reaction was completed after overnight stirring. The crude product that was purified by flash column chromatography with gradient elution (0-5 % EtOAc in n-hexane) yielding a transparent oil: 1.26 g, 1.33 mmol, 95 % yield.
¹H NMR (500 MHz, Benzene-d₆) δ 5.54-5.45 (m, 4H), δ 5.01 (m, 1H), δ 4.95-4.87 (m, 1H) δ 3.92-3.85 (m, 1H), δ 3.72 (t, 2H), δ 3.48-3.44 (m, 1H), δ 2.98 (t, 2H), 2.90 (t, 2H), δ 2.11-2.09 (m, 5H), δ 2.00-0.71 (br.m, 74H), δ 0.30 (d, 6H)

### Example of the lipid synthesis

### Example 37

### Synthesis of (21Z,24Z)-3-(2-hydroxyethyl)-11,11-dimethyl-9-(((9Z,12Z)-octadeca-9,12-dien-1-yl)oxy)-10,12-dioxa-3-aza-11-silatriaconta-21,24-dien-1-ol

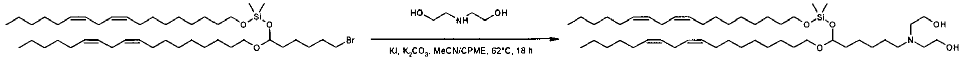

((21Z,24Z)-3-(2-hydroxyethyl)-11,11-dimethyl-9-(((9Z,12Z)-octadeca-9,12-dien-1-yl)oxy)-10,12-dioxa-3-aza-11-silatriaconta-21,24-dien-1-ol was prepared from (6Z,9Z,32Z,35Z)-22-(5-bromopentyl)-20,20-dimethyl-19,21,23-trioxa-20-silahentetraconta-6,9,32,35-tetraene (700.0 mg, 0.912 mmol, 1.0 eq.) and diethanolamine (95.81 mg, 0.912 mmol, 1.0 eq.) according to **General Procedure D.** The crude product was purified by flash column chromatography according to **Purification Method A** to obtain the pure product as a colourless oil, 70 mg, 0.088 mmol, 10 % yield.
¹H NMR (500 MHz, Benzene-d₆) δ 5.55-5.43 (m, 8H), δ 5.01 (m, 1H), δ 3.91-3.84 (m, 1H), δ 3.77 (t, 2H), δ 3.46-3.36 (m, 5H), δ 2.92-2.88 (m, 4H), δ 2.33 (t, 4H), δ 2.29 (t, 2H), δ 2.15-2.05 (m, 8H), δ 1.93-1.76 (m, 2H), δ 1.72-1.60 (m, 4H) δ 1.60-1.20 (m, 38H), δ 0.89 (m, 6H), δ 0.29 (d, 6H); TOF MS ES⁺ [M+H⁺]: 792.6918 m/z

### Example 38

### Synthesis of (23Z,26Z)-11-((8Z,11Z)-heptadeca-8,11-dien-1-yl)-3-(2-hydroxyethyl)-13,13-dimethyl-10,12,14-trioxa-3-aza-13-siladotriaconta-23,26-dien-1-ol

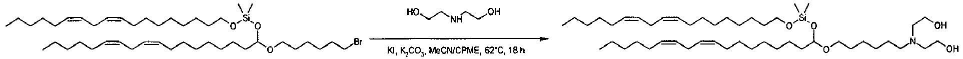

(23Z,26Z)-11-((8Z,11Z)-heptadeca-8,11-dien-1-yl)-3-(2-hydroxyethyl)-13,13-dimethyl-10,12,14-trioxa-3-aza-13-siladotriaconta-23,26-dien-1-ol was prepared from (20Z,23Z)-1-bromo-8-((8Z,11Z)-heptadeca-8,11-dien-1-yl)-10,10-dimethyl-7,9,11-trioxa-10-silanonacosa-20,23-diene (700.0 mg, 0.912 mmol, 1.0 eq.) and diethanolamine (95.81 mg, 0.912 mmol, 1.0 eq.) according to **General Procedure D.** The crude product was purified by flash column chromatography according to **Purification Method A** to obtain the pure product as a colourless oil, 140 mg, 0.177 mmol, 19 % yield.
¹H NMR (500 MHz, Benzene-d₆) δ 5.55-5.43 (m, 8H), δ 5.01 (m, 1H), δ 3.89-3.84 (m, 1H), δ 3.77 (t, 2H), δ 3.45-3.42 (m, 5H), δ 2.94-2.86 (m, 4H), δ 2.34 (t, 4H), δ 2.27 (t, 2H), δ 2.16-2.03 (m, 8H), δ 1.96-1.79 (m, 2H), δ 1.72-1.16 (m, 42H), δ 0.89 (m, 6H), δ 0.29 (d, 6H); TOF MS ES⁺ [M+H⁺]: 792.6908 m/z

### Example 39

### Synthesis of (Z)-3-(2-hydroxyethyl)-11,11-dimethyl-13-pentadecyl-10,12,14-trioxa-3-aza-11-siladotriacont-23-en-l-ol

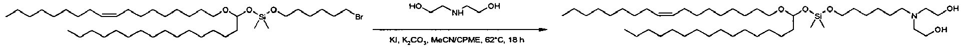

(Z)-3-(2-hydroxyethyl)-11,11-dimethyl-13-pentadecyl-10,12,14-trioxa-3-aza-11-siladotriacont-23-en-1-ol was prepared from (Z)-1-bromo-8,8-dimethyl-10-pentadecyl-7,9,11-trioxa-8-silanonacos-20-ene (624.5 mg, 0.837 mmol, 1.1 eq.) and diethanolamine (80 mg, 0.761 mmol, 1.0 eq.) according to **General Procedure D.** The crude product was purified by flash column chromatography according to **Purification Method A** to obtain the pure product as a colourless oil, 200 mg, 0.259 mmol, 34 % yield.
¹H NMR (500 MHz, Benzene-d₆) δ 5.54-5.47 (m, 2H), δ 5.01 (m, 1H), δ 3.89-3.84 (m, 1H), δ 3.77 (t, 2H), δ 3.47-3.40 (m, 5H), δ 2.35 (t, 4H), δ 2.29 (t, 2H), δ 2.14-2.09 (m, 4H), δ 1.96 -1.79 (m, 2H), δ 1.71-1.20 (br. m, 58H), δ 0.94-0.90 (m, 6H), δ 0.29 (d, 6H); TOF MS ES⁺ [M+H⁺]: 770.7049 m/z

### Example 40

Synthesis of (Z)-13-(heptadec-8-en-1-yl)-3-(2-hydroxyethyl)-1 1,1 1 -dimethyl-10,12,14-trioxa-3-aza-11-silatriacontan-1 -ol

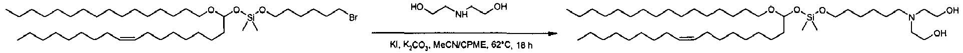

(Z)-13-(heptadec-8-en-1-yl)-3-(2-hydroxyethyl)-11,11-dimethyl-10,12,14-trioxa-3-aza-11-silatriacontan-1-ol was prepared from (Z)-1-bromo-10-(heptadec-8-en-1-yl)-8,8-dimethyl-7,9,11-trioxa-8-silaheptacosane (1.115 g, 1.151 mmol, 1.1 eq.) and diethanolamine (110 mg, 1.046 mmol, 1.0 eq.) according to **General Procedure D.** The crude product was purified by flash column chromatography according to Purification **Method A** to obtain the pure product as a colourless oil, 145 mg, 0.188 mmol, 18 % yield.
¹H NMR (500 MHz, Benzene-d₆) δ 5.52-5.46 (m, 2H), δ 5 00 (m, 1H), δ 3.91-3.84 (m, 1H), δ 3 77 (t, 2H), δ 3 48-3.40 (m, 5H), δ 2.37 (t, 4H), δ 2.30 (t, 2H), δ 2.13-2.08 (m, 4H), δ 1.94 -1.78 (m, 2H), δ 1.71-1.20 (br. m, 58H), δ 0.94-0.90 (m, 6H), δ 0.29 (d, 6H); TOF MS ES⁺[M+H⁺]: 770.7053 m/z

### Example 41

### Synthesis of (23Z,26Z)-3-(2-hydroxyethyl)-11,11-dimethyl-13-pentadecyl-10,12,14-trioxa-3-aza-11-siladotriaconta-23,26-dien-1-ol

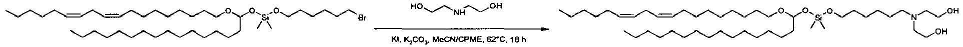

(23Z,26Z)-3-(2-hydroxyethyl)-11,11-dimethyl-13-pentadecyl-10,12,14-trioxa-3-aza-11-si ladotriaconta-23,26-dien-1-ol was prepared from (20Z,23Z)-1-bromo-8,8-dimethyl-10-pentadecyl-7,9,11-trioxa-8-silanonacosa-20,23-diene (934.2 mg, 1.255 mmol, 1.1 eq.) and diethanolamine (120 mg, 1.141 mmol, 1.0 eq.) according to **General Procedure D.** The crude product was purified by flash column chromatography according to **Purification Method A** to obtain the pure product as a colourless oil, 78 mg, 0.101 mmol, 9 % yield.
¹H NMR (500 MHz, Benzene-d₆) δ 5.57-5.43 (m, 4H), δ 5.00 (m, 1H), δ 3.92-3.84 (m, 1H), δ 3.76 (t, 2H), δ 3.47-3.40 (m, 5H), δ 2.90 (t, 2H), δ 2.38 (t, 4H), δ 2.24 (t, 2H), δ 2.13-2.02 (m, 4H), δ 1.98 -1.82 (m, 2H), δ 1.78-1.19 (br. m, 52H), δ 0.94-0.90 (m, 6H), δ 0.29 (d, 6H); TOF MS ES⁺ [M+H⁺]: 768.6927 m/z

### Example 42

### Synthesis of 13-((8Z,11Z)-heptadeca-8,11-dien-1-yl)-3-(2-hydroxyethyl)-11,11-dim ethyl-10,12,14-trioxa-3-aza-11-silatriacontan-1-ol

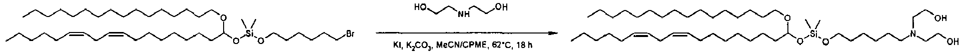

13-((8Z,11)-heptadeca-8,11-dien-1-yi)-3-(2-hydroxyethyl)-11,11-dimethyl-10,12,14-trioxa-3-aza-11-silatriacontan-1-ol was prepared from 1-bromo-10-((8Z,11Z)-heptadeca-8,11-dien-1-yl)-8,8-dimethyl-7,9,11-trioxa-8-silaheptacosane (1.40 g, 1.88 mmol, 1.1 eq.) and diethanolamine (180 mg, 1.71 mmol, 1.0 eq.) according to **General Procedure D.** The crude product was purified by flash column chromatography according to **Purification Method A** to obtain the pure product as a colourless oil, 630 mg, 0.82 mmol, 48 % yield.
¹H NMR (500 MHz, Benzene-d₆) δ 5.55-5.45 (m, 4H), δ 5.00 (m, 1H), δ 3.91-3.84 (m, 1H), δ 3.77 (t, 2H), δ 3.47-3.40 (m, 5H), δ 2.89 (t, 2H), δ 2.35 (t, 4H), δ 2.29 (t, 2H), δ 2.16-2.02 (m, 4H), δ 1.95 -1.75 (m, 2H), δ 1.74-1.19 (br. m, 52H), δ 0.94-0.87 (m, 6H), δ 0.29 (d, 6H); TOF MS ES⁺ [M+H⁺]: 768.6915 m/z

### Example 43

### Synthesis of (Z)-13-((8Z,11Z)-heptadeca-8,11-dien-1-yl)-3-(2-hydroxyethy1)-11,11-dimethyl-10,12, 14-trioxa-3-aza-11-siladotriacont-23-en-1-ol

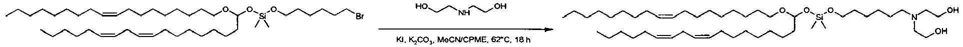

(Z)-13-((8Z,11Z)-heptadeca-8,11-dien-1-yl)-3-(2-hydroxyethyl)-11,11-dimethyl-10,12,14-trioxa-3-aza-11-siladotriacont-23-en-1-ol was prepared from (Z)-1-bromo-10-((8Z,11Z)-heptadeca-8,11-dien-1-yl)-8,8-dimethyl-7,9,11-trioxa-8-silanonacos-20-ene (700.87 mg, 0.910 mmol, 1.1 eq.) and diethanolamine (86.98 mg, 0.827 mmol, 1.1 eq.) according to **General Procedure D.** The crude product was purified by flash column chromatography according to **Purification Method A** to obtain the pure product as a colourless oil, 177 mg, 0.223 mmol, 27 % yield.
¹H NMR (500 MHz, Benzene-d₆) δ 5.54-5.43 (m, 6H), δ 5.00 (m, 1H), δ 3.89-3.82 (m, 1H), δ 3.77 (t, 2H), δ 3.50 (t, 4H), δ 3.45-3.39 (m, 1H), δ 2.98-2.85 (m, 2H), δ 2.41 (t, 4H), δ 2.34 (t, 2H), δ 2.16-2.03 (m, 8H), δ 1.95 -1.77 (m, 2H), δ 1.71-1.15 (br. m, 48H), δ 0.93-0.88 (m, 6H), δ 0.29 (d, 6H); TOF MS ES⁺ [M+H⁺]: 794.7070 m/z

### Example 44

### Synthesis of (23Z,26Z)-13-((Z)-heptadec-8-en-1-yl)-3-(2-hydroxyethyl)-11,11-dimethyl-10,12,14-trioxa-3-aza-11-s i ladotriaconta-23,26-d ien-1-ol

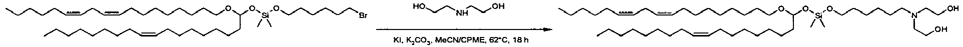

(23Z,26Z)-13-((Z)-heptadec-8-en-1-yl)-3-(2-hydroxyethyl)-11,11-dimethyl-10,12,14-trioxa-3-aza-11-siladotriaconta-23,26-dien-1-ol was prepared from (20Z,23Z)-1-bromo-10-((Z)-heptadee-8-en-1-yl)-8,8-dimethyl-7,9,11-trioxa-8-silanonacosa-20,23-diene (701.0 mg, 0.910 mmol, 1.1 eq.) and diethanolamine (82.0 mg, 0.827 mmol, 1.0 eq.) according to **General Procedure D.** The crude product was purified by flash column chromatography according to **Purification Method A** to obtain the pure product as a colourless oil, 165 mg, 0.208 mmol, 25 % yield.
¹H NMR (500 MHz, Benzene-d₆) δ 5.55-5.44 (m, 6H), δ 5.00 (m, 1H), δ 3.90-3.83 (m, 1H), δ 3.77 (t, 2H), δ 3.47 -3.40 (m, 5H), δ 2.92-2.88 (m, 2H), δ 2.35 (t, 4H), δ 2.29 (t, 2H), δ 2.19-2.03 (m, 8H), δ 1.97 -1.78 (m, 2H), δ 1.73-1.15 (br. m, 48H), δ 0.93-0.88 (m, 6H), δ 0.29 (d, 6H); TOF MS ES⁺ [M+H⁺]: 794.7068 m/z

### Example 45

### Synthesis of (Z)-13-((Z)-heptadec-8-en-1-yl)-3-(2-hydroxyethyl)-11,11-dimethyl-10,12,14-trioxa-3-aza-11-siladotriacont-23-en-1-ol

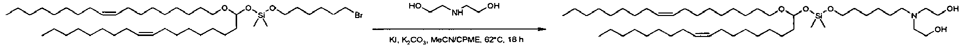

(Z)-13-((Z)-heptadec-8-en-1-yl)-3-(2-hydroxyethyl)-11,11 -dimethyl-10,12,14-trioxa-3-aza-11-siladotriacont-23-en-1-ol was prepared from (Z)-1-bromo-10-((Z)-heptadec-8-en-1-yl)-8,8-dimethyl-7,9,11-trioxa-8-silanonacos-20-ene (634.55 mg, 0.8218 mmol, 1.2 eq.) and diethanolamine (72.0 mg, 0.685 mmol, 1.0 eq.) according to **General Procedure D.** The crude product was purified by flash column chromatography according to **Purification Method A** to obtain the pure product as a colourless oil, 116 mg, 0.146 mmol, 21 % yield.
¹H NMR (500 MHz, Benzene-d₆) δ 5.55-5.46 (m, 2H), δ 5.00 (m, 1H), δ 3.90-3.85 (m, 1H), δ 3.77 (t, 2H), δ 3.45-3.40 (m, 5H), δ 2.34 (t, 4H), δ 2.28 (t, 2H), δ 2.13-2.08 (m, 8H), δ 2.00 -1.80 (m, 4H), δ 1.72-1.18 (br. m, 54H), δ 0.92 (t, 6H), δ 0.29 (d, 6H); TOF MS ES⁺ [M+H⁺]: 796.7222 m/z

### Example 46

### Synthesis of (23Z,26Z)-13-((8Z,11Z)-heptadeca-8,11-dien-1-yl)-3-(2-hydroxyethyl)-11,11-dimethyl-10,12,14-trioxa-3-aza-11 -siladotriaconta-23,26-dien-1 -ol

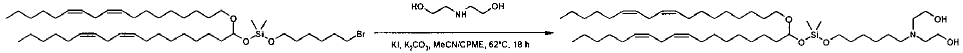

(23Z,26Z)-13-((8Z,11Z)-heptadeca-8,11-dien-1-yl)-3-(2-hydroxyethyl)-11,11-dimethyl-10,12,14-trioxa-3-aza-11-siladotriaconta-23,26-dien-1-ol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (660.0 mg, 0.859 mmol, 1.0 eq.) and diethanolamine (180.67 mg, 1.718 mmol, 2.0 eq.) according to **General Procedure D.** The crude product was purified by flash column chromatography according to **Purification Method A** to obtain the pure product as a colourless oil, 126 mg, 0.159 mmol, 18 % yield.
¹H NMR (500 MHz, Benzene-d₆) δ 5.55-5.43 (m, 8H), δ 5.00 (m, 1H), δ 3.89-3.84 (m, 1H), δ 3.77 (t, 2H), δ 3.47 -3.39 (m, 5H), δ 2.91-2.88 (m, 4H), δ 2.37 (t, 4H), δ 2.32 (t, 2H), δ 2.13-2.06 (m, 8H), δ 1.98 -1.75 (m, 2H), δ 1.73-1.15 (br. m, 42H), δ 0.91-0.88 (m, 6H), δ 0.29 (d, 6H); TOF MS ES⁺ [M+H⁺]: 792.6915 m/z

### Example 47

### Synthesis of 3-(2-hydroxyethyl)-11, 11-dimethyl-13-pentadecyl-10,12,14-trioxa-3-aza-11-silatriacontan-11-ol

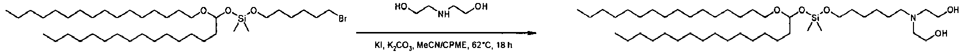

3-(2-hydroxyethyl)-11,11-dimethyl-13-pentadecyl-10,12,14-trioxa-3-aza-11-silatriacontan-1-ol was prepared from 1-bromo-8,8-dimethyl-10-pentadecyl-7,9,11-trioxa-8-silaheptacosane (828.7 mg, 1.151 mmol, 1.1 eq.) and diethanolamine (110.0 mg, 1.046 mmol, 1.0 eq.) according to **General Procedure D.** The crude product was purified by flash column chromatography according to **Purification Method A** to obtain the pure product as a colourless oil, 206 mg, 0.277 mmol, 26 % yield.
¹H NMR (500 MHz, CDCl₃) δ 5.12 (td, 1H), δ 4.02-3.94 (m, 1H), δ 3.88 (t, 2H), δ 3.57-3.50 (m, 5H), δ 2.44 (t, 4H), δ 2.38 (t, 2H), δ 2.09-1.88 (m, 2H), δ 1.84 -1.30 (br. m, 62H), δ 1.02 (t, 6H), δ 0.40 (d, 6H); TOF MS ES⁺ [M+H⁺]: 744.6933 m/z

### Example 48

Synthesis of (Z)-13-(heptadec-8-en-1-yl)-16-hexyl-3-(2-hydroxyethyl)-11,11-dimethyl-10,12,14-trioxa-3-aza-11-silatetracosan-1-ol

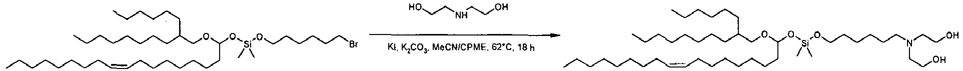

(Z)-13-(heptadec-8-en-1-yl)-16-hexyl-3-(2-hydroxyethyl)-11,11-dimethyl-10,12,14-trioxa-3-aza-11-silatetracosan-1-ol was prepared from (Z)-1-bromo-10-(heptadec-8-en-1-yl)-13-hexyl-8,8-dimethyl-7,9,11-trioxa-8-silahenicosane (1.34 g, 1.80 mmol, 1.1 eq.) and diethanolamine (172 mg, 1.64 mmol, 1.0 eq.) according to **General Procedure D.** The crude product was purified by flash column chromatography according to **Purification Method A** to obtain the pure product as a colourless oil, 291 mg, 0.380 mmol, 23 % yield.
¹H NMR (500 MHz, Benzene-d₆) δ 5.54-5.47 (m, 2H), δ 5.02 (m, 1H), δ 3.89-3.86 (m, 1H), δ 3.78 (t, 2H), δ 3.45 (t, 4H), δ 3.41-3.37 (m, 1H) δ 2.35 (t, 4H), δ 2.30 (t, 2H), δ 2.14-2.09 (m, 4H), δ 1.96 -1.78 (m, 2H), 1.77 -1.69 (m, 1H), δ 1.67-1.17 (br. m, 53H), δ 0.94-0.90 (m, 9H), δ 0.29 (d, 6H); TOF MS ES⁺[M+H⁺]: 770.7062 m/z

### Example 49

### Synthesis of (Z)-3-(2-hydroxyethyl)-13-((3R)-3-((3R,10S,13R)-3-methoxy-10,13-dimethylhexadecahydro-1H-cyclopenta[a]phenanthren-17-yl)butyl)-11,11-dim ethyl-10,12,14-trioxa-3-aza-11 -siladotriacont-23-en-1 -ol

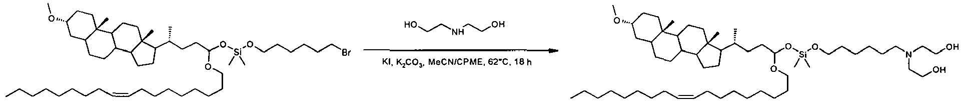

(Z)-3-(2-hydroxyethyl)-13-((3R)-3-((3R,10S,13R)-3-methoxy-10,13-dimethylhexadecahydro-1H-cyclopenta[a]phenanthren-17-yl)butyl)-11,11-dimethyl-10,12,14-trioxa-3-aza-11-siladotriacont-23-en-1-ol was prepared from (Z)-1-bromo-10-((3R)-3-((3R,10S,13R)-3-methoxy-10,13-dimethylhexadecahydro-1H-cyclopenta[a]phenanthren-17-yl)butyl)-8,8-dimethyl-7,9,11-trioxa-8-silanonacos-20-ene (1.49 g, 1.695 mmol, 1.1 eq.) and diethanolamine (162 mg, 11.541 mmol, 1 eq.) according to **General Procedure D.** The crude product was purified by flash column chromatography according to **Purification Method A** to obtain the pure product as a colourless oil, 337 mg, 0.373 mmol, 24 % yield.
¹H NMR (500 MHz, Benzene-d₆) δ 5.55-5.48 (m, 2H), δ 5.05-5.00 (m, 1H), δ 3.80 (t, 2H), δ 3.48-3.45 (m, 5H), δ 3.24 (s, 3H), δ 3.07-3.04 (m, 1H), δ 2.39-2.29 (m, 6H), δ 2.15-0.80 (br. m, 74H), δ 0.65 (d, 3H), δ 0.32 (d, 6H); TOF MS ES⁺ [M+H⁺]: 904.7784 m/z

### Example 50

### Synthesis of (23Z,26Z)-3-(2-hydroxyethyl)-13-((3R)-3-((3R,10S,13R)-3-methoxy-10,13-dimethylhexadecahydro-1H-cyclopenta[a]phenanthren-17-yl)butyl)-11,11-dimethyl-10,12,14-trioxa-3-aza-10-siladotriaconta-23,26-dien-1-ol

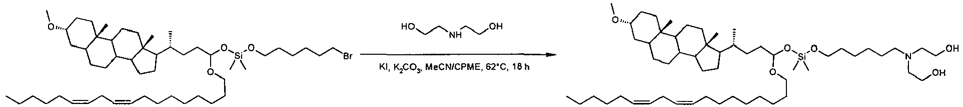

(23Z,26Z)-3-(2-hydroxyethyl)-13-((3R)-3-((3R,10S,13R)-3-methoxy-10,13-dimethylhexadecahydro-1H-cyclopenta[a]phenanthren-17-yl)butyl)-11,11-dimethyl-10,12,14-trioxa-3-aza-11-siladotriaconta-23,26-dien-1-ol was prepared from (20Z,23Z)-1-bromo-10-((3R)-3-((3R,10S,13R)-3-methoxy-10,13-dimethylhexadecahydro-1H-cyclopenta[a]phenanthren-17-yl)butyl)-8,8-dimethyl-7,9,11-trioxa-8-silanonacosa-20,23-diene (367.1 mg, 0.418 mmol, 1.1 eq.) and diethanolamine (40 mg, 0.380 mmol, 1.0 eq.) according to **General Procedure D.** The crude product was purified by flash column chromatography according to **Purification Method A** to obtain the pure product as a colourless oil, 124 mg, 0.137 mmol, 36 % yield.
¹H NMR (500 MHz, Benzene-d₆) δ 5.55-5.46 (m, 4H), δ 5.05-5.02 (m, 1H), δ 3.80 (t, 2H), δ 3.50-3.42 (m, 5H), δ 3.24 (d, 3H), δ 3.08-3.01 (m, 1H), δ 2.90 (t, 2H) δ 2.41-2.30 (m, 6H), δ 2.13-0.80 (br.m , 68H), δ 0.65 (d, 3H), δ 0.31 (d, 6H); TOF MS ES⁺ [M+H⁺]: 902.7617 m/z

### Example 51

### Synthesis of (3R,10S,13R)-17-((16R)-l-hydroxy-3-(2-hydroxyethyl)-11,11-dimethyl-13-(((Z)-octadec-9-en-1-yl)oxy)-10, 12-dioxa-3-aza-11-silaheptadecan-16-yl)-10,13-dimethylhexadecahydro-1H-cyclopenta[a]phenanthren-3-yl pivalate

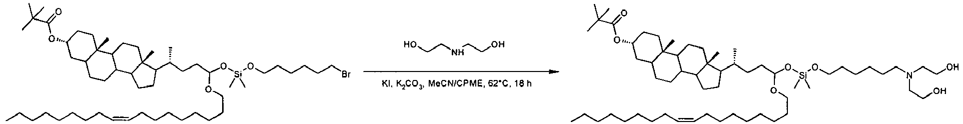

(3R,10S,13R)- 17-((16R)-1-hydroxy-3-(2-hydroxyethyl)- 11,11-dimethyl-13-(((Z)-octadec-9-en-1-yl)oxy)-10,12-dioxa-3-aza-11-silaheptadecan-16-yl)-10,13-dimethylhexadecahydro-1H-cyclopenta[a]phenanthren-3-yl pivalate was prepared from (3R,10S,13R)-17-((2R)-5-((((6-bromohexyl)oxy)dimethylsilyl)oxy)-5-(((Z)-octadec-9-en-1-yl)oxy)pentan-2-yl)-10,13-dimethylhexadecahydro-1H-cyclopenta[a]phenanthren-3-yl pivalate (973.6 mg, 1.042 mmol, 1.0 eq.) and diethanolamine (109.6 mg, 1.042 mmol, 1.0 eq.) according to **General Procedure D.** The crude product was purified by flash column chromatography according to **Purification Method A** to obtain the pure product as a colourless oil, 120 mg, 0.12 mmol, 12 % yield.
ESI LC MS [M+H⁺]: 974.962 m/z

### Example 52

### Synthesis of (3R,10S,13R)-17-((16R)-1-hydroxy-3-(2-hydroxyethyl)-11,11-dimethyl-13-(((9Z,12Z)-octadeca-9,12-dien-1-yl)oxy)-10,12-dioxa-3-aza-11-silaheptadecan-16-yl)-10,13-dimethylhexadecahydro-1H-cyclopenta[a]phenanthren-3-yl pivalate

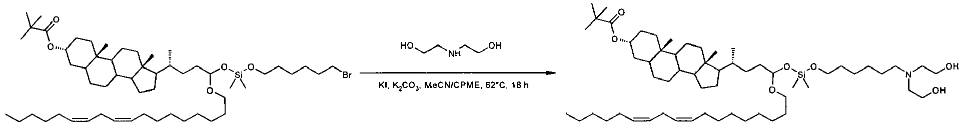

(3R,10S,13R)-17-((16R)-1-hydroxy-3-(2-hydroxyethyl)-11,11-dimethyl-13-(((9Z,12Z)-octadeca-9,12-dien-1-yl)oxy)-10,12-dioxa-3-aza-11-silaheptadecan-16-yl)-10,13-dimethylhexadecahydro-1H-cyclopenta[a]phenanthren-3-yl pivalate was prepared from (3R,10S,13R)-17-((2R)-5-((((6-bromohexyl)oxy)dimethylsilyl)oxy)-5-(((9Z,12Z)-octadeca-9,12-dien-1-yl)oxy)pentan-2-yl)-10,13-dimethylhexadecahydro-1H-cyclopenta[a]phenanthren-3-yl pivalate (1090 mg mg, 1.15 mmol, 1.1 eq.) and diethanolamine (109.6 mg, 1.042 mmol, 1.0 eq.) according to **General Procedure D.** The crude product was purified by flash column chromatography according to **Purification Method A** to obtain the pure product as a colourless oil, 106 mg, 0.11 mmol, 10 % yield.
ESI LC MS [M+H⁺]: 972.890 m/z

### Example 53

### Synthesis of 4-((20Z,23Z)-10-((8Z,11Z)-heptadeca-8,11-dien-1-yl)-8,8-dimethyl-7,9,11-trioxa-8-silanonacosa-20,23-dien-1-yl)morpholine

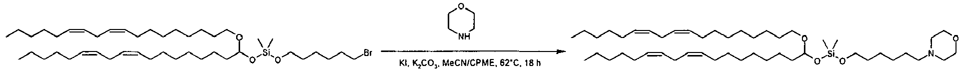

4-((20Z,23Z)-10-((8Z,11Z)-heptadeca-8,11-dien-1-yl)-8,8-dimethyl-7,9,11-trioxa-8-silanonacosa-20,23-dien-1-yl)morpholine was prepared from (20Z,23Z)-1-bromo-10-((8Z,11Z)-heptadeca-8,11-dien-1-yl)-8,8-dimethyl-7,9,1 1-trioxa-8-silanonacosa-20,23-diene (399.77 mg, 0.520 mmol, 1.0 eq.) and morpholine (90.68 mg, 1.041 mmol, 2.0 eq.) according to **General Procedure D.** The crude product was purified by flash column chromatography according to **Purification Method A** to obtain the pure product as a colourless oil, 116 mg, 0.150 mmol, 29 % yield.
¹H NMR (500 MHz, Benzene-d₆) δ 5.55-5.43 (m, 8H), δ 5.00 (m, 1H), δ 3.89-3.83 (m, 1H), δ 3.77 (t, 2H), δ 3.65-3.62 (m, 4H), δ 3.47-3.38 (m, 1H), δ 2.91-2.88 (m, 4H), δ 2.22-2.03 (m, 14H), δ 1.96 -1.78 (m, 2H), δ 1.71-1.15 (br. m, 42H), δ 0.91-0.88 (t, 6H), δ 0.28 (d, 6H); TOF MS ES⁺ [M+H⁺]: 774.6795 m/z

### Example 54

### Synthesis of (23Z,26Z)-13-((8Z,11Z)-heptadeca-8,11-dien-1-yl)-3,11,11-trimethyl-10,12,14-trioxa-3-aza-11-siladotriaconta-23,26-dien-1 -ol

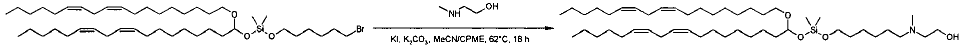

(23Z,26Z)-13-((8Z,11Z)-heptadeca-8,11-dien-1-yl)-3,11,11-trimethyl-10,12,14-trioxa-3-aza-11-siladotriaconta-23,26-dien-1-ol was prepared from (20Z,23Z)-1-bromo-10-((8Z,11Z)-heptadeca-8,11-dien-1-yl)-8,8-dimethyl-7,9,11-trioxa-8-silanonacosa-20,23-diene 660.0 mg, 0.860 mmol, 1.0 eq.) and 2-(methylamino)ethan-1-ol (129.1 mg, 1.719 mmol, 2.0 eq.) according to **General Procedure D.** The crude product was purified by flash column chromatography according to **Purification Method A** to obtain the pure product as a colourless oil, 335 mg, 0.439 mmol, 51 % yield.
¹H NMR (500 MHz, Benzene-d₆) δ 5.54-5.43 (m, 8H), δ 5.00 (m, 1H), δ 3.88-3.82 (m, 1H), δ 3.75 (t, 2H), δ 3.48 (t, 2H), δ 3.45-3.37 (m, 1H), δ 2.89 (t, 4H), δ 2.25-2.23 (t, 2H), δ 2.18-2.04 (m, 10H) δ 1.98 (s, 3H), δ 1.97-1.16 (br. m, 44H), δ 0.91-0.87 (t, 6H), δ 0.28 (d, 6H); TOF MS ES⁺ [M+H⁺]: 762.6829 m/z

### Example 55

Synthesis of 1-((20Z,23Z)-10-((8Z,11Z)-heptadeca-8,11-dien-1-yl)-8,8-dimethyl-7,9,11-trioxa-8-silanonacosa-20,23-dien-1-yl)piperidine

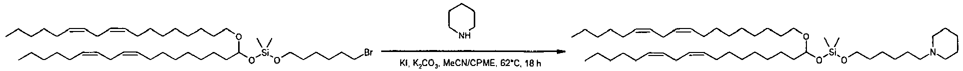

1-((20Z,23Z)-1 0-((8Z,11Z)-heptadeca-8, 1 1-dien- 1-yl)-8,8-dimethyl-7,9,1 1-trioxa-8-silanonacosa-20,23-dien-1-yl)piperidine was prepared from (20Z,23Z)-1-bromo-10-((8Z,11Z)-heptadeca-8,11-dien-1-yl)-8,8-dimethyl-7,9,11-trioxa-8-silanonacosa-20,23-diene (650.0 mg, 0.846 mmol, 1.0 eq.) and piperidine (144.11 mg, 1.693 mmol, 2.0 eq.) according to **General Procedure D.** The crude product was purified by flash column chromatography according to **Purification Method A** to obtain the pure product as a colourless oil, 109 mg, 0.141 mmol, 17 % yield.
¹H NMR (500 MHz, Benzene-d₆) δ 5.56-5.43 (m, 8H), δ 5.00 (m, 1H), δ 3.92-3.82 (m, 1H), δ 3.77 (t, 2H), δ 3.45-3.35 (m, 1H), δ 2.92-2.88 (m, 4H), δ 2.37-2.24 (m, 6H), δ 2.14-2.03 (m, 8H), δ 1.95-1.20 (br. m, 50H), δ 0.91-0.88 (t, 6H), δ 0.28 (d, 6H); LC ESI MS [M+H⁺]: 772.689 m/z

### Example 56

### Synthesis of 2-(4-((20Z,23Z)-10-((8Z,11Z)-heptadeca-8,11-dien-1-yl)-8,8-dimethyl-7,9,11-trioxa-8-silanonacosa-20,23-dien-1-yl)piperazin-1-yl)ethan-1-ol

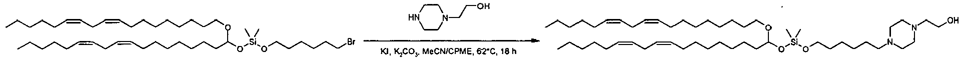

2-(4-((20Z,23Z)-10-((8Z,11Z)-heptadeca-8,11-dien-1-yl)-8,8-dimethyl-7,9,11-trioxa-8-silanonacosa-20,23-dien-1-yl)piperazin-1-yl)ethan-1-ol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (660 mg, 0.859 mmol, 1.0 eq.) and 2-(piperazin-1-yl)ethan-1-ol (223.73 mg, 1.719 mmol, 2.0 eq.) according to **General Procedure D.** The crude product was purified twice by flash column chromatography according to **Purification Method A and B** to obtain the pure product as a colourless oil, 176 mg, 0.215 mmol, 25 % yield.
¹H NMR (500 MHz, Benzene-d₆) δ 5.55-5.43 (m, 8H), δ 5.00 (m, 1H), δ 3.89-3.84 (m, 1H), δ 3.78 (t, 2H), δ 3.49 (t, 2H), δ 3.45-3.40 (m, 1H), δ 2.91-2.88 (m, 4H), δ 2.40-2.16 (m, 12H), δ 2.15-2.01 (m, 8H), δ 1.96 -1.77 (m, 2H), δ 1.73-1.19 (br. m, 42H), δ 0.91-0.88 (t, 6H), δ 0.28 (d, 6H); TOF MS ES⁺[M+H⁺]: 817.7241 m/z

### Example 57

### Synthesis of (24Z,27Z)-14-((8Z,11Z)-heptadeca-8,11-dien-1-yl)-4,12,12-trimethyl-1 1,13,15-trioxa-4-aza-12-silatritriaconta-24,27-diene-1,2-diol

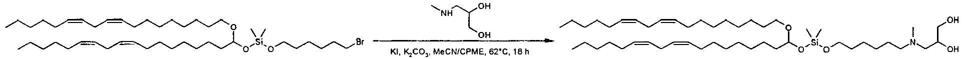

(24Z,27Z)-14-((8Z, 11Z)-heptadeca-8,11-dien-1-yl)-4,12,12-trimethyl-11,13,15-trioxa-4-aza-12-silatritriaconta-24,27-diene-1,2-diol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (642.91 mg, 0.837 mmol, 1.1 eq.) and 3-(methylamino)propane-1,2-diol (80.0 mg, 0.761 mmol, 1.0 eq.) according to **General Procedure D.** The crude product was purified by flash column chromatography according to **Purification Method A** to obtain the pure product as a colourless oil, 318 mg, 0.401 mmol, 53 % yield.
¹H NMR (500 MHz, Benzene-d₆) δ 5.55-5.42 (m, 8H), δ 5.00 (td, 1H), δ 3.90-3.82 (m, 1H), δ 3.76 (t, 2H), δ 3.69-3.56 (m, 2H), δ 3.46-3.38 (m, 2H), δ 2.89 (t, 4H), δ 2.45-2.38 (m, 1H), δ 2.25-1.15 (br. m, 58H), δ 0.89 (t, 6H), δ 0.29 (d, 6H); TOF MS ES⁺[M+H⁺]: 792.6913 m/z

### Example 58

### Synthesis of (25Z,28Z)-15-((8Z,11Z)-heptadeca-8,11-dien-1-yl)-5,13,13-trimethyl-12,14,16-trioxa-5-aza-13-silatetratriaconta-25,28-dien-1-ol

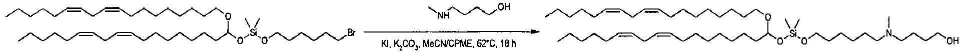

(25Z,28Z)-15-((8Z,11Z)-heptadeca-8,11-dien-1-yl)-5,13,13-trimethyl-12,14,16-trioxa-5-aza-13-silatetratriaconta-25,28-dien-1-ol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (655.2 mg, 0.853 mmol, 1.1 eq.) and 4-(methylamino)butan-1-ol (80.0 mg, 0.775 mmol, 1.0 eq.) according to **General Procedure D.** The crude product was purified by flash column chromatography according to **Purification Method A** to obtain the pure product as a colourless oil, 231 mg, 0.291 mmol, 37 % yield.
¹H NMR (500 MHz, Benzene-d₆) δ 5.55-5.43 (m, 8H), δ 5.01 (td, 1H), δ 3.91-3.84 (m, 1H), δ 3.76 (t, 2H), δ 3.63-3.60 (m, 2H), δ 3.45-3.37 (m, 1H), δ 2.91-2.89 (t, 4H), δ 2.17-2.06 (br. m, 12H), δ 1.97 (s, 3H), δ 1.95-1.78 (m, 2H), δ 1.71-1.18 (br. m, 46H), δ 0.90 (t, 6H), δ 0.30 (d, 6H); TOF MS ES⁺[M+H⁺]: 790.7114 m/z

### Example 59

### Synthesis of (24Z,27Z)-14-((8Z,11Z)-heptadeca-8,11-dien-1-yl)-4-(3-hydroxypropyl)-12,12-dimethyl-11,13,15-trioxa-4-aza-12-silatritriaconta-24,27-dien-l-ol

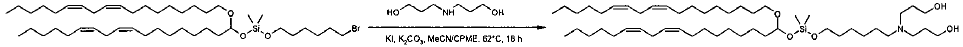

(24Z,27Z)-14-((8Z,11Z)-heptadeca-8,11-dien-1-yl)-4-(3-hydroxypropyl)-12,12-dimethyl-11,13,15-trioxa-4-aza-12-silatritriaconta-24,27-dien-1-ol was prepared from (20Z,23Z)-1-bromo-10-((8Z,1 1Z)-heptadeca-8,11-dien-1-yl)-8,8-dimethyl-7,9,11-trioxa-8-silanonacosa-20,23-diene (634.5 mg, 0.826 mmol, 1.1 eq.) and dipropanolamine (100.0 mg, 0.751 mmol, 1.0 eq.) according to **General Procedure D.** The crude product was purified by flash column chromatography according to **Purification Method A** to obtain the pure product as a colourless oil, 116 mg, 0.141 mmol, 19% yield.
¹H NMR (500 MHz, Benzene-d₆) δ 5.54-5.46 (m, 8H), δ 5.01 (td, 1H), δ 3.91-3.85 (m, 1H), δ 3.78 (t, 2H), δ 3.66 (t, 4H), δ 3.46-3.40 (m, 1H), δ 2.92-2.89 (t, 4H), δ 2.43 (t, 4H), δ 2.26-2.22 (m, 2H), δ 2 13-2.06 (m, 8H), δ 1.96-1.80 (m, 2H), δ 1.71-1.20 (br. m, 46H), δ 0.90 (t, 6H), δ 0.28 (d, 6H); TOF MS ES⁺[M+H⁺]: TOF MS ES⁺ [M+H⁺]: 820.7225 m/z

### Example 60

### Synthesis of (25Z,28Z)-15-((8Z,11Z)-heptadeca-8,11-dien-1-yl)-5-(4-hydroxybutyl)-13,13-dimethyl-12,14,16-trioxa-5-aza-13-silatetratriaconta-25,28-dien-1-ol

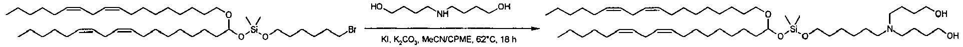

(25Z,28Z)-15-((8Z,11Z)-heptadeca-8,11-dien-1-yl)-5-(4-hydroxybutyl)-13,13-dimethyl-12,14,16-trioxa-5-aza-13-silatetratriaconta-25,28-dien-1-ol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (629.1 mg, 0.819 mmol, 1.1 eq.) and dibutanolamine (120.0 mg, 0.744 mmol, 1.0 eq.) according to **General Procedure D.** The crude product was purified by flash column chromatography according to **Purification Method A** to obtain the pure product as a colourless oil, 150 mg, 0.177 mmol, 24 % yield.
¹H NMR (500 MHz, Benzene-d₆) δ 5.54-5.46 (m, 8H), δ 5.02 (td, 1H), δ 3.89-3.84 (m, 1H), δ 3.79 (t, 2H), δ 3.59 (t, 4H), δ 3.47-3.40 (m, 1H), δ 2.93-2.88 (t, 4H), δ 2.34-2.30 (t, 6H), δ 2.13-2.06 (m, 8H), δ 1.93-1.80 (m, 2H), δ 1.70-1.19 (br. m, 50H), δ 0.90 (t, 6H), δ 0.28 (d, 6H); TOF MS ES⁺ [M+H⁺]: 848.7518 m/z

### Example 61

### Synthesis of (1-((20Z,23Z)-10-((8Z,11Z)-heptadeca-8,11-dien-1-yl)-8,8-dimethyl-7,9,11-trioxa-8-silanonacosa-20,23-dien-1-yl)pyrrolidin-2-yl)methanol

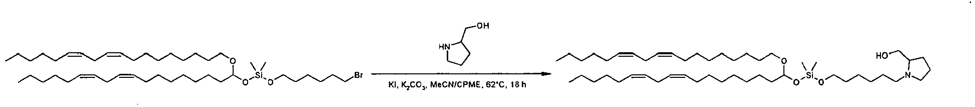

(1-((20Z,23Z)-10-((8Z,11Z)-heptadeca-8,11-dien-1-yl)-8,8-dimethyl-7,9,11-trioxa-8-silanonacosa-20,23-dien-1-yl)pyrrolidin-2-yl)methanol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (835.7 mg, 1.088 mmol, 1.1 eq.) and prolinol (100.0 mg, 0.989 mmol, 1.0 eq.) according to **General Procedure D.** The crude product was purified by flash column chromatography according to **Purification Method A** to obtain the pure product as a colourless oil, 270 mg, 0.342 mmol, 35 % yield.
¹H NMR (500 MHz, Benzene-d₆) δ 5.56-5.43 (m, 8H), δ 5.01 (m, 1H), δ 3.91-3.84 (m, 1H), δ 3.77 (t, 2H), δ 3.61-3.57 (dd, 1H), δ 3.47-3.32 (m, 2H), δ 3.01-2.96 (m, 1H), δ 2.92-2.88 (m, 4H), δ 2.62-2.53 (m, 1H), δ 2.28-2.23 (m, 1H), δ 2.17-1.17 (br. m, 58H), δ 0.92-0.86 (t, 6H), δ 0.31 (d, 6H); TOF MS ES⁺[M+H⁺]: 788.6969 m/z

### Example 62

### Synthesis of (1-((20Z,23Z)-10-((8Z,11Z)-heptadeca-8,11-dien-1-yl)-8,8-dimethyl-7,9,11-trioxa-8-silanonacosa-20,23-dien-1-yl)piperidin-2-yl)methanol

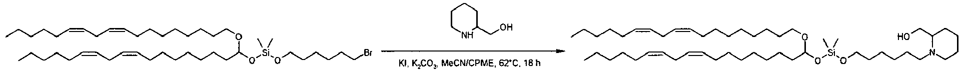

(1-((20Z,23Z)-10-((8Z,11Z)-heptadeca-8,11-dien-1-yl)-8,8-dimethyl-7,9,11-trioxa-8-silanonacosa-20,23-dien-1-yl)piperidin-2-yl)methanol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (733.6 mg, 0.955 mmol, 1.1 eq.) and piperidin-2-ylmethanol (100.0 mg, 0.868 mmol, 1.0 eq.) according to **General Procedure D.** The crude product was purified by flash column chromatography according to **Purification Method A** to obtain the pure product as a colourless oil, 82 mg, 0.102 mmol, 12 % yield.
¹H NMR (500 MHz, Benzene-d₆) δ 5.55-5.43 (m, 8H), δ 5.01 (m, 1H), δ 3.89-3.84 (m, 1H), δ 3.76 (t, 2H), δ 3.72-3.68 (dd, 1H), δ 3.45-3.36 (m, 2H), δ 2.90-2.88 (m, 4H), δ 2.83-2.78 (m, 1H), δ 2.67-2.60 (m, 1H), δ 2.19-1.24 (br. m, 60H), δ 1.17-1.07 (t, 1H), δ 0.92-0.86 (t, 6H), δ 0.29 (d, 6H); TOF MS ES⁺ [M+H⁺]: 802.7117 m/z

### Example 63

### Synthesis of (1-((20Z,23Z)-10-((8Z,11Z)-heptadeca-8,11-dien-1-yl)-8,8-dimethyl-7,9,11-trioxa-8-silanonacosa-20,23-dien-1-yl)piperidin-4-yl)methanol

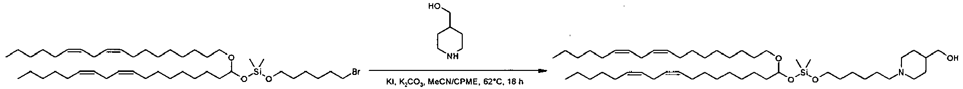

(1-((20Z,23Z)-10-((8Z,11Z)-heptadeca-8,11-dien-1-yl)-8,8-dimethyl-7,9,11-trioxa-8-silanonacosa-20,23-dien-1-yl)piperidin-4-yl)methanol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (700.3 mg, 0.912 mmol, 1.0 eq.) and piperidin-4-ylmethanol (105.0 mg, 0.912 mmol, 1.0 eq.) according to **General Procedure D.** The crude product was purified by flash column chromatography according to **Purification Method A** to obtain the pure product as a colourless oil, 294 mg, 0.366 mmol, 40 % yield.
¹H NMR (500 MHz, Benzene-d₆) δ 5.55-5.43 (m, 8H), δ 5.01 (m, 1H), δ 3.92-3.84 (m, 1H), δ 3.76 (t, 2H), δ 3.45-3.36 (m, 1H), δ 3.23 (d, 2H), δ 2.94-2.83 (m, 6H), δ 2.27-2.24 (m, 2H), δ 2.13-2.05 (m, 8H), δ 2.95-1.77 (m, 4H), δ 1.71-1.21 (br. m, 47H), δ 0.91-0.88 (t, 6H), δ 0.28 (d, 6H); TOF MS ES⁺[M+H⁺]: 802.7123 m/z

### Example 64

### Synthesis of (22Z,25Z)-4,12,12-trimethyl-10-(((9Z,12Z)-octadeca-9,12-dien-1-yl)oxy)-11,13-dioxa-4-aza-12-silahentriaconta-22,25-diene-1,2-diol

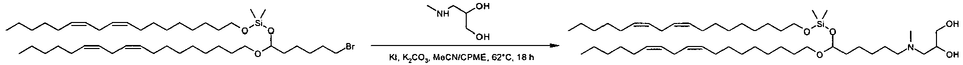

(22Z,25Z)-4,12,12-trimethyl-10-(((9Z, 12Z)-octadeca-9,12-dien-1-yl)oxy)-11,13-dioxa-4-aza-12-silahentriaconta-22,25-diene-1,2-diol was prepared from (6Z,9Z,32Z,35Z)-22-(5-bromopentyl)-20,20-dimethyl-19,21,23-trioxa-20-silahentetraconta-6,9,32,35-tetraene (708.7 mg, 0.923 mmol, 1.0 eq.) and 3-(methylamino)propane-1,2-diol (97.0 mg, 0.923 mmol, 1.0 eq.) according to **General Procedure D.** The crude product was purified by flash column chromatography according to **Purification Method A** to obtain the pure product as a colourless oil, 180 mg, 0.227 mmol, 25 % yield.
¹H NMR (500 MHz, Benzene-d₆) δ 5.55-5.43 (m, 8H), δ 5.00 (td, 1H), δ 3.89-3.84 (m, 1H), δ 3.77 (t, 2H), δ 3.68-3.57 (m, 2H), δ 3.47-3.38 (m, 2H), δ 2.91-2.88 (t, 4H), δ 2.43-2.38 (m, 1H), δ 2.25-2.17 (m, 1H), δ 2.16-2.01 (m, 8H), δ 1.97 (t, 3H), δ 1.93-1.74 (m, 2H), δ 1.73-1.58 (m, 4H), δ 1.57-1.15 (br. m, 40H), δ 0.89 (t, 6H), δ 0.28 (d, 6H); TOF MS ES⁺[M+H⁺]: 792.6909 m/z

### Example 65

### Synthesis of (20Z,23Z)-10-((8Z,11Z)-heptadeca-8,11-dien-1-yl)-N,8,8-trimethyl-N-(prop-2-yn-1-yl)-7,9,11-trioxa-8-silanonacosa-20,23-dien-1-amine

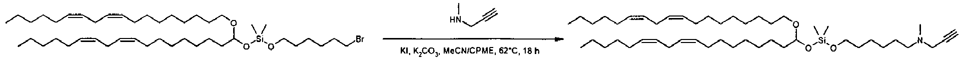

(20Z,23Z)-10-((8Z,11Z)-heptadeca-8,11-dien-1-yl)-N,8,8-trimethyl-N-(prop-2-yn-1-yl)-7,9,11-trioxa-8-silanonacosa-20,23-dien-1-amine was prepared from (20Z,23Z)-1-bromo-10-((8Z,11Z)-heptadeca-8,11-dien-1-yl)-8,8-dimethyl-7,9,11-trioxa-8-silanonacosa-20,23-diene (1500.6 mg, 1.953 mmol, 1.0 eq.) and N-methylprop-2-yn-1-amine (148.5 mg, 2.149 mmol, 1.1 eq.) according to **General Procedure D.** The crude product was purified by flash column chromatography according to **Purification Method A** to obtain the pure product as a colourless oil, 996 mg, 1.317 mmol, 67 % yield.
¹H NMR (500 MHz, CDCl₃) δ 5.42-5.29 (m, 8H), δ 4.81-4.78 (td, 1H), δ 3.69-3.62 (m, 3H), δ 3.34-3.28 (m, 3H), δ 2.77 (t, 4H), δ 2.40 (t, 2H), δ 2.30 (s, 3H), δ 2.20 (t, 1H), δ 2.07-2.02 (m, 8H), δ 1.70-1.24 (br. m, 44H), δ 0.91-0.87 (t, 6H), δ 0.16 (d, 6H); LC ESI MS [M+H⁺]: 756.704 m/z

### Example 66

### Synthesis of (4R,5S,6R)-3-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-6-(hydroxymethyl)tetrahydro-2H-pyran-2,4,5-triol

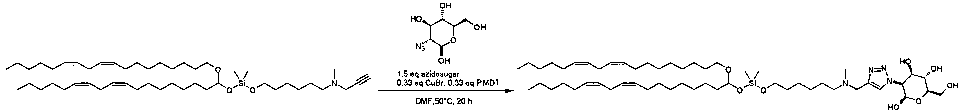

(4R,5S,6R)-3-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-6-(hydroxymethyl)tetrahydro-2H-pyran-2,4,5-triol was prepared from (20Z,23Z)-10-((8Z,11Z)-heptadeca-8,11-dien-1-yl)-N,8,8-trimethyl-N-(prop-2-yn-1-yl)-7,9,11-trioxa-8-silanonacosa-20,23-dien-1-amine (246.0 mg, 0.325 mmol, 1.0 eq.) and (3R,4R,5S,6R)-3-azido-6-(hydroxymethyl)tetrahydro-2H-pyran-2,4,5-triol (100.1 mg, 0.488 mmol, 1.5 eq.) according to **General Procedure E.** The crude product was purified by flash column chromatography according to **Purification Method** C to obtain the pure product as a colourless oil, 111 mg, 0.115 mmol, 35 % yield.
TOF MS ES⁺ [M+H⁺]: 961.7375 m/z

### Example 67

Synthesis of N-((3R,4R,5S,6R)-2-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-4,5-dihydrooy-6-(hydroxymethyl)tetrahydro-2H-pyran-3-yl)acetamide

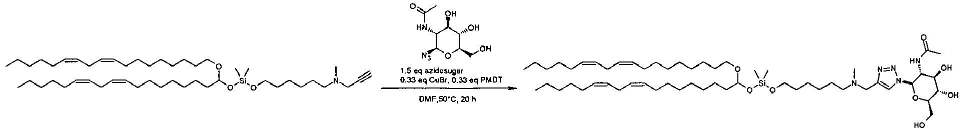

N-((3R,4R,5S,6R)-2-(4-((22Z,25Z)-12-((8Z,11Z)-heptadeca-8,11-dien-1-yl)-2,10,10-trimethyl-9,11,13-trioxa-2-aza-1H-1,2,3-triazol-1-yl)-4,5-dihydroxy-6-(hydroxymethyl)tetrahydro-2H-pyran-3-yl)acetamide was prepared from (20Z,23Z)-10-((8Z,11Z)-heptadeca-8,11-dien-1-yl)-N,8,8-trimethyl-N-(prop-2-yn-1-yl)-7,9,11-trioxa-8-silanonacosa-20,23-dien-1-amine (300.0 mg, 0.397 mmol, 1.0 eq.) and N-((2R,3R,4R,5S,6R)-2-azido-4,5-dihydroxy-6-(hydroxymethyl)tetrahydro-2H-pyran-3-yl)acetamide (146.5 mg, 0.595 mmol, 1.5 eq.) according to **General Procedure E.** The crude product was purified by flash column chromatography according to **Purification Method C** to obtain the pure product as a colourless oil, 112 mg, 0.112 mmol, 28 % yield.
TOF MS ES⁺ [M+H⁺]: 1002.7641 m/z

### Example 68

### Synthesis of 1,4-bis((20Z,23Z)-10-((8Z,11Z)-heptadeca-8,11-dien-1-yl)-8,8-dimethyl-7,9,11-trioxa-8-silanonacosa-20,23-dien-1-yl)piperazine

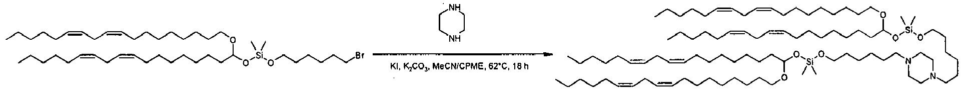

1,4-bis((20Z,23Z)-10-((8Z, 11Z)-heptadeca-8,11-dien-1-yl)-8,8-dimethyl-7,9,11-trioxa-8-silanonacosa-20,23-dien-1-yl)piperazine was prepared from (20Z,23Z)-1-bromo-10-((8Z,11Z)-heptadeca-8,11-dien-1-yl)-8,8-dimethyl-7,9,11-trioxa-8-silanonacosa-20,23-diene (1049.6 mg, 1.366 mmol, 2.2 eq.) and piperazine (53.5 mg, 0.621 mmol, 1.0 eq.) according to **General Procedure D.** The crude product was purified by flash column chromatography according to **Purification Method A** to obtain the pure product as a colourless oil, 181 mg, 0.124 mmol, 20 % yield.
¹H NMR (500 MHz, CDCl₃) δ 5.64-5.53 (m, 16H), δ 5.10 (m, 2H), δ 3.99-3.94 (m, 2H), δ 3.86 (t, 4H), δ 3.55-3.46 (m, 4H), δ 3.00-2.98 (m, 8H), δ 2.57 (br. s, 6H) δ 2.39 (t, 4H), δ 2.24-2.15 (m, 16H), δ 2.05-1.88 (m, 4H), δ 1.83-1.27 (br. m, 84H), δ 1.02-0.98 (t, 12H), δ 0.38 (d, 12H)

### Example 69

### Synthesis of (26Z,29Z)-16-((8Z,11Z)-heptadeca-8,11-dien-1-yl)-3-((20Z,23Z)-10-((8Z,11Z)-heptadeca-8,11-dien-1-yl)-8,8-dimethyl-7,9,11-trioxa-8-silanonacosa-20,23-dien-1-yl)-6-(2-hydroxyethyl)-14,14-dimethyl-13,15,17-trioxa-3,6-diaza-14-silapentatriaconta-26,29-dien-1-ol

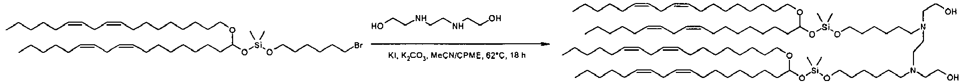

(26Z,29Z)-16-((8Z,11Z)-heptadeca-8,11-dien-1-yl)-3-((20Z,23Z)-10-((8Z,11Z)-heptadeca-8,11-dien-1-yl)-8,8-dimethyl-7,9,11-trioxa-8-silanonacosa-20,23-dien-1-yl)-6-(2-hydroxyethyl)-14,14-dimethyl-13,15,17-trioxa-3,6-diaza-14-silapentatriaconta-26,29-dien-1-ol was prepared from (20Z,23Z)-1-bromo-10-((8Z,11Z)-heptadeca-8,11-dien-1-yl)-8,8-dimethyl-7,9,11-trioxa-8-silanonacosa-20,23-diene (1003.0 mg, 1.306 mmol, 2.2 eq.) and 2,2'-(ethane-1,2-diylbis(azanediyl))bis(ethan-1-ol) (88.0 mg, 0.594 mmol, 1.0 eq.) according to **General Procedure D.** The crude product was purified by flash column chromatography according to **Purification Method** A to obtain the pure product as a colourless oil, 131 mg, 0.086 mmol, 14 % yield.
¹H NMR (500 MHz, Benzene-d₆) δ 5.55-5.44 (m, 16H), δ 5.01 (m, 2H), δ 3.92-3.85 (m, 2H), δ 3.79 (t, 4H), δ 3.59 (m, 4H), δ 3.46-3.39 (m, 2H), δ 2.92-2.88 (m, 8H), δ 2.39-2.32 (m, 12H), δ 2.16-2.05 (m, 16H), δ 1.97-1.17 (br. m, 88H), δ 0.92-0.88 (t, 12H), δ 0.30 (d, 12H)

### Preparation and characterisation of lipid nanoparticles, transfection and viability studies

The synthesized lipids were tested in term of *in vitro* transfection efficacy and toxicity on HeLa cells. In order to perform the mentioned biological tests, appropriate LNPs had to be formulated from the synthesized lipids. The particle size, PDI (polydispersity index) of resulting LNPs were determined by means of Dynamic Light Scattering technique (DLS). GFP-mRNA was transfected into the cells and in parallel with the transfection experiments, toxicity of the investigated LNPs was determined by MTT assay. The apparent pKa in LNPs was determined by using TNS titration based on the method described: Angew Chem Int Ed Engl. 2012, 51 (34), 8529-8533, doi: 10.1002/anie.201203263).

### LNP formulation

*Solutions required:*
0.33 ug/uL mRNA solution in DEPC-treated water (RiboPro, Off-The-Shelf mRNA; eGFP with Cap1)
0.04 ug/uL mRNA solution in DEPC-treated water (RiboPro, Off-The-Shelf mRNA; eGFP with Cap1)

**Table 1: Lipid formulation solution in absolute ethanol**

| Reagent | mol/L |
|---|---|
| Tested lipid | 7.36E-03 |
| ALC-0159 (CAS# 1849616-42-7) | 2.54E-04 |
| DSPC (CAS# 4539-70-2) | 1.49E-03 |
| Chol (CAS# 57-88-5) | 7.36E-03 |
| DOTAP (CAS# 132172-61-3) | 7.36E-03 |

MEM (Sigma, cat# M5650-500mL)
Dilutor: 10 mL MEM + 0,036 mL ethanol
Lipofectamine solution (Thermofisher/Invitrogen, cat# 11668-019, 1 mg/mL solution)

### Procedure:

1. Negative control:
   a) Place 360 uL of Dilutor into an Eppendorf
2. Tested lipid formulation:
   In 1.5 mL eppendorf:
   a) Place 2.9 uL of 0.33 ug/uL mRNA solution into Eppendorf tube
   b) Add 1.94 µL of supplied ethanolic solution of tested lipid and immediately mix by working pipette up-and-down several times (work the solution up and down several times before transferring to saturate pipette tip with ethanol vapors)
   c) Incubate for 15 minutes.
   d) Add 536 µL of MEM. Mix the solution by vortexing.
   e) Incubate for 30 minutes.
3. Lipofectamine-50 control preparation (based on manufacturer's instructions)
   a) Place 13,5 µL of 0.04 ug/µL mRNA solution into 1.5 mL sterile Eppendorf tube.
   b) Add 527 µL of MEM
   c) Add 2,16 µL of Lipofectamine stock solution. Vortex.
   d) Incubate 15 min
4. Lipofectamine-6 control preparation:
   a) Place 45 µL Lipofectamine-50 solution into 1.5 mL sterile Eppendorf tube.
   b) Add 315 µL of MEM. Vortex.
   c) Incubate 15 min.

### Size and size distribution measurements with dynamic light scattering

1. Transfer 100 uL of freshly prepared LNP formulation in DLS micro cuvette.
2. Place the formulation into Zetasizer pre-heated to 37 C.
3. Equilibrate the sample for 5 minutes.
4. Perform the size measurement in triplicate (settings: position and gain auto).
5. For QC examine correlation curve to have no disturbances. Report Z-average and PDI based on cumulants fit.

### Transfection efficiency with GFP-mRNA

### Solutions required:

MEM (Sigma, cat# M5650-500mL)
Fetal Bovine Serum, FBS (Sigma, cat# F7524-500mL)
Solutions of the formulations (see above)
Dilutor: 10 mL MEM + 0,036 mL ethanol
Phosphate buffered saline, PBS

### Procedure:

1. Fill the edge wells of a 96-well plate with 100 µL media.
2. Plate 10,000 HeLa cells per well in 96 well plate. Do not use edge wells. Total 60 wells should be seeded. Incubate in MEM+10% FBS at 37°C in a humidified atmosphere with 5% CO₂ for 24h.
3. Examine under the microscope. Confluency must be at or nearly 80%.
4. Gently remove the media (aspirate). Place 50 µL of the following formulations:
   a) 6 wells with dilutor - negative control
   b) 6 wells with Lipofectamine-50 - positive control.
   c) 6 wells with Lipofectamine-6 - secondary positive control
   d) 6 wells per formulations - test samples.
5. Incubate plate for 1 hours at 37°C.
6. Add 50 µL of MEM+20% FBS (which will become 10% upon dilution).
7. Place plates into the incubator at 37°C in a humidified atmosphere with 5% CO₂ for 23 h (total 24 hours of incubation).
8. After 24 h, aspirate the media without disturbing the cells. Gently wash cells with 100 µL of warm PBS. Aspirate PBS.
9. Add 100 µL of fresh PBS into washed cells and read GFP fluorescence on the plate reader.
10. This can be followed with viability assessment with the MTT assay.

### Cell viability assessment with MTT assay

### Solutions required:

MEM (Sigma, cat# M5650-500mL)
Fetal Bovine Serum, FBS (Sigma, cat# F7524-500mL)
Thiazolyl Blue Tetrazolium Bromide, MTT (TCI Chemicals, cat# D0801-5G)
Solutions of the formulations (see "Preparation of GFP mRNA loaded LNP" protocol)
Dilutor: 10 mL MEM + 0,036 mL ethanol
Phosphate buffered saline, PBS

### Procedure:

1. Plate 10,000 HeLa cells per well in 96 well plate. Do not use edge wells. Total 60 wells should be seeded. Incubate in MEM+10%FBS at 37°C in a humidified atmosphere with 5% CO₂ for 24 h.
2. Examine under the microscope. Confluency must be at or nearly 80%.
3. Gently remove the media (aspirate). Place 50 µL of the following formulations:
   a) 6 wells with dilutor - negative control
   b) 6 wells with Lipofectamine-50 - positive control.
   c) 6 wells with Lipofectamine-6 - secondary positive control
   d) 6 wells per formulations - test samples.
4. Incubate plate for 1 hours at 37 °C.
5. Add 50 uL of MEM+20% FBS (which will become 10% upon dilution).
6. Place plates into the incubator at 37 C for 23 hours (total 24 hours of incubation).
7. Prepare MTT solution at 1 mg/mL in sterile PBS. Filter-sterilize the solution using a 0.22 µm) filter and protect it from light. This solution needs to be prepared fresh before the assay.
8. After the treatment duration, remove the treatment medium carefully from each well.
   a. Add 100 µL of the diluted MTT solution to each well.
   b. Incubate the plate for 2-4 hours at 37°C in a humidified atmosphere with 5% CO₂.
9. After incubation, carefully remove the MTT solution from each well without disturbing the formazan crystals. Add 100-150 µL of a solubilization solution (isopropanol with 0.04 M HCl) to each well to dissolve the formazan crystals. Gently pipette up and down or shake the plate on an orbital shaker for 5-10 minutes to ensure complete solubilization.
10. Measure the absorbance of each well at 570 nm using a microplate reader. Measure the absorbance at a reference wavelength of 650 nm to subtract background absorbance.
11. Calculate the relative cell viability (%) by comparing with blank (untreated culture).

Results of the above experiments using lipids according to the invention as well as the reference compound are summarized in Table 2 below.

**Table 2: LNP characterisation, transfection and toxicity**

| **Example** | **Size (nm)** | **PDI** | **pKa** | **Transfection 89 ng mRNA/well** | **Cell Viability %** |
|---|---|---|---|---|---|
| 46 | 180 | 0.128 | 7.73 | 93448.3 | 51.5 |
| 54 | 185 | 0.092 | 7.21 | 78375.2 | 40.2 |
| 56 | | | | 50019.2 | 52.1 |
| ALC-0315 ref. | | | | 15892.1 | |

### Conclusion

Thanks to the innovative modular approach leading to this type of novel ionizable cationic lipid library as described in the present invention, an extensive landscape of chemical space becomes readily accessible. By utilizing commercially available starting materials and implementing proprietary borane catalysts (as described in WO 2022/129966), compounds of formula (I) can be synthesized.

Based on the preliminary results, the selected lipids were capable for the formation of LNPs with good particle size and PDI. Moreover, these lipids have been shown to deliver GFP mRNA into cells with moderate cellular toxicity. Remarkably, the exemplified lipids exhibited higher transfection efficacy under these conditions compared to the reference compound ALC-0315.

## Claims

1. A compound of formula (1) 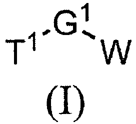 or its salt or stereoisomer,
wherein:
G¹ is
unsubstituted C₂-C₁₂ alkylene,
-(CH₂)ₓ-CH=CH-(CH₂)_{y}-, wherein x is an integer selected from 1 to 9, y is an integer selected from 1 to 9, and the sum of x+y is an integer selected from 2 to 10, or
-(CH₂)_{w}-X-(CH₂)_{z}-, wherein w is an integer selected from 1 to 10, z is an integer selected from 2 to 10, the sum of w+z is an integer selected from 3 to 11, wherein -(CH₂)_{z}- is attached to N, and X is selected from O, S, -S-S-, SO and SO₂;
T' is 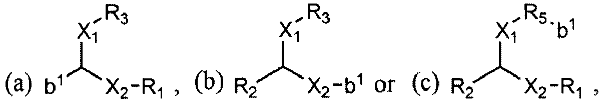
wherein b¹ is a bond to G¹,
X₁ and X₂ are the same or different and each independently represents O or S,
R₁ is linear C₁-C₃₂ alkyl, branched C₃-C₃₂ alkyl, in both cases the chain optionally containing one S, SO, SO₂, -S-S-, O, or Si(R^{a})₂, wherein R^{a} is C₁-C₆ alkyl, at any position in the carbon chain with the proviso that the heteroatom is not in the alpha or omega position of the carbon chain, linear or branched C₃-C₃₂ alkenyl containing one or more double bonds with the proviso that there is at least one -CH₂- group between the double bond and X₂,
R₁ is CH₃-(CH₂)_{d}-V₁-(CH₂)ₑ-, wherein d is an integer selected from 1 to 20, e is an integer selected from 1 to 20, and the sum of d+e is an integer selected from 2 to 29, and V₁ is a C₃-C₆ cycloalkylene, or
R₁ is V₂-(CH₂)_{f}-, wherein f is an integer selected from 1 to 30, and V₂ is a C₃-C₆ cycloalkyl, or
R₁ is 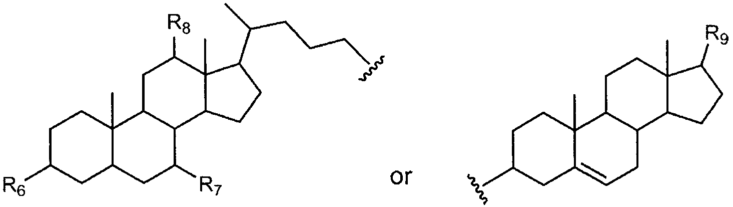
wherein R₆, R₇, R₈ are the same or different and each is independently H, OH, -C₁-C₆ alkoxy, -O-C(O)-C₁-C₆ alkyl or fluorine,
R₉ is linear or branched C₃-C₁₂ alkyl or C₃-C₁₂ alkenyl containing one double bond, or
R₁ is 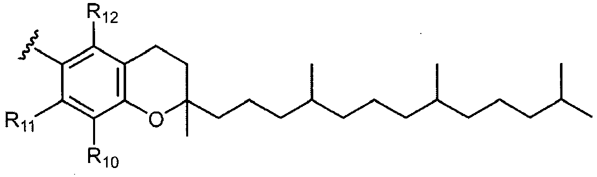 wherein R₁₀, R₁₁, R₁₂ are the same or different and each is independently H, F or methyl, or
R₁ is 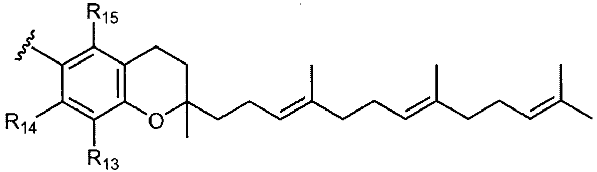 wherein R₁₃, R₁₄, R₁₅ are the same or different and each is independently H, F or methyl, or
R₁ is 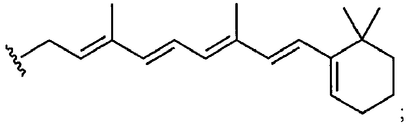
R₂ is linear C₁-C₃₂ alkyl, branched C₃-C₃₂ alkyl, in both cases optionally containing one S, SO, SO₂, -S-S-, O, or Si(R^{b})₂, wherein R^{b} is C₁-C₆ alkyl, at any position in the carbon chain with the proviso that the heteroatom is not in the alpha or omega position of the carbon chain, linear or branched C₃-C₃₂ alkenyl containing one or more double bonds with the proviso that there is at least one -CH₂- group between the double bond and the carbon linking X₁ and X₂, or
R₂ is CH₃-(CH₂)_{g}-V₃-(CH₂)ₕ-, wherein g is an integer selected from 1 to 20, h is an integer selected from 1 to 20, and the sum of g+h is an integer selected from 2 to 28, wherein V₃ is a C₃-C₆ cycloalkylene, or
R₂ is V₄-(CH₂)ₜ-, wherein t is an integer selected from 1 to 29, wherein V₃ is a C₃-C₆ cycloalkyl, or
R₂ is 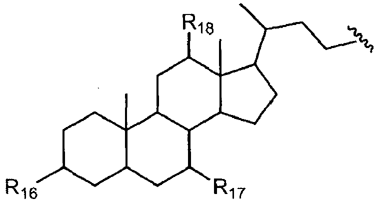 wherein R₁₆, R₁₇, R₁₈ are the same or different and each is independently H, OH, -C₁-C₆ alkoxy, -O-C(O)-C₁-C₆ alkyl or fluorine; or
R₁ is connected to R₃, wherein R₃ has the meaning as defined above, wherein Y₁, X₃ and b₂ are as defined above and R₄ forms together with R₁ an alkylene or alkenylene containing one double bond, and thus R₁ and R₃ form together with the intervening atoms of (a) a 5-32-membered ring, or
R₂ is connected to R₃, wherein R₃ has the meaning as defined above, wherein Y₁, X₃ and b₂ are as defined above and R₄ forms together with R₂ an alkylene or alkenylene containing one double bond, and thus R₂ and R₃ form together with the intervening atoms of (b) a 5-32-membered ring, wherein the carbon atom of R₂ at the first or second position numbered from that carbon atom which is attached to the carbon linking X₁ and X₂, can be optionally replaced by O or S heteroatom, or
R₂ is connected to R₁ and R₂ forms together with R₁ an alkylene or alkenylene containing one double bond and thus R₂ and R₁ form together with the intervening atoms of (c) a 5-32-membered ring, wherein the carbon atom of R₂ at the first or second position numbered from that carbon atom which is attached to the carbon linking X₁ and X₂, can be optionally replaced by O or S heteroatom;
R₃ is 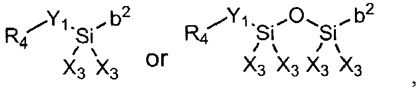
R₅-b¹ is 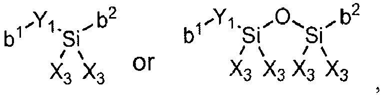 wherein
b² is a link to X₁,
Y₁ is -O- or -CH₂- or -O-CH₂-CH₂- wherein -CH₂- is attached to Si,
each X₃ is independently C₁-C₄ alkyl or C₁-C₄ alkoxy,
R₄ is linear C₁-C₃₂ alkyl, branched C₃-C₃₂ alkyl, in both cases the chain optionally containing one S, SO, SO₂, -S-S-, O, or Si(R^{c})₂, wherein R^{c} is C₁-C₆ alkyl, at any position in the carbon chain with the proviso that the heteroatom is not in the alpha or omega position of the carbon chain, linear or branched C₃-C₃₂ alkenyl containing one or more double bonds with the proviso that there is at least one -CH₂- group between the double bond and Y₁, or
R₄ is 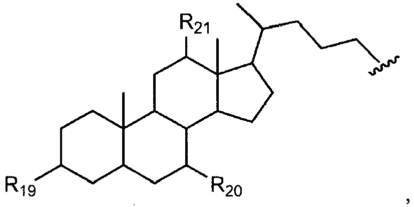 wherein R₁₉. R₂₀, R₂, are the same or different and each is independently H, OH, -C₁-C₆ alkoxy, -O-C(O)-C₁-C₆ alkyl or fluorine, or
R₄ is CH₃-(CH₂)ᵤ-V₅-(CH₂)ₛ-, wherein u is an integer selected from 1 to 20, s is an integer selected from 1 to 20, and the sum of u+s is an integer selected from 2 to 29, and V₅ is a C₃-C₆ carbocycle, or
R₄ is V₆-(CH₂)ᵢ-, wherein i is an integer selected from 1 to 30, and V₆ is a C₃-C₆ carbocycle, or
R₄ is 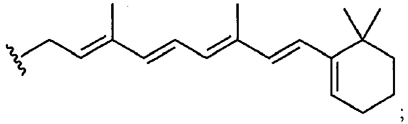
W is 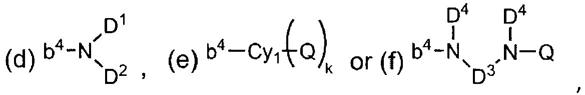 wherein
b⁴ is a bond to G¹;
D¹ and D² are independently selected from the following:
linear C₁-C₈ alkyl, branched C₃-C₈ alkyl, 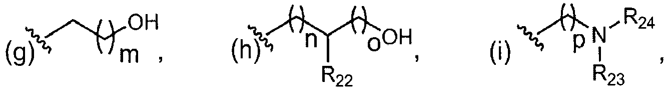 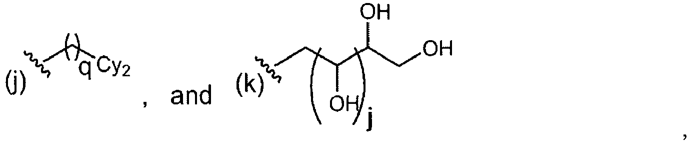 wherein
R₂₂ is C₁-C₆ alkyl, cyclopentyl, cyclohexyl, hydroxyl, hydroxymethyl, hydroxyethyl, phenyl, benzyl, 4-hydroxy benzyl, 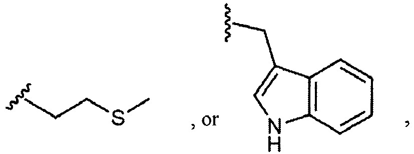
R₂₃ and R₂₄ are independently selected from H and C₁-C₆ alkyl, or one of R₂₃ and R₂₄ is H and the other forms together with the joint N atom a guanidyl group;
m is an integer selected from 1 to 6,
n is an integer selected from 0 to 6,
o is an integer selected from 0 to 6,
p is an integer selected from 2 to 6,
q is an integer selected from 0 to 6,
j is an integer selected from 1 to 4,
Cy₂ is a C₃-C₆ cycloalkyl optionally substituted by one or more -OH, or
Cy₂ is a pyranose, furanose ring, which can be linked via any of its OH group, wherein the pyranose or furanose ring is optionally substituted by a -NH-CO-CH₃ group, adenine, guanine, uracil, cytosine, thymine, a mono- or oligosaccharide, or by a 4-, 5-, 6- or 7-membered heterocyclic ring containing 1, 2, 3 or 4 heteroatoms selected from N, O or S, or
Cy₂ is a 4-, 5-, 6- or 7-membered heterocyclic ring containing 1, 2, 3 or 4 heteroatoms selected from O, N or S, wherein the heterocyclic ring is optionally substituted by R₂₅,
wherein R₂₅ represents C₁-C₆ alkyl, an arginine containing peptide, a pyranose or furanose ring attached by any of their ring-carbon atoms to the 4-, 5-, 6- or 7-membered heterocyclic ring, wherein the pyranose or furanose ring is optionally substituted by a -NH-CO-CH₃ group, adenine, guanine, uracil, cytosine, thymine or a mono- or oligosaccharide,
R₂₅ can be a group selected from (g) or (h), wherein m, n, o and R₂₂ are as defined above,
or
R₂₅ can be a 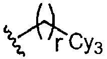 group wherein r is an integer selected from 1 to 4, and Cy₃ is a 4-, 5-, 6- or 7-membered heterocyclic ring containing 1, 2, 3 or 4 heteroatoms selected from O, N and S, optionally substituted by C₁-C₆ alkyl;
Cy₁ is a 4-, 5-, 6- or 7-membered heterocyclic ring containing at least one N atom which is attached to G¹ via b⁴, and optionally containing 1, 2 or 3 further heteroatoms selected from O, N and S,
wherein the heterocyclic ring is optionally substituted with Q, which is linked to the heterocyclic ring via any N or C atom of the heterocyclic ring, with the proviso that the N atom which is substituted with Q is not adjacent to that N atom that is attached to G¹ via b⁴,
k is an integer selected from 0 and 1;
Q is T²-G²
wherein
G² is defined as G¹ above, where G¹ and G² may be identical or different;
T² is defined as T¹ above and wherein T' and T² may be identical or different; or
Q is defined as D¹ above;
D³ is C₂-C₈ alkylene;
D⁴ is selected from (g) above, wherein m is defined as above.

2. The compound of claim 1, wherein G¹ linear C₂-C₁₂ alkylene, preferably C₄-C₉ alkylene.

3. The compound of claim 1 or 2, wherein R₁ is linear or branched C₁, C₂, C₃, C₄, C₆, C₇, C₈, C₉, C₁₀, C₁₁, C₁₂, C₁₃ C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂ alkyl and/or R₂ is linear or branched C₁, C₂, C₃, C₄, C₆, C₇, C₈, C₉, C₁₀, C₁₁, C₁₂, C₁₃ C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂ alkyl preferably R₁ is linear or branched C₁₂, C₁₄, C₁₅, C₁₆, C₁₇, C₁₈, C₂₀, C₂₂, C₂₄, C₂₆, C₂₈, C₃₀, C₃₂ alkyl and/or R₂ is linear or branched C₁₁, C₁₃, C₁₄, C₁₅, C₁₆, C₁₇, C₁₉, C₂₁, C₂₃, C₂₅, C₂₇, C₂₉, C₃₁ alkyl.

4. The compound of claim 1 or 2, wherein R₁ is linear or branched C₃, C₄, C₆, C₇, C₈, C₉, C₁₀, C₁₁, C₁₂, C₁₃ C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂ alkenyl and/or R₂ is linear or branched C₃, C₄, C₆, C₇, C₈, C₉, C₁₀, C₁₁, C₁₂, C₁₃ C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂ alkenyl preferably R₁ is linear or branched C₉, C₁₂, C₁₄, C₁₅, C₁₆, C₁₈, C₂₀, C₂₆ alkenyl and/or R₂ is linear or branched C₈, C₁₁, C₁₃, C₁₅, C₁₇, C₁₉, C₂₅ alkenyl.

5. The compound of claim 1 or 2, wherein R₁ and/or R₄ is selected from the following:

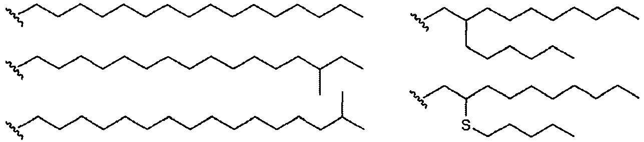

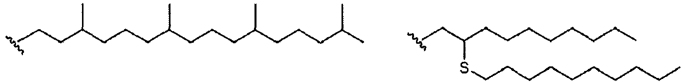

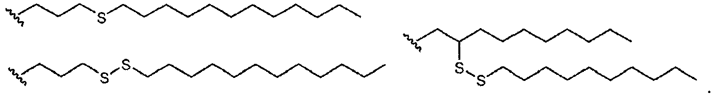

6. The compound of claim 1 or 2, wherein R₁ and/or R₄ is selected from the following:

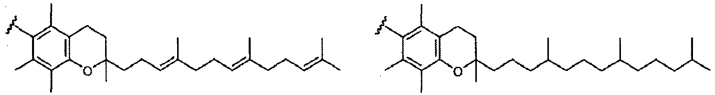

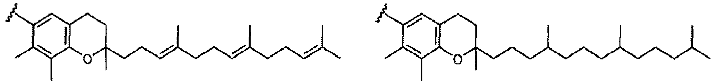

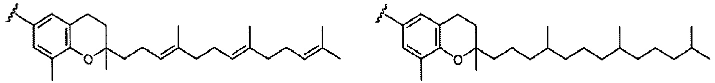

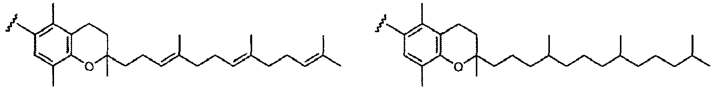

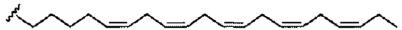

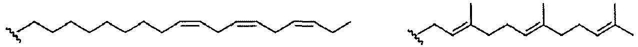

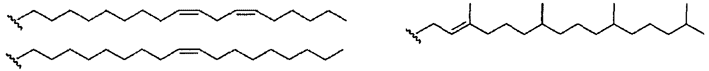

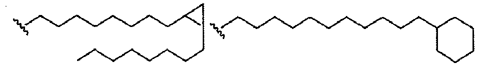

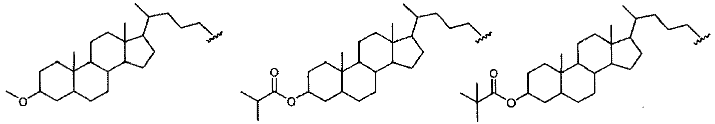

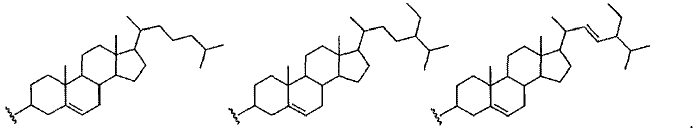

7. The compound according to any one of claims 1, 2, 5 or 6, wherein R₂ is selected from the following:

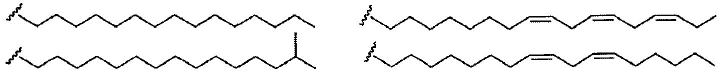

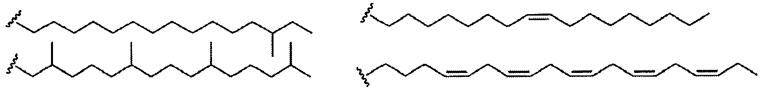

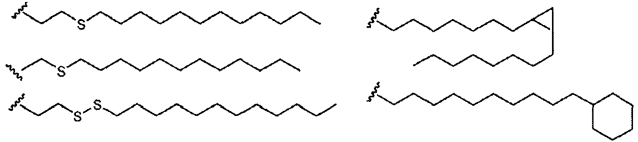

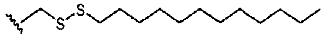

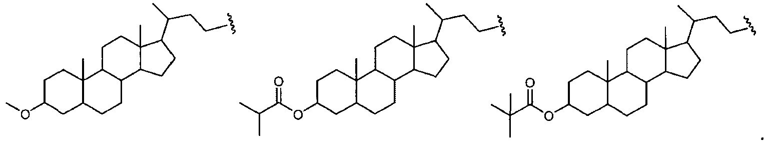

8. The compound of claim 1 or 2, wherein T¹-G¹ is selected from the following:

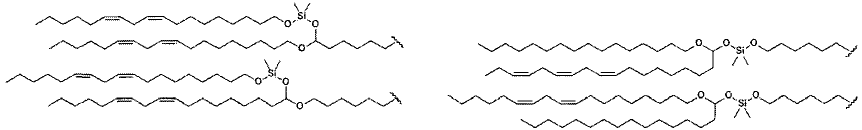

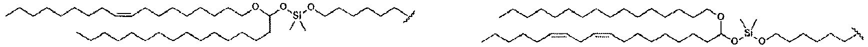

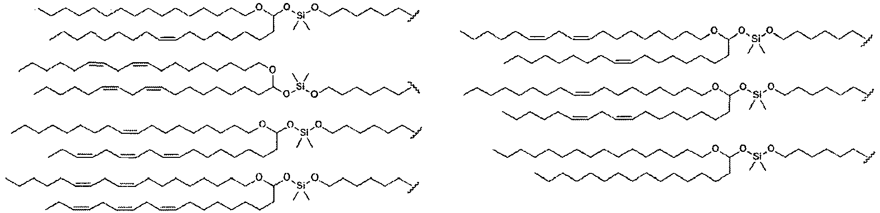

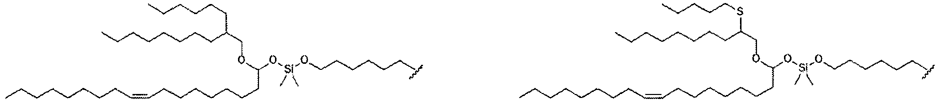

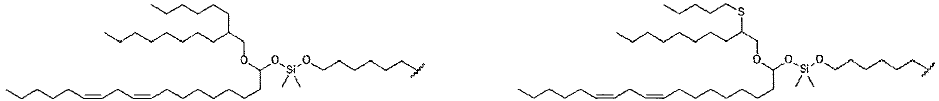

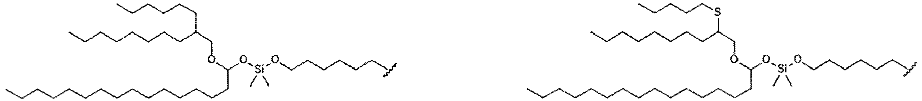

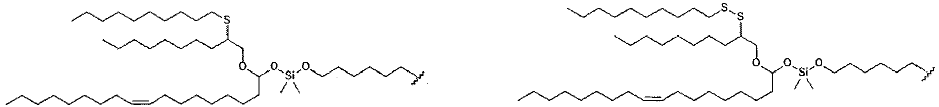

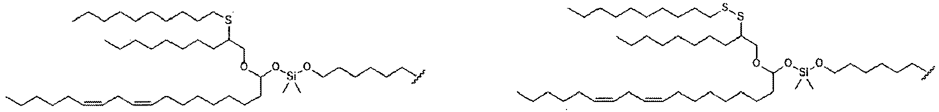

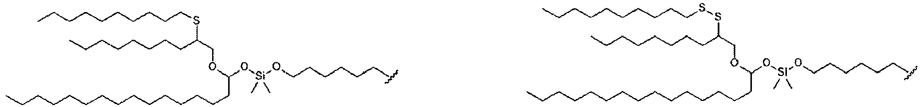

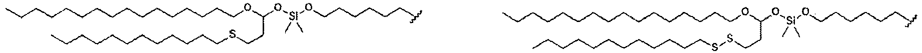

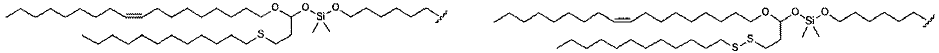

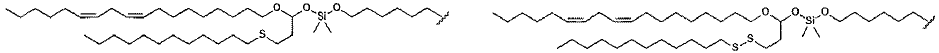

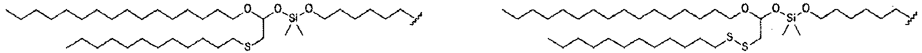

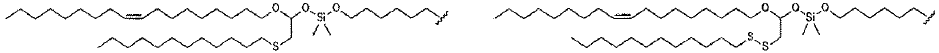

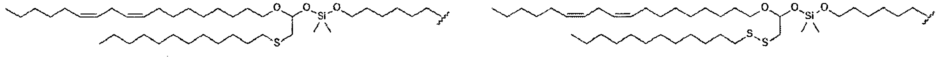

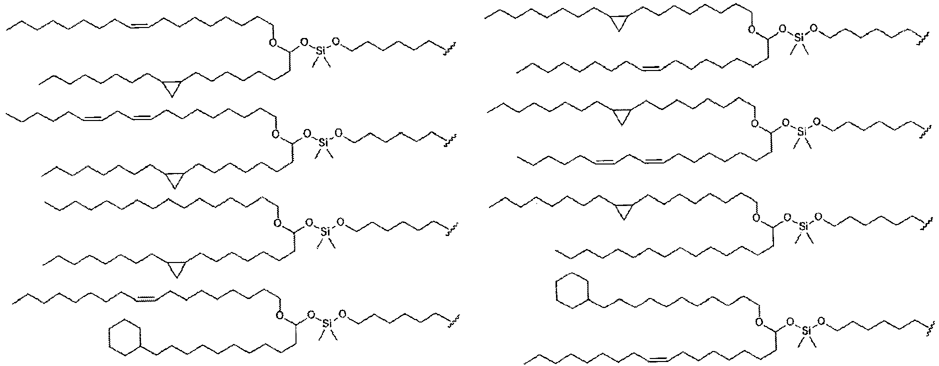

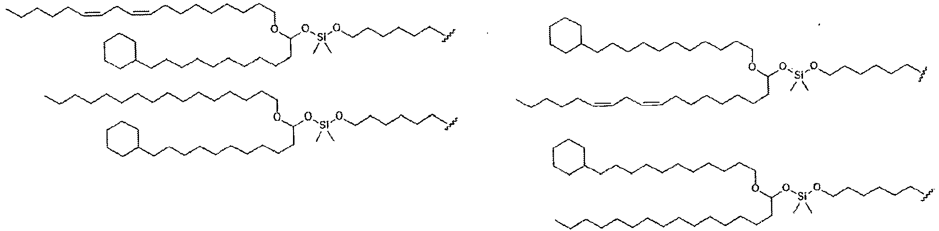

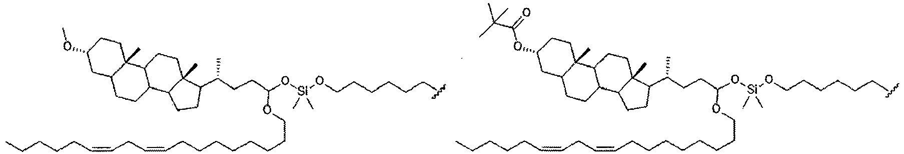

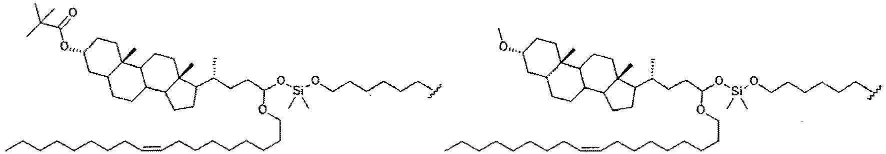

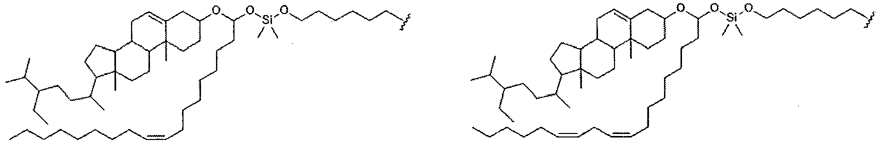

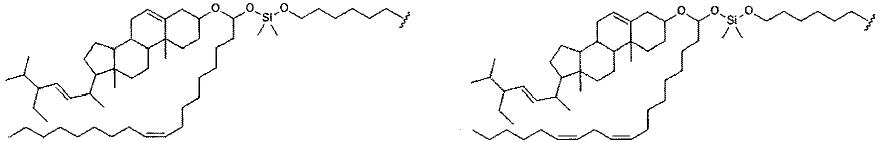

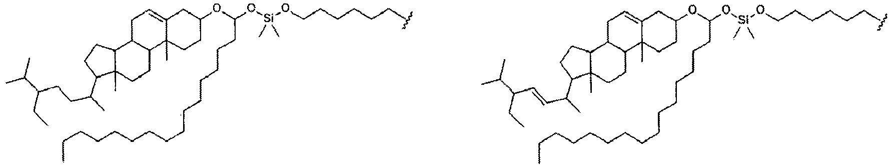

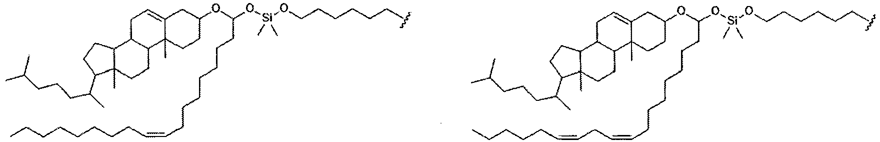

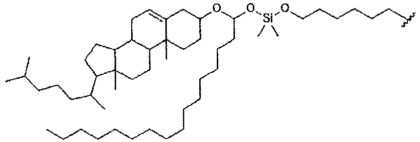

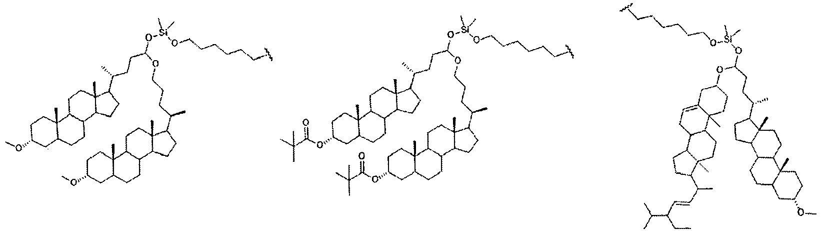

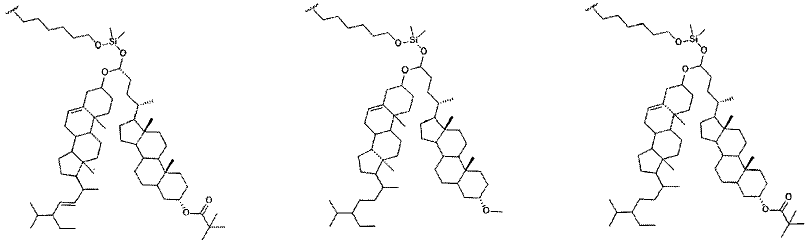

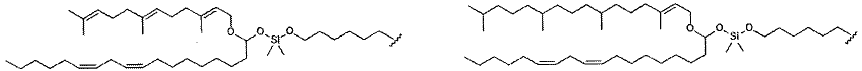

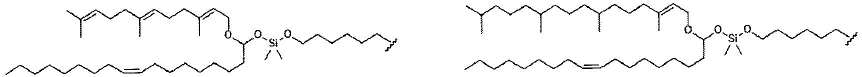

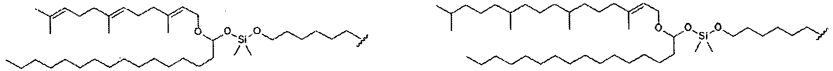

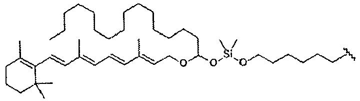

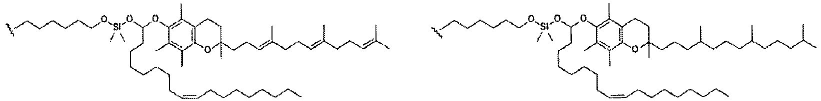

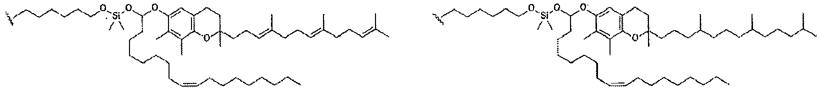

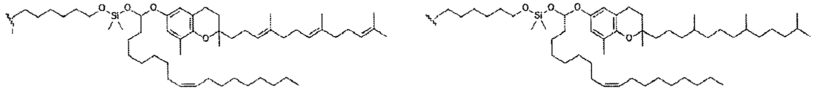

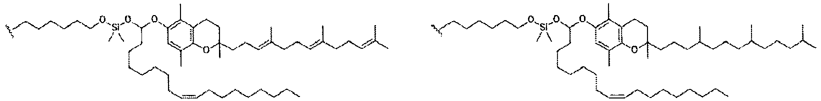

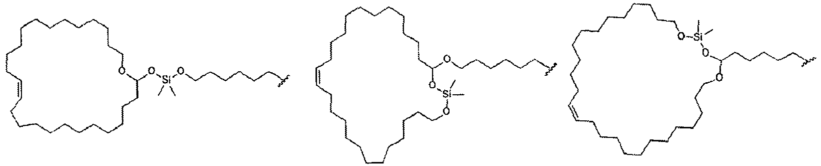

9. The compound according to any one of claims 1 to 8, wherein W is (d).

10. The compound according to any one of claims 1 to 8 wherein W is selected from the following: 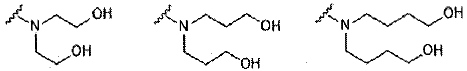 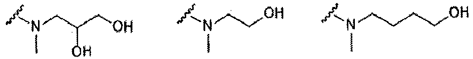 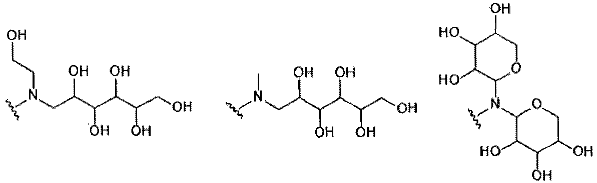 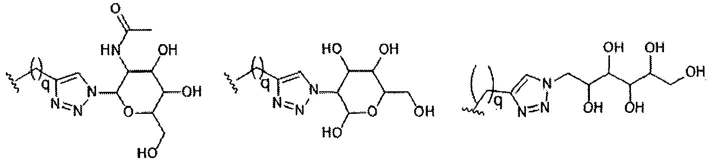 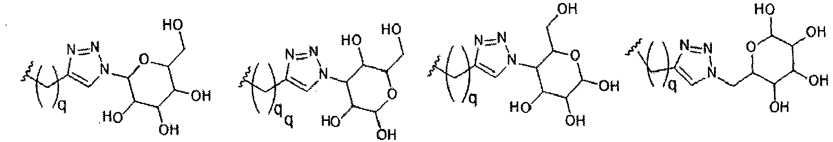 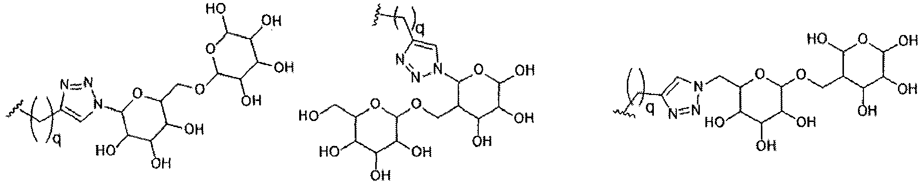 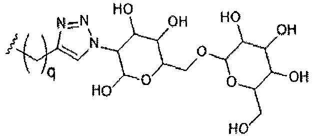 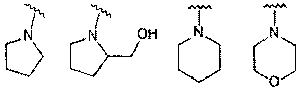 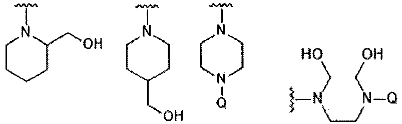 , wherein Q is defined above.

11. The compound of claim 1, which is selected from the following:
| Structure | IUPAC name |
|---|---|
| 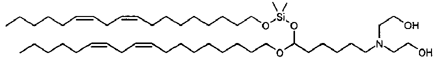 | (21Z,24Z)-3-(2-hydroxyethyl)-11,11-dimethyl-9-(((9Z,12Z)-octadeca-9,12-dien-1-yl)oxy)-10,12-dioxa-3-aza-11-silatriaconta-21,24-dien-1-ol |
| 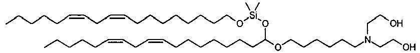 | (23Z,26Z)-11-((8Z,11Z)-heptadeca-8,11-dien-1-yl)-3-(2-hydroxyethyl)-13,13-dimethyl-10,12,14-trioxa-3-aza-13-siladotriaconta-23,26-dien-l-ol |
| 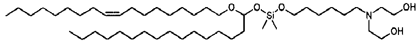 | (Z)-3-(2-hydroxyethyl)-11,11-dimethyl-13-pentadecyl-10,12,14-trioxa-3-aza-11-siladotriacont-23-en-1-ol |
| 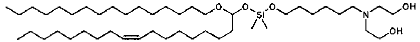 | (Z)-13-(heptadec-8-en-1-yl)-3-(2-hydroxyethyl)-11,11-dimethyl-10,12,14-trioxa-3-aza-11-silatriacontan-l-ol |
| 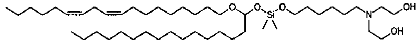 | (23Z,26Z)-3-(2-hydroxyethyl)-11,11-dimethyl-13-pentadecyl-10,12,14-trioxa-3-aza-11-siladotriaconta-23,26-dien-1-ol |
| 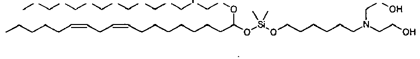 | 13-((8Z,11Z)-heptadeca-8,11-dien-1-yl)-3-(2-hydroxyethyl)-11,11-dimethyl-10,12,14-trioxa-3-aza-11-silatriacontan-1-ol |
| 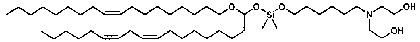 | (Z)-13-((8Z,11Z)-heptadeca-8,11-dien-1-yl)-3-(2-hydroxyethyl)-11,11-dimethyl-10,12,14-trioxa-3-aza-11-siladotriacont-23-en-1-ol |
| 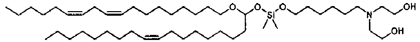 | (23Z,26Z)-13-((Z)-heptadec-8-en-1-yl)-3-(2-hydroxyethyl)-11,11-dimethyl-10,12,14-trioxa-3-aza-l 1-siladotriaconta-23,26-dien-l-ol |
| 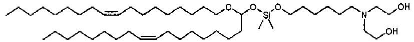 | (Z)-13-((Z)-heptadec-8-en-1-yl)-3-(2-hydroxyethyl)-11,11-dimethyl-10,12,14-trioxa-3-aza-11-siladotriacont-23-en-1-ol |
| 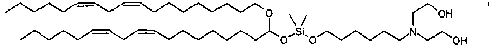 | (23Z,26Z)-13-((8Z,11Z)-heptadeca-8,11-dien-1-yl)-3-(2-hydroxyethyl)-11,11-dimethyl-10,12,14-trioxa-3-aza-11-siladotriaconta-23,26-dien-1-ol |
| 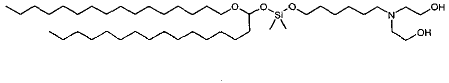 | 3-(2-hydroxyethyl)-11,11-dimethyl-13-pentadecyl-10,12,14-trioxa-3-aza-11-silatriacontan-l-ol |
| 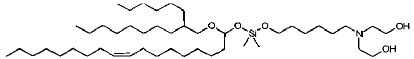 | (Z)-13-(heptadec-8-en-1-yl)-16-hexyl-3-(2-hydroxyethyl)-11,11-dimethyl-10,12,14-trioxa-3-aza-11-silatetracosan-1-ol |
| 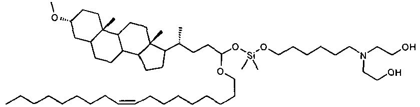 | (Z)-3-(2-hydroxyethyl)-13-((3R)-3-((3R,10S,13R)-3-methoxy-10,13-dimethylhexadecahydro-1H-cyclopenta[a]phenanthren-17-yl)butyl)-11,11-dimethyl-10,12,14-trioxa-3-aza-11-siladotriacont-23-en-1-ol |
| 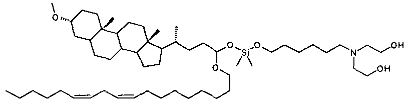 | (23Z,26Z)-3-(2-hydroxyethyl)-13-((3R)-3-((3R,10S,13R)-3-methoxy-10,13-dimethylhexadecahydro-1H-cyclopenta[a]phenanthren-17-yl)butyl)-11,11-dimethyl-10,12,14-trioxa-3-aza-11-siladotriaconta-23,26-dien-1-ol |
| 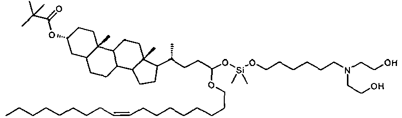 | (3R,10S,13R)-17-((16R)-1-hydroxy-3-(2-hydroxyethyl)-11,11-dimethyl-13-(((Z)-octadec-9-en-1-yl)oxy)-10,12-dioxa-3-aza-11-silaheptadecan-16-yl)-10,13-dimethylhexadecahydro-1H-cyclopenta[a]phenanthren-3-yl pivalate |
| 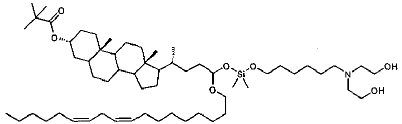 | (3R,10S,13R)-17-((16R)-1-hydroxy-3-(2-hydroxyethyl)-11,11-dimethyl-13-(((9Z,12Z)-octadeca-9,12-dien-1-yl)oxy)-10,12-dioxa-3-aza-11-silaheptadecan-16-yl)-10,13-dimethylhexadecahydro-1H-cyclopenta[a]phenanthren-3-yl pivalate |
| 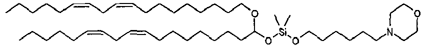 | 4-((20Z,23Z)-10-(8Z,11Z)-heptadeca-8, 11-dien-1 -yl)-8,8-dimethyl-7,9,11-trioxa-8-silanonacosa-20,23-dien-1-yl)morpholine |
| 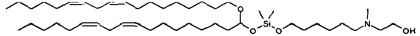 | (23Z,26Z)-13-((8Z,11Z)-heptadeca-8,11-dien-1-yl)-3,11,11-trimethyl-10,12,14-trioxa-3-aza-11-siladotriaconta-23,26-dien-1-ol |
| 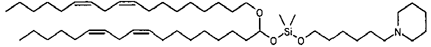 | 1-((20Z,23Z)-10-((8Z,11Z)-heptadeca-8,11-dien-1-yl)-8,8-dimethyl-7,9,11-trioxa-8-silanonacosa-20,23-dien-1-yl)piperidine |
| 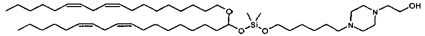 | 2-(4-((20Z,23Z)-10-((8Z,11Z)-heptadeca-8,11-dien-1-yl)-8,8-dimethyl-7,9,11-trioxa-8-silanonacosa-20,23-dien-1-yl)piperazin-1-yl)ethan-1-ol |
| 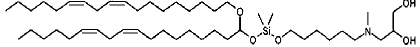 | (24Z,27Z)-14-((8Z,11Z)-heptadeca-8,11-dien-1-yl)-4,12,12-trimethyl-11,13,15-trioxa-4-aza-12-silatritriaconta-24,27-diene-1,2-diol |
| 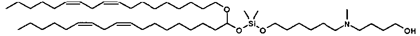 | (25Z,28Z)-15-((8Z,11Z)-heptadeca-8,11-dien-1-yl)-5,13,13-trimethyl-12,14,16-trioxa-5-aza-13-silatetratriaconta-25,28-dien-1-ol |
| 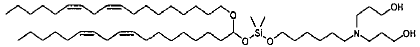 | (24Z,27Z)-14-((8Z,11Z)-heptadeca-8,11-dien-1-yl)-4-(3-hydroxypropyl)-12,12-dimethyl-11,13,15-trioxa-4-aza-12-silatritriaconta-24,27-dien-1-ol |
| 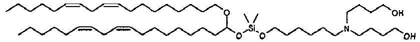 | (25Z,28Z)-15-((8Z,11Z)-heptadeca-8,11-dien-1-yl)-5-(4-hydroxybutyl)-13,13-dimethyl-12,14,16-trioxa-5-aza-13-silatetratriaconta-25,28-dien-l-ol |
| 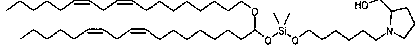 | (1-((20Z,23Z)-10-((8Z,11Z)-heptadeca-8,11-dien-1-yl)-8,8-dimethyl-7,9,11-trioxa-8-silanonacosa-20,23-dien-l-yl)pyrrolidin-2-yl)methanol |
| 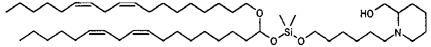 | (1-((20Z,23Z)-10-((8Z,11Z)-heptadeca-8,11-dien-1-yl)-8,8-dimethyl-7,9,11-trioxa-8-silanonacosa-20,23-dien-1-yl)piperidin-2-yl)methanol |
| 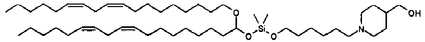 | (1-((20Z,23Z)-10-((8Z,11Z)-heptadeca-8,11-dien-1-yl)-8,8-dimethyl-7,9,11-trioxa-8-silanonacosa-20,23-dien-1-yl)piperidin-4-yl)methanol |
| 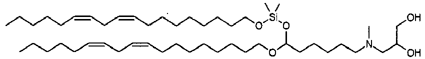 | (22Z,25Z)-4,12,12-trimethyl-10-(((9Z,12Z)-octadeca-9,12-dien-1-yl)oxy)-11,13-dioxa-4-aza-12-silahentriaconta-22,25-diene-1,2-diol |
| 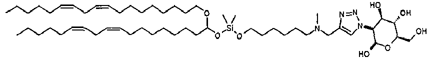 | (4R,5S,6R)-3-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-6-(hydroxymethyl)tetrahydro-2H-pyran-2,4,5-triol |
| 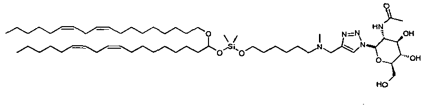 | N-((3R,4R,5S,6R)-2-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-4,5-dihydroxy-6-(hydroxymethyl)tetrahydro-2H-pyran-3-yl)acetamide |
| 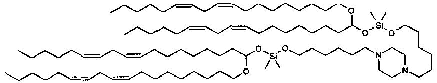 | 1,4-bis((20Z,23Z)-10-((8Z,11Z)-heptadeca-8,11-dien-1-yl)-8,8-dimethyl-7,9,11-tri oxa-8-silanonacosa-20,23-dien-1-yl)piperazine |
| 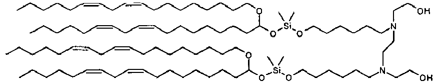 | (26Z,29Z)-16-((8Z,11Z)-heptadeca-8,11-dien-1-yl)-3-((20Z,23Z)-10-((8Z,11Z)-heptadeca-8,11-dien-1-yl)-8,8-dimethyl-7,9,11-trioxa-8-silanonacosa-20,23-dien-1-yl)-6-(2-hydroxyethyl)-14,14-dimethyl-13,15,17-trioxa-3,6-diaza-14-silapentatriaconta-26,29-dien-1-ol |
| 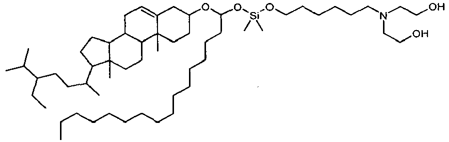 | 13-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silaoctacosan-1-ol |
| 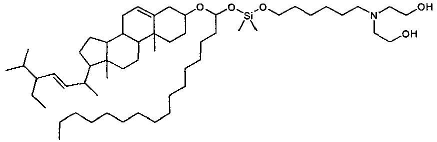 | (E)-13-((17-(5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silaoctacosan-1-ol |
| 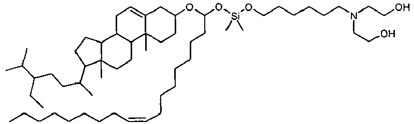 | (Z)-13-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silatriacont-21-en-1-ol |
| 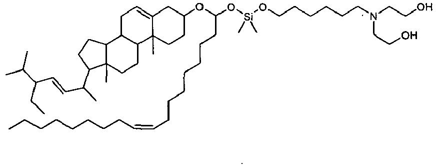 | (Z)-13-((17-((E)-5-ethyl-6-methylhept-3-en-2-yl)-10,13-dimethyl-2,3,4,7,8,9,10,1 1,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silatriacont-21-en-1-ol |
| 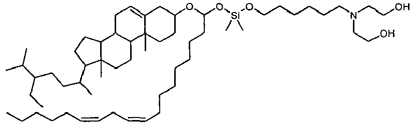 | (21Z,24Z)-13-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silatriaconta-21,24-dien-1-ol |
| 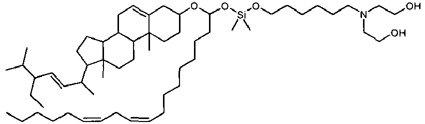 | (21Z,24Z)-13-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silatriaconta-21,24-dien-1-ol |
| 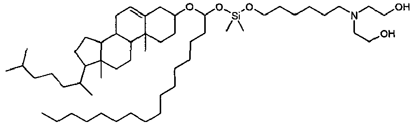 | 13-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silaoctacosan-1-ol |
| 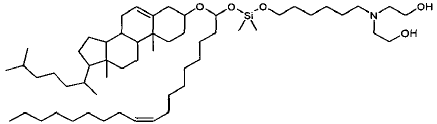 | (Z)-13-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silatriacont-21-en-1-ol |
| 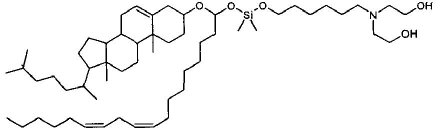 | (21Z,24Z)-13-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)-3-(2-hydroxyethyl)-11,11-dimethyl-10,12-dioxa-3-aza-11-silatriaconta-21,24-dien-1-ol |
| 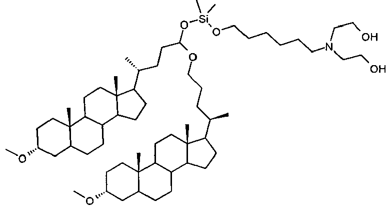 | (18R)-3-(2-hydroxyethyl)-18-((3R,10S,13R)-3-methoxy-10,13-dimethylhexadecahydro-1H-cyclopenta[a]phenanthren-17-yl)-13-((3R)-3-((3R,10S,13R)-3-methoxy-10,13-dimethylhexadecahydro-1H-cyclopenta[a]phenanthren-17-yl)butyl)-11,11-dimethyl-10,12,14-trioxa-3-aza-11-silanonadecan-1-ol |
| 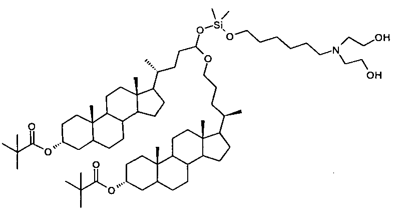 | (3R,10S,13R)-17-((18R)-13-((3R)-3-((3R,10S,13R)-10,13-dimethyl-3-(pivaloyloxy)hexadecahydro-1H-cyclopenta[a]phenanthren-17-yl)butyl)-1-hydroxy-3-(2-hydroxyethyl)-11,11-dimethyl-10,12,14-trioxa-3-aza-11-silanonadecan-18-yl)-10,13-dimethylhexadecahydro-1H-cyclopenta[a]phenanthren-3-yl pivalate |
| 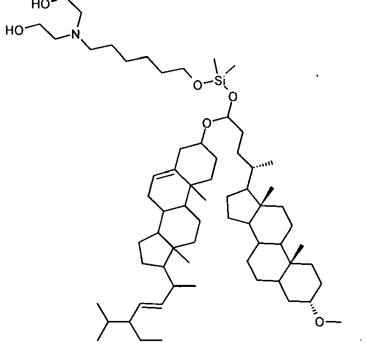 | (3S,10R,13S)-17-((16S)-13-((17-((E)-5-ethyl1-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)-1-hydroxy-3-(2-hydroxyethyl)-11,11-dimethyl-10,12-dioxa-3-aza-11-silaheptadecan-16-yl)-10,13-dimethylhexadecahydro-1H-cyclopenta[a]phenanthren-3-yl pivalate |
| 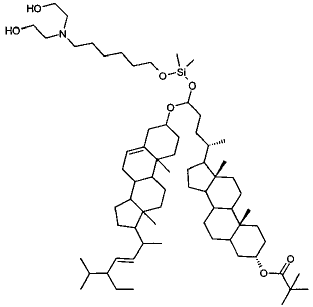 | (3S,10R,13S)-17-((16S)-13-((17-((E)-5,6-dimethylhept-3-en-2-yl)-10,13-dimethyl-2,3,4,7,8,9,10,11,12,13,14,15,16,17-tetradecahydro-1H-cyclopenta[a]phenanthren-3-yl)oxy)-1-hydroxy-3-(2-hydroxyethyl)-11,11-dimethyl-10,12-dioxa-3-aza-11-silaheptadecan-16-yl)-10,13-dimethylhexadecahydro-1H-cyclopenta[a]phenanthren-3-yl pivalate |
| 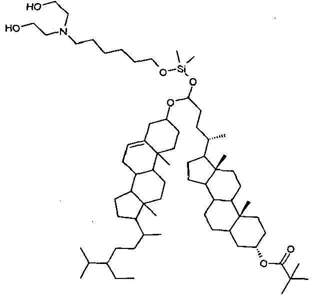 | (3S,10R,13S)-17-((16S)-13-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)-1-hydroxy-3-(2-hydroxyethyl)-11,11-dimethyl-10,12-dioxa-3-aza-11-silaheptadecan-16-yl)-10,13-dimethylhexadecahydro-1H-cyclopenta[a]phenanthren-3-yl pivalate |
| 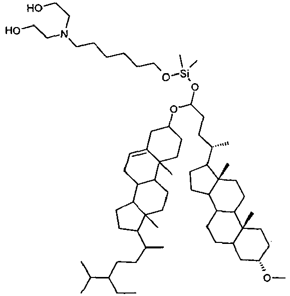 | (16S)-13-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)-3-(2-hydroxyethyl)-16-((3S,10R,13S)-3-methoxy-10,13-dimethylhexadecahydro-1H-cyclopenta[a]phenanthren-17-yl)-11,11-dimethyl-10,12-dioxa-3-aza-11-silaheptadecan-1-ol |
| 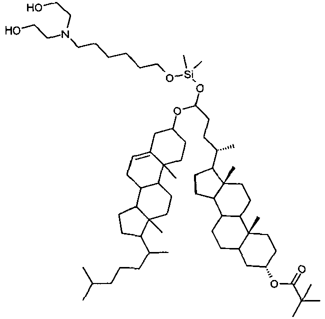 | (3S,10R,13S)-17-((16S)-13-((10,13-dimethyl-17-(6-methylheptan-2-yl)-2,3,4,7,8,9,10,1 1,12,13,14,15,16,17-tetradecahydro-1H-cyclopenta[a]phenanthren-3-yl)oxy)-1-hydroxy-3-(2-hydroxyethyl)-11,11-dimethyl-10,12-dioxa-3-aza-3-silaheptadecan-16-yl)-10,13-dimethylhexadecahydro-1H-cyclopenta[a]phenanthren-3-yl pivalate |
| 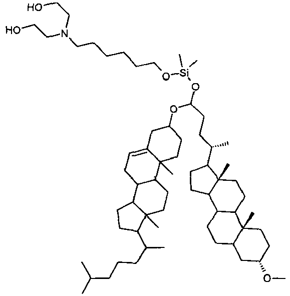 | (16S)-13-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)-3-(2-hydroxyethyl)-16-((3S,10R,13S)-3-methoxy-10,13-dimethylhexadecahydro-1H-cyclopenta[a]phenanthren-17-yl)-11,11-dimethyl-10,12-dioxa-3-aza-11-silaheptadecan-1-ol |
| 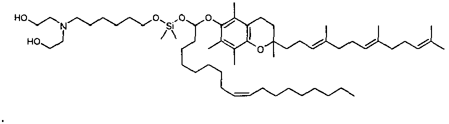 | (Z)-3-(2-hydroxyethyl)-11,11-dimethyl-13-((2,5,7,8-tetramethyl-2-((3E,7E)-4,8,12-trimethyltrideca-3,7,11-trien-1-yl)chroman-6-yl)oxy)-10,12-dioxa-3-aza-11-silatriacont-21-en-1-ol |
| 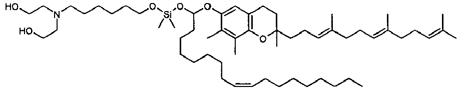 | (Z)-3-(2-hydroxyethyl)-11,11-dimethyl-13-((2,7,8-trimethyl-2-((3E,7E)-4,8,12-trimethyltrideca-3,7,11-trien-1-yl)chroman-6-yl)oxy)-10,12-dioxa-3-aza-11-silatriacont-21-en-1-ol |
| 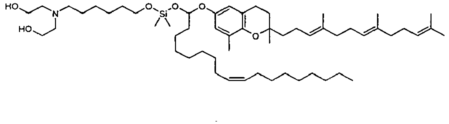 | (Z)-13-((2,8-dimethyl-2-((3E,7E)-4,8,12-trimethyltrideca-3,7,11-trien-1-yl)chrom an-6-yl)oxy)-3-(2-hydroxyethyl)-11,11-dimethyl-10,12-dioxa-3-aza-11-silatriacont-21-en-1-ol |
| 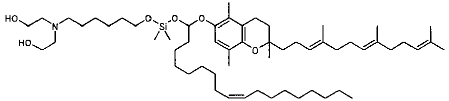 | (Z)-3-(2-hydroxyethyl)-11,11-dimethyl-13-((2,5,8-trimethyl-2-((3E,7E)-4,8,12-trimethyltrideca-3,7,11-trien-1-yl)chroman-6-yl)oxy)-10,12-dioxa-3-aza-11-silatriacont-21-en-1-ol |
| 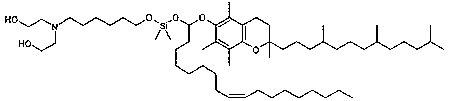 | (Z)-3-(2-hydroxyethyl)-11,11-dimethyl-13-((2,5,7,8-tetramethyl-2-(4,8,12-trimethyltridecyl)chroman-6-yl)oxy)-10,12-dioxa-3-aza-11-silatriacont-21-en-1-ol |
| 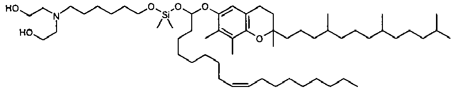 | (Z)-3-(2-hydroxyethyl)-11,11-dimethyl-13-((2,7,8-trimethyl-2-(4,8,12-trimethyltridecyl)chroman-6-yl)oxy)-1 0,12-dioxa-3-aza-11-silatriacont-21-en-1-ol |
| 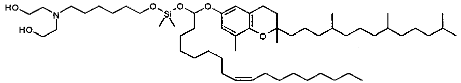 | (Z)-13-((2,8-dimethyl-2-(4,8,12-trimethyltridecyl)chroman-6-yl)oxy)-3-(2-hydroxyethyl)-11,11-dimethyl-10,12-dioxa-3-aza-11-silatriacont-21-en-1-ol |
| 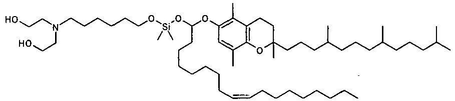 | (Z)-3-(2-hydroxyethyl)-11,11-dimethyl-13-((2,5,8-trimethyl-2-(4,8,12-trimethyltridecyl)chroman-6-yl)oxy)-10,12-dioxa-3-aza-11-silatriacont-21-en-1-ol |
| 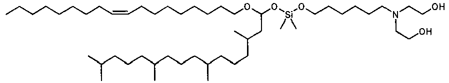 | (Z)-3-(2-hydroxyethyl)-11,11-dimethyl-13-(2,6,10,14-tetramethylpentadecyl)-10,12,14-trioxa-3-aza-l 1-siladotriacont-23-en-1-ol |
| 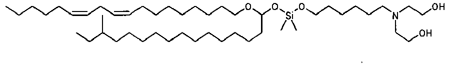 | (23Z,26Z)-3-(2-hydroxyethyl)-11,11-dimethyl-13-(13-methylpentadecyl)-10,12,14-trioxa-3-aza-11-siladotriaconta-23,26-dien-1-ol |
| 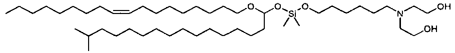 | (Z)-3-(2-hydroxyethyl)-11,11-dimethyl-13-(14-methylpentadecyl)-10,12,14-trioxa-3-aza-11-siladotriacont-23-en-1-ol |
| 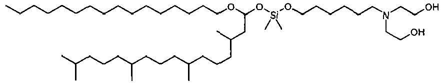 | 3-(2-hydroxyethyl)-11,11-dimethyl-13-(2,6,10,14-tetramethylpentadecyl)-10,12,14-trioxa-3-aza-11-silatriacontan-1-ol |
| 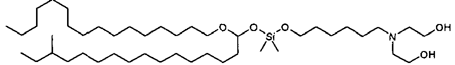 | 3-(2-hydroxyethyl)-11,11-dimethyl-13-(13-methylpentadecyl)-10,12,14-trioxa-3-aza-11-silatriacontan-1-ol |
| 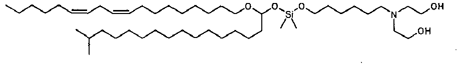 | (23 Z,26Z)-3-(2-hydroxyethyl)-11,11-dimethyl-13-(14-methylpentadecyl)-10,12,14-trioxa-3-aza-11 -siladotriaconta-23,26-dien-1 -ol |
| 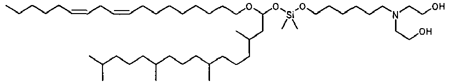 | (23Z,26Z)-3-(2-hydroxyethyl)-11,11-dimethyl-13-(2,6,10,14-tetramethylpentadecyl)-10,12,14-trioxa-3-aza-11-siladotriaconta-23,26-dien-1-ol |
| 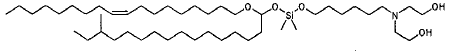 | (Z)-3-(2-hydroxyethyl)-11,11-dimethyl-13-(13-methylpentadecyl)-10,12,14-trioxa-3-aza-11-siladotriacont-23-en-1-ol |
| 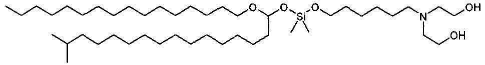 | 3-(2-hydroxyethyl)-11,11-dimethyl-13-(14-methylpentadecyl)-10,12,14-trioxa-3-aza-11-silatriacontan-1-ol |
| 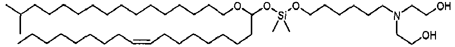 | (Z)-13-(heptadec-8-en-1-yl)-3-(2-hydroxyethyl)-11,11,29-trimethyl-10,12,14-trioxa-3-aza-11-silatriacontan-1-ol |
| 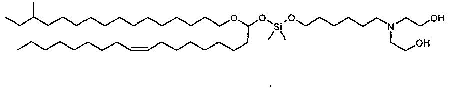 | (Z)-13-(heptadec-8-en-1-yl)-3-(2-hydroxyethyl)-11,11,28-trimethyl-10,12,14-trioxa-3-aza-11-silatriacontan-1-ol |
| 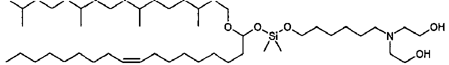 | (Z)-13-(heptadec-8-en-1-yl)-3-(2-hydroxyethyl)-11,11,17,21,25,29-hexamethyl-10,12,14-trioxa-3-aza-11-silatriacontan-1-ol |
| 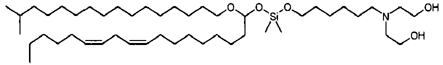 | 13-((8Z,11Z)-heptadeca-8,11-dien-1-yl)-3-(2-hydroxyethyl)-11,11,29-trimethyl-10,12,14-trioxa-3-aza-11-silatriacontan-1-ol |
| 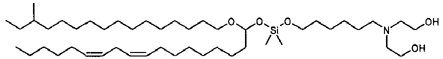 | 13-((8Z,11Z)-heptadeca-8,11-dien-1-yl)-3-(2-hydroxyethyl)-11,11,28-trimethyl-10,12,14-trioxa-3-aza-11-silatriacontan-1-ol |
| 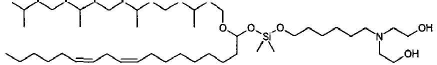 | 13-((8Z,11Z)-heptadeca-8,11-dien-1-yl)-3-(2-hydroxyethyl)-11,11,17,21,25,29-hexamethyl-10,12,14-trioxa-3-aza-11-silatriacontan-1-ol |
| 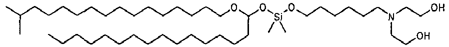 | 3-(2-hydroxyethyl)-11,11,29-trimethyl-13-pentadecyl-10,12,14-trioxa-3-aza-11-silatriacontan-l-ol |
| 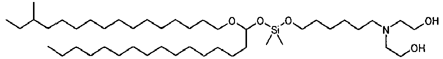 | 3-(2-hydroxyethyl)-11,11,28-trimethyl-13-pentadecyl-10,12,14-trioxa-3-aza-11-silatriacontan-1-ol |
| 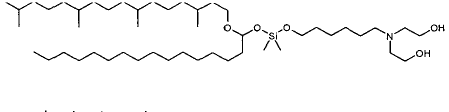 | 3-(2-hydroxyethyl)-11,11,17,21,25,29-hexamethyl-13-pentadecyl-10,12,14-trioxa-3-aza-11-silatriacontan-1-ol |
| 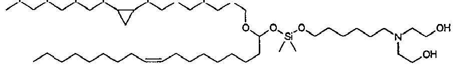 | (Z)-13-(heptadec-8-en-1-yl)-3-(2-hydroxyethyl)-11,11-dimethyl-22-(2-octylcyclopropyl)-10,12,14-trioxa-3-aza-11-siladocosan-1-ol |
| 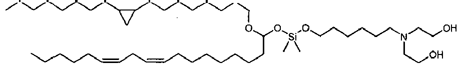 | 13-((8Z,11Z)-heptadeca-8,11-dien-1-yl)-3-(2-hydroxyethyl)-11,11-dimethyl-22-(2-octylcyclopropyl)-10,12,14-trioxa-3-aza-11-siladocosan-1-ol |
| 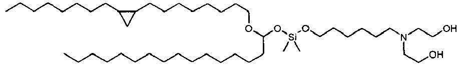 | 3-(2-hydroxyethyl)-11,11-dimethyl-22-(2-octylcyclopropyl)-13-pentadecyl-10,12,14-trioxa-3-aza-11-siladocosan-1-ol |
| 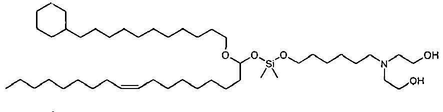 | (Z)-25-cyclohexyl-13-(heptadec-8-en-1-yl)-3-(2-hydroxyethyl)-11,11-dimethyl-10,12,14-trioxa-3-aza-11-silapentacosan-1-ol |
| 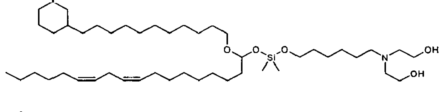 | 25-cyclohexyl-13-((8Z,11Z)-heptadeca-8,11-dien-1-yl)-3-(2-hydroxyethyl)-11,11-dimethyl-10,12,14-trioxa-3-aza-11-silapentacosan-1-ol |
| 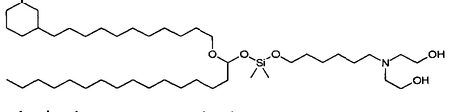 | 25-cyclohexyl-3-(2-hydroxyethyl)-11,11-dimethyl-13-pentadecyl-10,12,14-trioxa-3-aza-11-silapentacosan-1-ol |
| 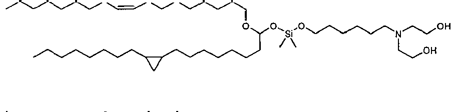 | (Z)-3-(2-hydroxyethyl)-11,11-dimethyl-13-(7-(2-octylcyclopropyl)heptyl)-10,12,14-trioxa-3-aza-11-siladotriacont-23-en-1-ol |
| 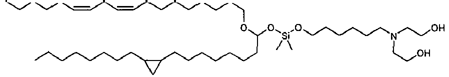 | (23Z,26Z)-3-(2-hydroxyethyl)-11,11-dimethyl-13-(7-(2-octylcyclopropyl)heptyl)-10,12,14-trioxa-3-aza-11-siladotriaconta-23,26-dien-1-ol |
| 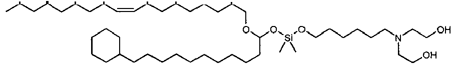 | (Z)-13-(10-cyclohexyldecyl)-3-(2-hydroxyethyl)-11,11-dimethyl-10,12,14-trioxa-3-aza-11-siladotriacont-23-en-1-ol |
| 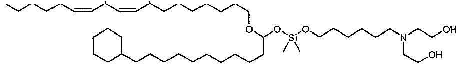 | (23Z,26Z)-13-(10-cyclohexyldecyl)-3-(2-hydroxyethyl)-11,11-dimethyl-10,12,14-trioxa-3-aza-11-siladotriaconta-23,26-dien-1-ol |
| 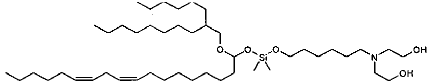 | 13-((8Z,11Z)-heptadeca-8,11-dien-1-yl)-16-hexyl-3-(2-hydroxyethyl)-11,11-dimethyl-10,12,14-trioxa-3-aza-11-silatetracosan-1-ol |
| 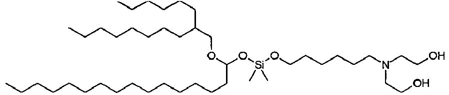 | 16-hexyl-3-(2-hydroxyethyl)-11,11-dimethyl-13-pentadecyl-10,12,14-trioxa-3-aza-11-silatetracosan-1-ol |
| 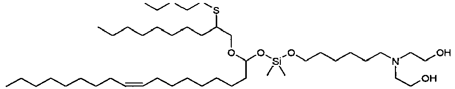 | (Z)-13-(heptadec-8-en-1-yl)-3-(2-hydroxyethyl)-11,11-dimethyl-16-octyl-10,12,14-trioxa-17-thia-3-aza-11-siladocosan-1-ol |
| 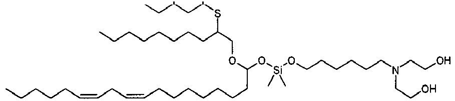 | 13-((8Z,11Z)-heptadeca-8,11-dien-1-yl)-3-(2-hydroxyethyl)-11,11-dimethyl-16-octyl-10,12,14-trioxa-17-thia-3-aza-11-siladocosan-1-ol |
| 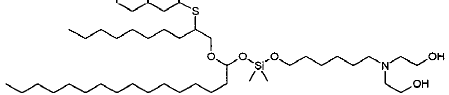 | 3-(2-hydroxyethyl)-11,11-dimethyl-16-octyl-13-pentadecyl-10,12,14-trioxa-17-thia-3-aza-11-siladocosan-l-ol |
| 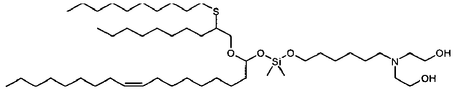 | (Z)-13-(heptadec-8-en-1-yl)-3-(2-hydroxyethyl)-11,11-dimethyl-16-octyl-10,12,14-trioxa-17-thia-3-aza-11-silaheptacosan-1-ol |
| 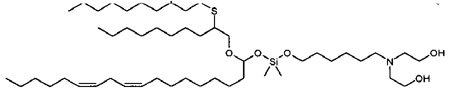 | 13-((8Z,11Z)-heptadeca-8,11-dien-1-yl)-3-(2-hydroxyethyl)-11,11-dimethyl-16-octyl-10,12,14-trioxa-17-thia-3-aza-11-silaheptacosan-1-ol |
| 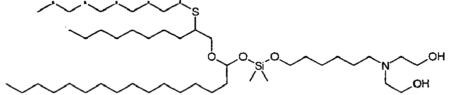 | 3-(2-hydroxyethyl)-11,11-dimethyl-16-octyl-13-pentadecyl-10,12,14-trioxa-17-thia-3-aza-11 - silaheptacosan-l-ol |
| 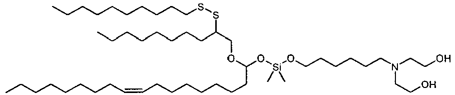 | (Z)-13-(heptadec-8-en-1-yl)-3-(2-hydroxyethyl)-11,11-dimethyl-16-octyl-10,12,14-trioxa-17,18-dithia-3-aza-11-silaoctacosan-1-ol |
| 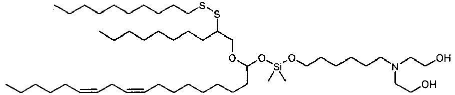 | 13-((8Z,11Z)-heptadeca-8,11-dien-1-yl)-3-(2-hydroxyethyl)-11,11-dimethyl-16-octyl-10,12,14-trioxa-17,18-dithia-3-aza-11-silaoctacosan-1-ol |
| 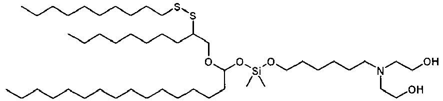 | 3-(2-hydroxyethyl)-11,11-dimethyl-16-octyl-13-pentadecyl-10,12,14-trioxa-17,18-dithia-3-aza-11-silaoctacosan-1-ol |
| 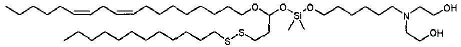 | 3-(2-hydroxyethyl)-11,11-dimethyl-13-(((9Z,12Z)-octadeca-9,12-dien-1-yl)oxy)-10,12-dioxa-16,17-dithia-3-aza-11-silanonacosan-1-ol |
| 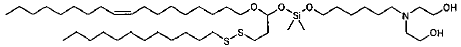 | (Z)-3-(2-hydroxyethyl)-11,11-dimethyl-13-(octadec-9-en-1-yloxy)-10,12-dioxa-16,17-dithia-3-aza-11-silanonacosan-1-ol |
| 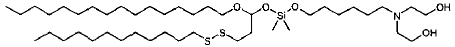 | 13-(hexadecyloxy)-3-(2-hydroxyethyl)-11,11-dimethyl-10,12-dioxa-16,17-dithia-3-aza-11-silanonacosan-1-ol |
| 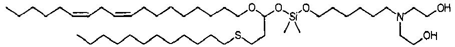 | (23Z,26Z)-13-(2-(dodecylthio)ethyl)-3-(2-hydroxyethyl)-11,11-dimethyl-10,12,14-trioxa-3-aza-11-siladotriaconta-23,26-dien-1-ol |
| 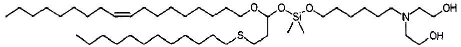 | (Z)-13-(2-(dodecylthio)ethyl)-3-(2-hydroxyethyl)-11,11-dimethyl-10,12,14-trioxa-3-aza-11-siladotriacont-23-en-1-ol |
| 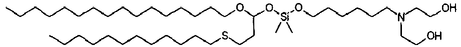 | 13-(2-(dodecylthio)ethyl)-3-(2-hydroxyethyl)-11,11-dimethyl-10,12,14-trioxa-3-aza-11-silatriacontan-1-ol |
| 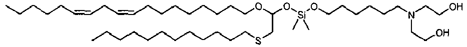 | (23Z,26Z)-13-((dodecylthio)methyl)-3-(2-hydroxyethyl)-11,11-dimethyl-10,12,14-trioxa-3-aza-11-iladotriaconta-23,26-dien-1-ol |
| 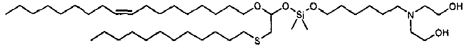 | (Z)-13-((dodecylthio)methyl)-3-(2-hydroxyethyl)-11,11-dimethyl-10,12,14-trioxa-3-aza-11-siladotriacont-23-en-1-ol |
| 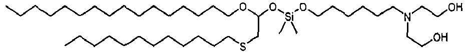 | 13-((dodecylthio)methyl)-3-(2-hydroxyethyl)-11,11-dimethyl-10,12,14-trioxa-3-aza-11-silatriacontan-1-ol |
| 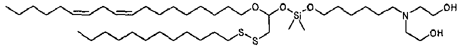 | 3-(2-hydroxyethyl)-11,11-dimethyl-13-(((9Z,12Z)-octadeca-9,12-dien-1-yl)oxy)-10,12-dioxa-15,16-dithia-3-aza-11-silaoctacosan-1-ol |
| 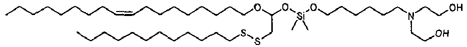 | (Z)-3-(2-hydroxyethyl)-11,11-dimethyl-13-(octadec-9-en-1-yloxy)-10,12-dioxa-15,16-dithia-3-aza-11-silaoctacosan-1-ol |
| 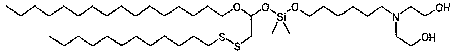 | 13-(hexadecyloxy)-3-(2-hydroxyethyl)-11,11-dimethyl-10,12-dioxa-15,16-dithia-3-aza-11-silaoctacosan-1-ol |
| 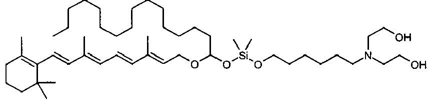 | (16E, 18E,20E,22E)-3-(2-hydroxyethyl)-11,11,17,21-tetramethyl-13-pentadecyl-23-(2,6,6-trimethylcyclohex-1-en-1-yl)-10,12,14-trioxa-3-aza-11-silatricosa-16,18,20,22-tetraen-1-ol |
| 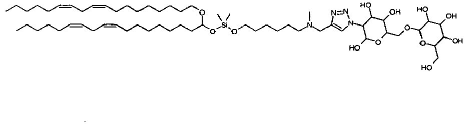 | 3-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-6-(((3,4,5-trihydroxy-6-(hydroxymethyl)tetrahydro-2H-pyran-2-yl)oxy)methyl)tetrahydro-2H-pyran-2,4,5-triol |
| 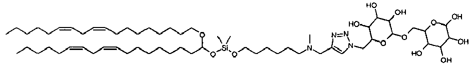 | 6-(((6-((4-((22Z,25Z)-12-((8Z,11Z)-heptadeca-8,11-dien-1-yl)-2,10,10-trimethyl-9,11,13-trioxa-2-aza-10-si lahentriaconta-22,25-dien-1 - yl)-1H-1,2,3-triazol-1-yl)methyl)-3,4,5-trihydroxytetrahydro-2H-pyran-2-yl)oxy)methyl)tetrahydro-2H-pyran-2,3,4,5-tetraol |
| 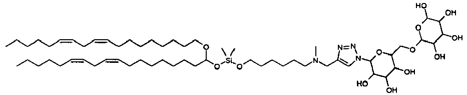 | 6-((6-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-3,4,5-trihydroxytetrahydro-2H-pyran-2-yl)methoxy)tetrahydro-2H-pyran-2,3,4,5-tetraol |
| 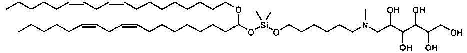 | (27Z,30Z)-17-((8Z,11Z)-heptadeca-8,11-dien-1-yl)-7,15,15-trimethyl-14,16,18-trioxa-7-aza-15-silahexatriaconta-27,30-diene-1,2,3,4,5-pentaol |
| 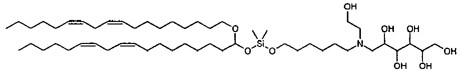 | (27Z,30Z)-17-((8Z,11Z)-heptadeca-8,11-dien-1-yl)-7-(2-hydroxyethyl)-15,15-dimethyl-14,16,18-trioxa-7-aza-15-silahexatriaconta-27,30-diene-1,2,3,4,5-pentaol |
| 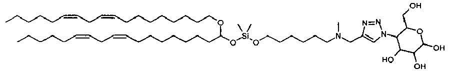 | 5-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-6-(hydroxymethyl)tetrahydro-2H-pyran-2,3,4-triol |
| 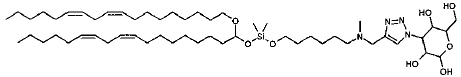 | 4-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-6-(hydroxymethyl)tetrahydro-2H-pyran-2,3,5-triol |
| 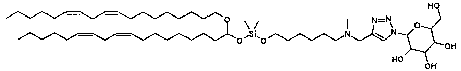 | 2-(4-((22Z,25Z)-12-((8Z,11Z)-heptadeca-8,11-dien-1-yl)-2,10,10-trimethyl-9,11,13-trioxa-2-aza-10-silahentriaconta-22,25-dien-1-yl)-1H-1,2,3-triazol-1-yl)-6-(hydroxymethyl)tetrahydro-2H-pyran-3,4,5-triol |
| 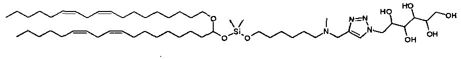 | 6-(4-((22Z,25Z)12-((8Z,11Z)-heptadeca-8,11-dien-1-yl)-2,10,10-trimethyl-9,11,13-trioxa-2-aza-10-silahentriaconta-22,25-dien-1-yl)-1H-1,2, 3-triazol-1-yl)hexane-1,2,3,4, 5-pentaol |
| 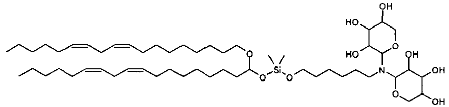 | 2,2'-(((20Z,23Z)-10-((8Z,11Z)-heptadeca-8,11-dien-1-yl)-8,8-dimethyl-7,9,11-trioxa-8-silanonacosa-20,23-dien-1-yl)azanediyl)bis(tetrahydro-2H-pyran-3,4,5-triol) |
| 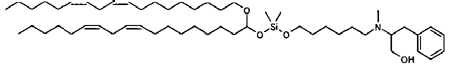 | (23Z,26Z)-2-benzyl-13-((8Z,11Z)-heptadeca-8,11-dien-1-yl)-3,11,11-trimethyl-10,12,14-trioxa-3-aza-11-siladotriaconta-23,26-dien-1-ol |
| 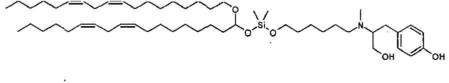 | (23Z,26Z)-13-((8Z,11Z)-heptadeca-8,11-dien-1-yl)-2-(4-hydroxybenzyl)-3,11,11-trimethyl-10,12,14-trioxa-3-aza-11-siladotriaconta-23,26-dien-1-ol |
| 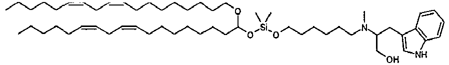 | (23Z,26Z)-2-((1H-indol-3-yl)methyl)-13-((8Z,11Z)-heptadeca-8,11-dien-1-yl)-3,11,11-trimethyl-10,12,14-trioxa-3-aza-11-siladotriaconta-23,26-dien-1-ol |
| 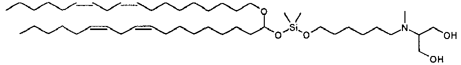 | 2-(((20Z,23Z)-10-((8Z,11Z)-heptadeca-8,11-dien-1-yl)-8,8-dimethyl-7,9,11-trioxa-8-silanonacosa-20,23-dien-1-yl)(methyl)amino)propane-1,3-diol |
| 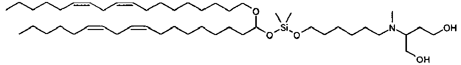 | 2-(((20Z,23Z)-10-((8Z,11Z)-heptadeca-8,11-dien-1-yl)-8,8-dimethyl-7,9,11-trioxa-8-silanonacosa-20,23-dien-1-yl)(methyl)amino)butane-1,4-diol |
| 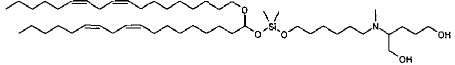 | 2-(((20Z,23Z)-10-((8Z,11Z)-heptadeca-8,11-dien-1-yl)-8,8-dimethyl-7,9,11-trioxa-8-silanonacosa-20,23-dien-1-yl)(methyl)amino)pentane-1,5-diol |
| 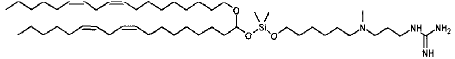 | 1-((24Z,27Z)-14-((8Z,11Z)-heptadeca-8,11-dien-1-yl)-4,12,12-trimethyl-11,13,15-trioxa-4-aza-12-silatritriaconta-24,27-dien-1-yl)guanidine |
| 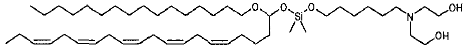 | 3-(2-hydroxyethyl)-11,11-dimethyl-13-((4Z,7Z,10Z,13Z,16Z)-nonadeca-4,7,10,13,16-pentaen-1-yl)-10,12,14-trioxa-3-aza-11-silatriacontan-1-ol |
| 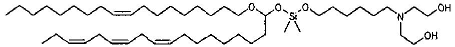 | (Z)-13-((8Z,11Z,14Z)-heptadeca-8,11,14-trien-1-yl)-3-(2-hydroxyethyl)-11,11-dimethyl-10,12,14-trioxa-3-aza-11-siladotriacont-23-en-1-ol |
| 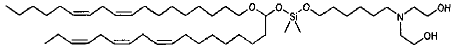 | (23Z,26Z)-13-((8Z,11Z,14Z)-heptadeca-8,11,14-trien-1-yl)-3-(2-hydroxyethyl)-11,11-dimethyl-10,12,14-trioxa-3-aza-11-siladotriaconta-23,26-dien-1-ol |
| 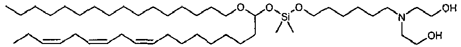 | 13-((8Z, 11Z, 14Z)-heptadeca-8,11,14-trien-1 - yl)-3-(2-hydroxyethyl)-11,11-dimethyl-10,12,14-trioxa-3-aza-11-silatriacontan-1-ol |
| 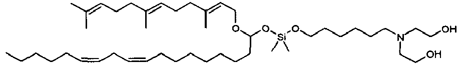 | (16E,20E)-13-((8Z,11Z)-heptadeca-8,11-dien-1-yl)-3-(2-hydroxyethyl)-11,11,17,21,25-pentamethyl-10,12,14-trioxa-3-aza-11-silahexacosa-16,20,24-trien-1-ol |
| 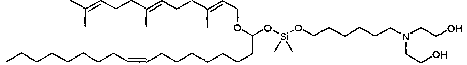 | (16E,20E)-13-((Z)-heptadec-8-en-1-yl)-3-(2-hydroxyethyl)-11,11,17,21,25-pentamethyl-10,12,14-trioxa-3-aza-11-silahexacosa-16,20,24-trien-1-ol |
| 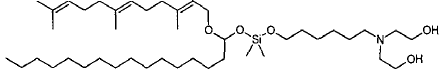 | (16E,20E)-3-(2-hydroxyethyl)-11,11,17,21,25-pentamethyl-13-pentadecyl-10,12,14-trioxa-3-aza-11-silahexacosa-16,20,24-trien-1-ol |
| 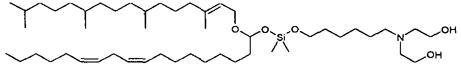 | (E)-13-((8Z,11Z)-heptadeca-8,11-dien-1-yl)-3-(2-hydroxyethyl)-11,11,17,21,25,29-hexamethyl-10,12,14-trioxa-3-aza-11-silatriacont-16-en-1 -ol |
| 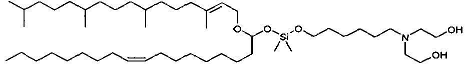 | (E)-13-((Z)-heptadec-8-en-1-yl)-3-(2-hydroxyethyl)-11,11,17,21,25,29-hexamethyl-10,12,14-trioxa-3-aza-11-silatriacont-16-en-1-ol |
| 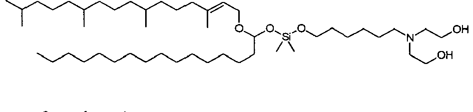 | (E)-3-(2-hydroxyethyl)-11,11,17,21,25,29-hexamethyl-13-pentadecyl-10,12,14-trioxa-3-aza-11-silatriacont-16-en-1-ol |
| 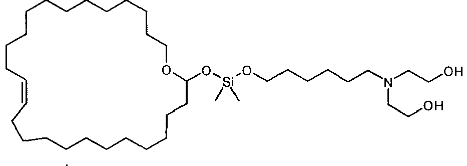 | (Z)-2,2'-((6-((dimethyl((oxacycloheptacos-14-en-2-yl)oxy)silyl)oxy)hexyl)azanediyl)bis(ethan-1-ol) |
| 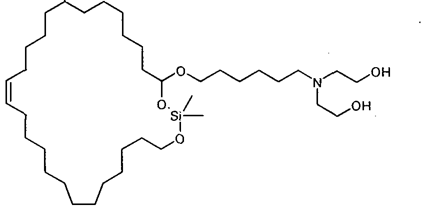 | (Z)-2,2'-((6-((2,2-dimethyl-1,3-dioxa-2-silacyclononacos-16-en-4-yl)oxy)hexyl)azanediyl)bis(ethan-1-ol) |
| 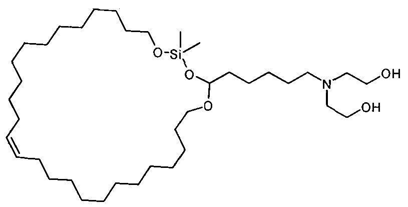 | (Z)-2,2'-((6-((2,2-dimethyl-1,3-dioxa-2-silacyclononacos-16-en-4-yi)oxy)hexyl)azanediyl)bis(ethan-1-ol) |
| 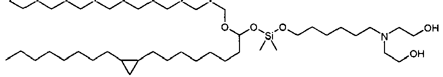 | 3-(2-hydroxyethyl)-11,11-dimethyl-13-(7-(2-octylcyclopropyl)heptyl)-10,12,14-trioxa-3-aza-11-silatriacontan-1-ol |
| 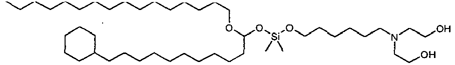 | 13-(10-cyclohexyldecyl)-3-(2-hydroxyethyl)-11,11-dimethyl-10,12,14-trioxa-3-aza-11-silatriacontan-1-ol |

12. A compound having the following structure (II)) 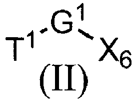 wherein
G¹ is as defined in claim 1;
T¹ is (a) or (c)
wherein
R₁ is linear C₁₈-C₃₂ alkyl, branched C₁₈-C₃₂ alkyl, in both cases the chain optionally containing one S, SO, SO₂, -S-S-, O, or Si(R^{a})₂, wherein R^{a} is C₁-C₆ alkyl, at any position in the carbon chain with the proviso that the heteroatom is not in the alpha or omega position of the carbon chain, or linear or branched C₁₈-C₃₂ alkenyl containing one double bond or linear or branched C₂₁-C₃₂ alkenyl containing more double bonds with the proviso that there is at least one -CH₂-group between the double bond and X₂,or
R₁ is linear C₁-C₃₂ alkyl or branched C₁-C₃₂ alkyl, in both cases the chain containing one -S-Sat any position in the carbon chain with the proviso that the is not in the alpha or omega position of the carbon chain, or branched C₈-C₃₂ alkenyl containing more double bonds with the proviso that there is at least one -CH₂- group between the double bond and X₂,
R₁ is CH₃-(CH₂)_{d}-V₁-(CH₂)ₑ-, wherein d is an integer selected from 1 to 20, e is an integer selected from 1 to 20, and the sum of d+e is an integer selected from 2 to 29, and V₁ is a C₃-C₆ cycloalkylene, or
R₁ is V₂-(CH₂)_{f}-, wherein f is an integer selected from 1 to 30, and V₂ is a C₃-C₆ cycloalkyl, or
R₁ is 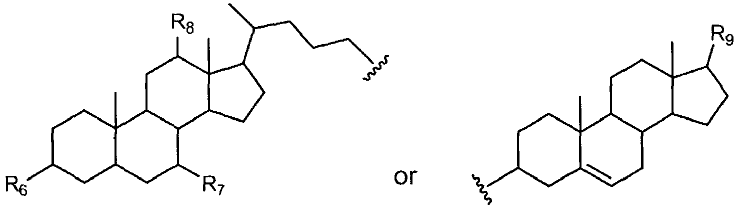
wherein R₆, R₇, R₈ are the same or different and each is independently H, OH, -C₁-C₆ alkoxy, -O-C(O)-C₁-C₆ alkyl or fluorine, wherein at least one of R₆, R₇, R₈ is , -O-C(O)-C₁-C₆ alkyl,
R₉ is linear or branched C₃-C₁₂ alkyl or C₃-C₁₂ alkenyl containing one double bond, or
R₁ is 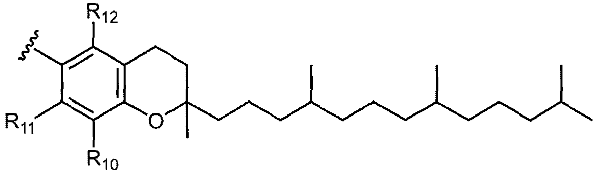 wherein R₁₀, R₁₁, R₁₂ are the same or different and each is independently H, F or methyl, or
R₁ is 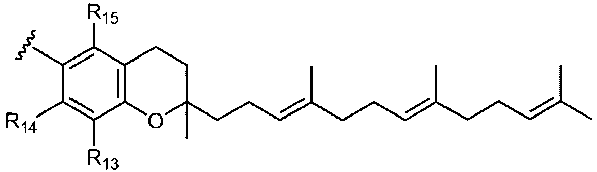 wherein R₁₃, R₁₄, R₁₅ are the same or different and each is independently H, F or methyl, or
R₁ is 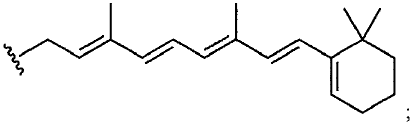
and b¹, X₁, X₂, R₂, R₃, and R₅ are as defined in claim 1; or
T¹ is (b) or (c) wherein
R₂ is linear C₁₈-C₃₂ alkyl, branched C₁₈-C₃₂ alkyl, in both cases optionally containing one S, SO, SO₂, -S-S-, O, or Si(R^{b})₂, wherein R^{b} is C₁-C₆ alkyl, at any position in the carbon chain with the proviso that the heteroatom is not in the alpha or omega position of the carbon chain, or linear or branched C₁₈-C₃₂ alkenyl containing one double bond or linear or branched C₂₁-C₃₂ alkenyl containing more double bonds with the proviso that there is at least one -CH₂- group between the double bond and the carbon linking X₁ and X₂, or
R₂ is linear C₁-C₃₂ alkyl or branched C₃-C₃₂ alkyl, in both cases containing one -S-S- at any position in the carbon chain with the proviso that the heteroatom is not in the alpha or omega position of the carbon chain, or branched C₈-C₃₂ alkenyl containing more double bonds with the proviso that there is at least one -CH₂- group between the double bond and the carbon linking X₁ and X₂, or
R₂ is CH₃-(CH₂)_{g}-V₃-(CH₂)ₕ-, wherein g is an integer selected from 1 to 20, h is an integer selected from 1 to 20, and the sum of g+h is an integer selected from 2 to 28, wherein V₃ is a C₃-C₆ cycloalkylene, or
R₂ is V₄-(CH₂)ₜ-, wherein t is an integer selected from 1 to 29, wherein V₃ is a C₃-C₆ cycloalkyl, or
R₂ is 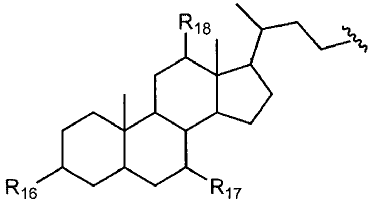
wherein R₁₆, R₁₇, R₁₈ are the same or different and each is independently H, OH, -C₁-C₆ alkoxy, -O-C(O)-C₁-C₆ alkyl or fluorine, wherein at least one of R₁₆, R₁₇, R₁₈ is , - O-C(O)-C₁-C₆ alkyl;
and b¹, X₁, X₂, R₁, R₃, and R₅ are as defined in claim 1; or
T¹ is (c) wherein
R₁ is 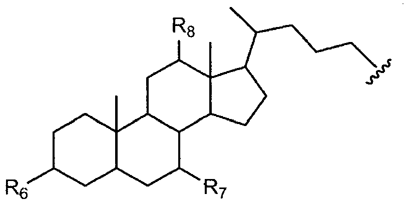 wherein R₆, R₇, R₈ are the same or different and each is independently H, OH, -C₁-C₆ alkoxy, -O-C(O)-C₁-C₆ alkyl, and
R₂ is 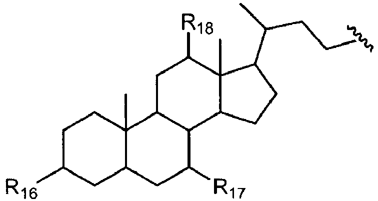 wherein R₁₆, R₁₇, R₁₈ are the same or different and each is independently H, OH, -C₁-C₆ alkoxy, -O-C(O)-C₁-C₆ alkyl or fluorine
and b¹, X₁, X₂, R₃, and R₅ are as defined in claim 1; or T¹ is (a) wherein
R₁ is connected to R₃, wherein R₃ has the meaning as defined above, wherein Y₁, X₃ and b₂ are as defined above and R₄ forms together with R₁ an alkylene or alkenylene containing one double bond, and thus R₁ and R₃ form together with the intervening atoms of (a) a 31-32-membered ring, and b¹, X₁, X₂, are as defined in claim 1; or
T¹ is (b) wherein
R₂ is connected to R₃, wherein R₃ has the meaning as defined above, wherein Y₁, X₃ and b₂ are as defined above and R₄ forms together with R₂ an alkylene or alkenylene containing one double bond, and thus R₂ and R₃ form together with the intervening atoms of (b) a 31-32-membered ring, wherein the carbon atom of R₂ at the first or second position numbered from that carbon atom which is attached to the carbon linking X₁ and X₂, can be optionally replaced by O or S heteroatom, and b¹, X₁, X₂ are as defined in claim 1; or
T¹ is (c) wherein
R₂ is connected to R₁ and R₂ forms together with R₁ an alkylene or alkenylene containing one double bond and thus R₂ and R₁ form together with the intervening atoms of (c) a 31-32-membered ring, wherein the carbon atom of R₂ at the first or second position numbered from that carbon atom which is attached to the carbon linking X₁ and X₂, can be optionally replaced by O or S heteroatom, and b¹, X₁, X₂, and R₅ are as defined in claim 1; or;
T¹ is (a) or (b) wherein
R₃ is 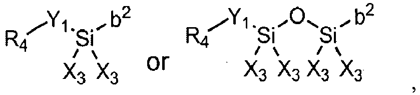
wherein R₄ is linear C₁₈-C₃₂ alkyl, branched C₁₈-C₃₂ alkyl, in both cases the chain optionally containing one S, SO, SO₂, -S-S-, O, or Si(R^{a})₂, wherein R^{a} is C₁-C₆ alkyl, at any position in the carbon chain with the proviso that the heteroatom is not in the alpha or omega position position of the carbon chain, or linear or branched C₁₈-C₃₂ alkenyl containing one double bond or linear or branched C₂₁-C₃₂ alkenyl containing more double bonds with the proviso that there is at least one -CH₂- group between the double bond and Y₁,or
R₄ is linear C₁-C₃₂ alkyl or branched C₁-C₃₂ alkyl, in both cases the chain containing one -S-S- at any position in the carbon chain with the proviso that the heteroatom is not in the alpha or omega position of the carbon chain, or branched C₈-C₃₂ alkenyl containing more double bonds with the proviso that there is at least one -CH₂- group between the double bond and Y₁ or
R₄ is 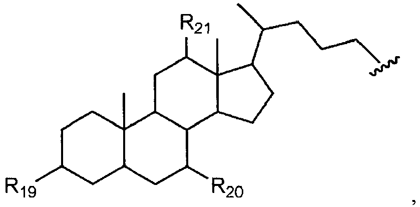 wherein R₁₉. R₂₀, R₂₁ are the same or different and each is independently H, OH, -C₁-C₆ alkoxy, -O-C(O)-C₁-C₆ alkyl or fluorine, wherein at least one of R₁₉, R₂₀, R₂₁ is -O-C(O)-C₁-C₆ alkyl; or
R₄ is CH₃-(CH₂)ᵤ-V₅-(CH₂)ₛ-, wherein u is an integer selected from 1 to 20, s is an integer selected from 1 to 20, and the sum of u+s is an integer selected from 2 to 29, and V₅ is a C₃-C₆ carbocycle, or
R₄ is V₆-(CH₂)ᵢ-, wherein i is an integer selected from 1 to 30, and V₆ is a C₃-C₆ carbocycle, or
R₄ is 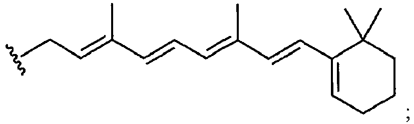 Y₁, X₃ and b₂ are as defined in claim 1; and
b¹, X₁, X₂, and R₁, are as defined in claim 1;
and X₆ is selected from Cl, Br, 1, OMs, OTs, OTf.

13. Compound of claim 12 which has any of the following structures:
| Structure | IUPAC name |
|---|---|
| 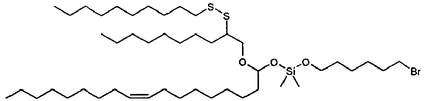 | (Z)-1-bromo-10-(heptadec-8-en-1-yl)-8,8-dimethyl-13-octyl-7,9,11-trioxa-14,15-dithia-8-silapentacosane |
| 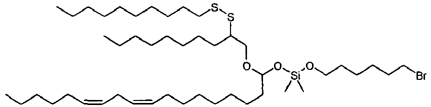 | 1-bromo-10-((8Z,11Z)-heptadeca-8,11-dien-1-yl)-8,8-dimethyl-13-octyl-7,9,11-trioxa-14,15-dithia-8-silapentacosane |
| 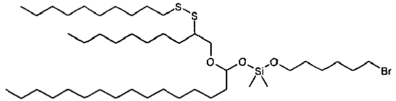 | 1-bromo-8,8-dimethyl-13-octyl-10-pentadecyl-7,9,11-trioxa-14,15-dithia-8-silapentacosane |
| 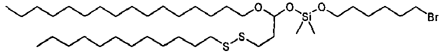 | 1-bromo-10-(hexadecyloxy)-8,8-dimethyl-7,9-dioxa-13,14-dithia-8-silahexacosane |
| 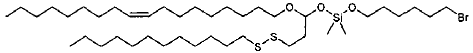 | (Z)-1-bromo-8,8-dimethyl-10-(octadec-9-en-1-yloxy)-7,9-dioxa-13,14-dithia-8-silahexacosane |
| 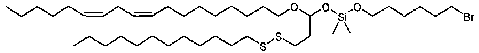 | 1-bromo-8,8-dimethyl-10-(((9Z,12Z)-octadeca-9,12-dien-1-yl)oxy)-7,9-dioxa-13,14-dithia-8-silahexacosane |
| 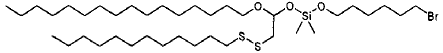 | 1-bromo-10-(hexadecyloxy)-8,8-dimethyl-7,9-dioxa-12,13-dithia-8-silapentacosane |
| 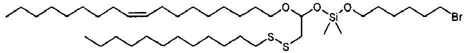 | (Z)-1-bromo-8,8-dimethyl-10-(octadec-9-en-1-yloxy)-7,9-dioxa-12,13-dithia-8-silapentacosane |
| 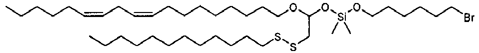 | 1-bromo-8,8-dimethyl-10-(((9Z,12Z)-octadeca-9,12-dien-1-yl)oxy)-7,9-dioxa-12,13-dithia-8-silapentacosane |
| 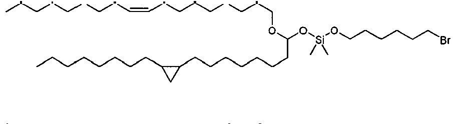 | (Z)-1-bromo-8,8-dimethyl-10-(7-(2-octylcyclopropyl)heptyl)-7,9,11-trioxa-8-silanonacos-20-ene |
| 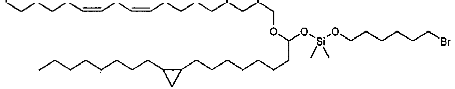 | (20Z,23Z)-1-bromo-8,8-dimethyl-10-(7-(2-octylcyclopropyl)heptyl)-7,9,11-trioxa-8-silanonacosa-20,23-diene |
| 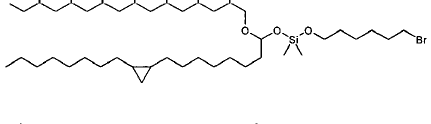 | 1-bromo-8,8-dimethyl-10-(7-(2-octylcyclopropyl)heptyl)-7,9,11-trioxa-8-silaheptacosane |
| 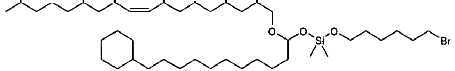 | (Z)-1-bromo-10-(10-cyclohexyldecyl)-8,8-dimethyl-7,9,11-trioxa-8-silanonacos-20-ene |
| 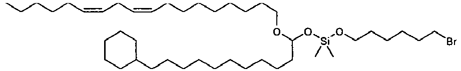 | (20Z,23Z)-1-bromo-10-(10-cyclohexyldecyl)-8,8-dimethyl-7,9,11-trioxa-8-silanonacosa-20,23-diene |
| 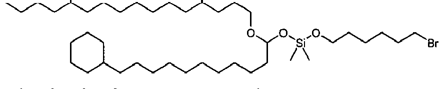 | 1-bromo-10-(10-cyclohexyldecyl)-8,8-dimethyl-7,9,11-trioxa-8-silaheptacosane |
| 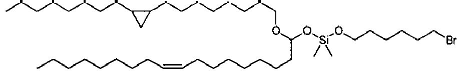 | (Z)-1-bromo-10-(heptadec-8-en-1-yl)-8,8-dimethyl-19-(2-octylcyclopropyl)-7,9,11-trioxa-8-silanonadecane |
| 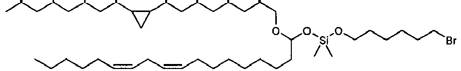 | 1-bromo-10-((8Z,11Z)-heptadeca-8,11-dien-1-yl)-8,8-dimethyl-19-(2-octylcyclopropyl)-7,9,11-trioxa-8-silanonadecane |
| 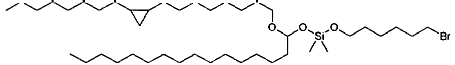 | 1-bromo-8,8-dimethyl-19-(2-octylcyclopropyl)-10-pentadecyl-7,9,11-trioxa-8-silanonadecane |
| 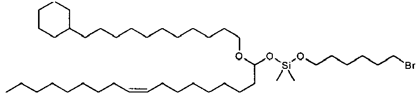 | (Z)-1-bromo-22-cyclohexyl-10-(heptadec-8-en-1-yl)-8,8-dimethyl-7,9,11-trioxa-8-siladocosane |
| 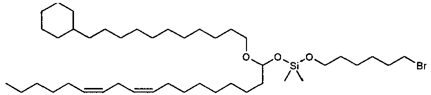 | 1-bromo-22-cyclohexyl-10-((8Z,11Z)-heptadeca-8,11-dien-1-yl)-8,8-dimethyl-7,9,11-trioxa-8-siladocosane |
| 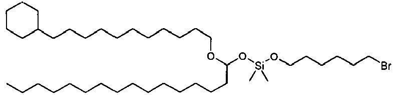 | 1-bromo-22-cyclohexyl-8,8-dimethyl-10-pentadecyl-7,9,11-trioxa-8-siladocosane |
| 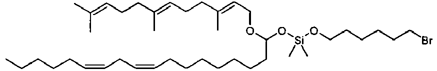 | (13E,17E)-1-bromo-10-((8Z,11Z)-heptadeca-8,11-dien-1-yl)-8,8,14,18,22-pentamethyl-7,9,11-trioxa-8-silatricosa-13,17,21-triene |
| 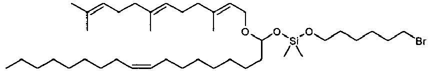 | (13E,17E)-1-bromo-10-((Z)-heptadec-8-en-1-yl)-8,8,14,18,22-pentamethyl-7,9,11-trioxa-8-silatricosa-13,17,21-triene |
| 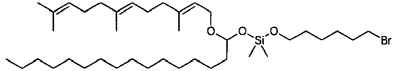 | (13E,17E)-1-bromo-8,8,14,18,22-pentamethyl-10-pentadecyl-7,9,11-trioxa-8-silatricosa-13,17,21-triene |
| 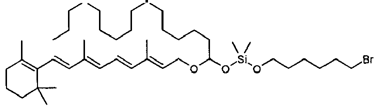 | (13E,15E,17E,19E)-1-bromo-8,8,14,18-tetramethyl-10-pentadecyl-20-(2,6,6-trimethylcyclohex-1-en-1-yl)-7,9,11-trioxa-8-silaicosa-13,15,17,19-tetraene |
| 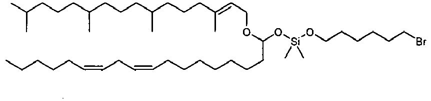 | (E)-1-bromo-10-((8Z,11Z)-heptadeca-8,11-dien-1-yl)-8,8,14,18,22,26-hexamethyl-7,9,11-trioxa-8-silaheptacos-13-ene |
| 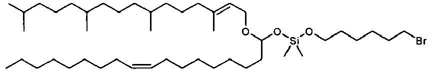 | (E)-1-bromo-10-((Z)-heptadec-8-en-1-yl)-8,8,14,18,22,26-hexamethyl-7,9,11-trioxa-8-silaheptacos-13-ene |
| 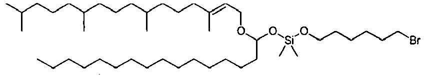 | (E)-1-bromo-8,8,14,18,22,26-hexamethyl-10-pentadecyl-7,9,11-trioxa-8-silaheptacos-13-ene |
| 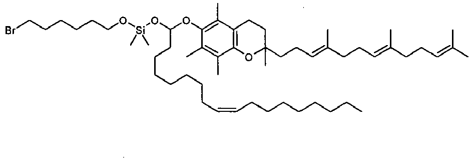 | ((6-bromohexyl)oxy)dimethyl(((Z)-1-((2,5,7,8-tetramethyl-2-((3E,7E)-4,8,12-trimethyltrideca-3,7,11-trien-1-yl)chroman-6-yl)oxy)octadec-9-en-1-yl)oxy)silane |
| 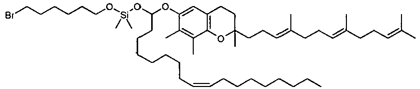 | ((6-bromohexyl)oxy)dimethyl(((Z)-1-((2,7,8-trimethyl-2-((3E,7E)-4,8,12-trimethyltrideca-3,7,11-trien-1-yl)chroman-6-yl)oxy)octadec-9-en-1-yl)oxy)silane |
| 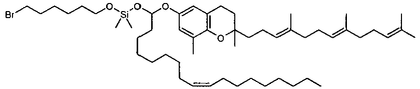 | ((6-bromohexyl)oxy)(((Z)-1-((2,8-dimethyl-2-((3E,7E)-4,8,12-trimethyltrideca-3,7,11-trien-1-yl)chroman-6-yl)oxy)octadec-9-en-1-yl)oxy)dimethylsilane |
| 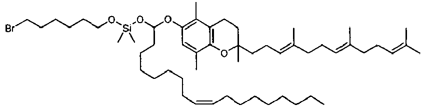 | ((6-bromohexyl)oxy)dimethyl(((Z)-1-((2,5,8-trimethyl-2-((3E,7E)-4,8,12-trimethyltrideca-3,7,11-trien-1-yl)chroman-6-yl)oxy)octadec-9-en-1-yl)oxy)silane |
| 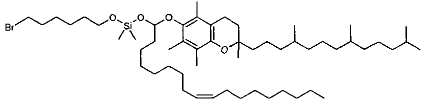 | (Z)-((6-bromohexyl)oxy)dimethyl((1-((2,5,7,8-tetramethyl-2-(4,8,12-trimethyltridecyl)chroman-6-yl)oxy)octadec-9-en-1-yl)oxy)silane |
| 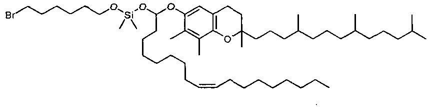 | (Z)-((6-bromohexyl)oxy)dimethyl((1-((2,7,8-trimethyl-2-(4,8,12-trimethyltridecyl)chroman-6-yl)oxy)octadec-9-en-1-yl)oxy)silane |
| 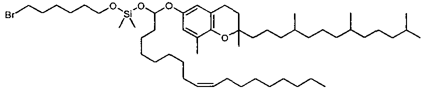 | (Z)-((6-bromohexyl)oxy)((1-((2,8-dimethyl-2-(4,8,12-trimethyltridecyl)chroman-6-yl)oxy)octadec-9-en-1-yl)oxy)dimethylsilane |
| 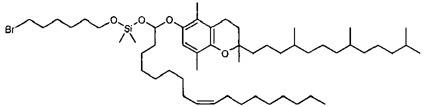 | (Z)-((6-bromohexyl)oxy)dimethyl((1-((2,5,8-trimethyl-2-(4,8,12-trimethyltridecyl)chroman-6-yl)oxy)octadec-9-en-1-yl)oxy)silane |
| 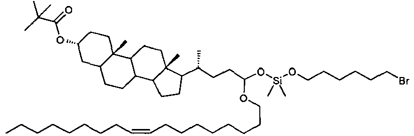 | (3R,10S,13R)-17-((2R)-5-((((6-bromohexyl)oxy)dimethylsilyl)oxy)-5-(((Z)-octadec-9-en-1-yl)oxy)pentan-2-yl)-10,13-dimethylhexadecahydro-1H-cyclopenta[a]phenanthren-3-yl pivalate |
| 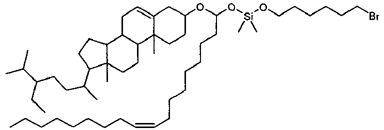 | (Z)-((6-bromohexyl)oxy)((1-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)octadec-9-en-1-yl)oxy)dimethylsilane |
| 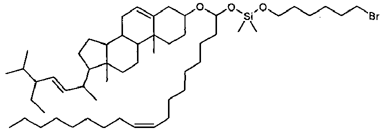 | ((6-bromohexyl)oxy)(((Z)-1-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)octadec-9-en-1-yl)oxy)dimethylsilane |
| 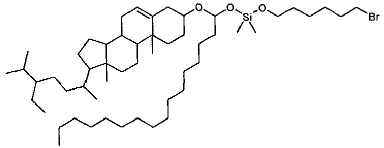 | ((6-bromohexyl)oxy)((1-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)hexadecyl)oxy)dimethylsilane |
| 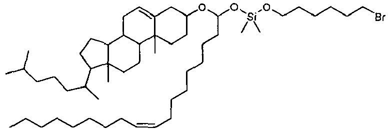 | (Z)-((6-bromohexyl)oxy)((1-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)octadec-9-en-1-yl)oxy)dimethylsilane |
| 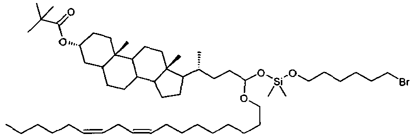 | (3R,10S,13R)-17-((2R)-5-((((6-bromohexyl)oxy)dimethylsilyl)oxy)-5-(((9Z,12Z)-octadeca-9,12-dien-1-yl)oxy)pentan-2-yl)-10,13-dimethylhexadecahydro-1H-cyclopenta[a]phenanthren-3-yl pivalate |
| 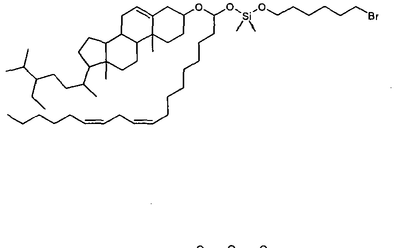 | ((6-bromohexyl)oxy)(((9Z,12Z)-1-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)octadeca-9,12-dien-1-yl)oxy)dimethylsilane |
| 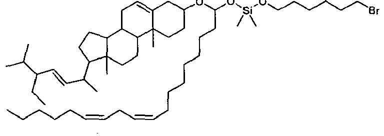 | ((6-bromohexyl)oxy)(((9Z,12Z)-1-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)octadeca-9,12-dien-1- yl)oxy)dimethylsilane |
| 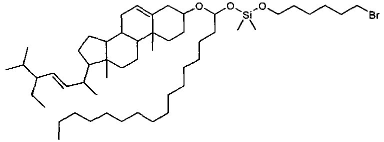 | (E)-((6-bromohexyl)oxy)((1-((17-(5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)hexadecyl)oxy)dimethylsilane |
| 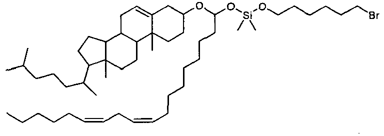 | ((6-bromohexyl)oxy)(((9Z,12Z)-1-((10,13-dimethyl-17-(6-methylheptan-2-yl)-2,3,4,7,8,9,10,11,12,13,14,15,16,17-tetradecahydro-1H-cyclopenta[a]phenanthren-3 -y l)oxy)octadeca-9,12-dien-1-yl)oxy)dimethylsilane |
| 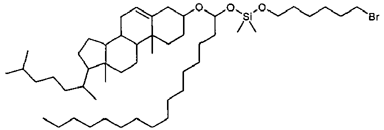 | ((6-bromohexyl)oxy)((1-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)hexadecyl)oxy)dimethylsilane |
| 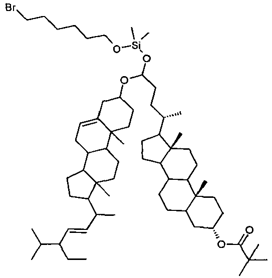 | (3S,10R,13S)-17-((2S)-5-((((6-bromohexyl)oxy)dimethylsilyl)oxy)-5-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)pentan-2-yl)-10,13-dimethylhexadecahydro-1H-cyclopenta[a]phenanthren-3-yl pivalate |
| 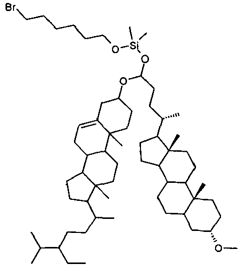 | ((6-bromohexyl)oxy)(((4S)-1-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)-4-((3S,10R,13S)-3-methoxy-10,13-dimethylhexadecahydro-1H-cyclopenta[a]phenanthren-17-yl)pentyl)oxy)dimethylsilane |
| 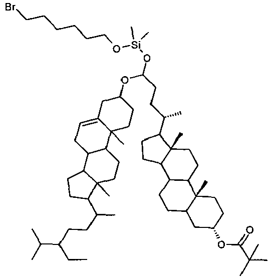 | (3S,10R,13S)-17-((2S)-5-((((6-bromohexyl)oxy)dimethylsilyl)oxy)-5-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)pentan-2-yl)-10,13-dimethylhexadecahydro-1H-cyclopenta[a]phenanthren-3-ylpivalate |
| 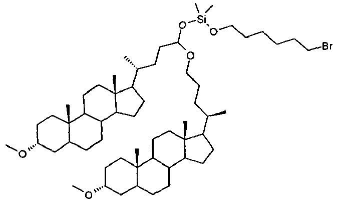 | (2R)-16-bromo-2-((3R,10S,13R)-3-methoxy-10,13-dimethylhexadecahydro-1H-cyclopenta[a]phenanthren-17-yl)-7-((3R)-3-((3R,10S,13R)-3-methoxy- 10,13-dimethylhexadecahydro-1H-cyclopenta[a]phenanthren-17-yl)butyl)-9,9-dimethyl-6,8,10-trioxa-9-silahexadecane |
| 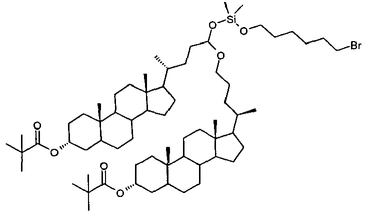 | (3R,10S,13R)-17-((2R)-16-bromo-7-((3R)-3-((3R,10S,13R)-10,13-dimethyl-3-(pivaloyloxy)hexadecahydro-1H-cyclopenta[a]phenanthren-17-yl)butyl)-9,9-dimethyl-6,8,10-trioxa-9-silahexadecan-2-yl)-10,13-dimethylhexadecahydro-1H-cyclopenta[a]phenanthren-3-yl pivalate |
| 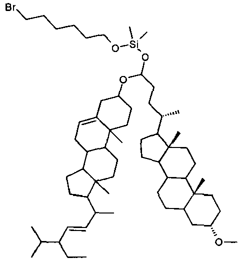 | ((6-bromohexyl)oxy)(((4S)-1-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)-4-((3S,10R,13S)-3-methoxy-10,13-dimethylhexadecahydro-1H-cyclopenta[a]phenanthren-17-yl)pentyl)oxy)dimethylsilane |

14. Compound having any of the following structure (III), 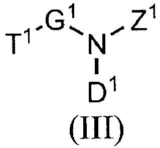
G¹ and D¹ are as defined in claim 1 and T¹ is as defined in claim 13;
or
G¹, T¹ are as defined in claim 1 and D¹ is linear C₁-C₈ alkyl, branched C₃-C₈ alkyl.
and
Z¹ is 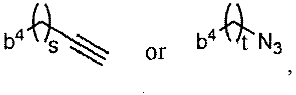 wherein b⁴ is a bond to N
s is an integer from 1 to 6
t is an integer from 2 to 6.

15. A compound of claim 14, which has any of the following structures:
| Structure | IUPAC name |
|---|---|
| 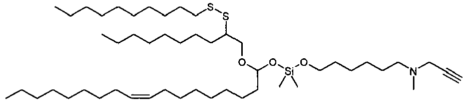 | (Z)-10-(heptadec-8-en-1-yl)-N, 8,8-trimethyl-13-octyl-N-(prop-2-yn-1-yl)-7,9,11-trioxa-14,15-dithia-8-silapentacosan-1-amine |
| 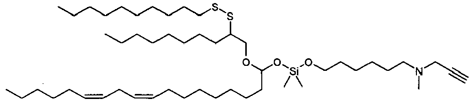 | 10-((8Z,11Z)-heptadeca-8,11-dien-1-yl)-N,8,8-trimethyl-13-octyl-N-(prop-2-yn-1-yl)-7,9,11-trioxa-14,15-dithia-8-silapentacosan-1-amine |
| 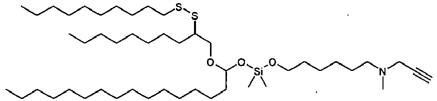 | N,8,8-trimethyl-13-octyl-10-pentadecyl-N-(prop-2-yn-1-yl)-7,9,11-trioxa-14,15-dithia-8-silapentacosan-1-amine |
| 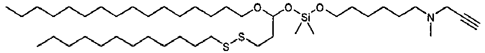 | 10-(hexadecyloxy)-N,8,8-trimethyl-N-(prop-2-yn-1-yl)-7,9-dioxa-13,14-dithia-8-silahexacosan-1-amine |
| 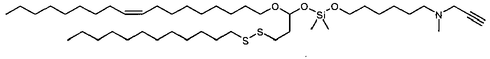 | (Z)-N,8,8-trimethyl-10-(octadec-9-en-1-yloxy)-N-(prop-2-yn-1-yl)-7,9-dioxa-13,14-dithia-8-silahexacosan-1-amine |
| 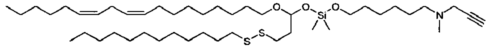 | N,8,8-trimethyl-10-(((9Z,12Z)-octadeca-9,12-dien-1-yl)oxy)-N-(prop-2-yn-1-yl)-7,9-dioxa-13,14-dithia-8-silahexacosan-1-amine |
| 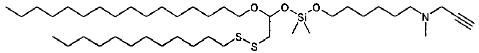 | 10-(hexadecyloxy)-N,8,8-trimethyl-N-(prop-2-yn-1-yl)-7,9-dioxa-12,13-dithia-8-silapentacosan-1-amine |
| 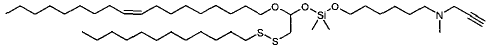 | (Z)-N,8,8-trimethyl-10-(octadec-9-en-1-yloxy)-N-(prop-2-yn-1-yl)-7,9-dioxa-12,13-dithia-8-silapentacosan-1-amine |
| 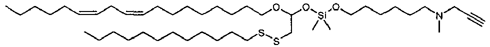 | N,8,8-trimethyl-10-(((9Z,12Z)-octadeca-9,12-dien-1-yl)oxy)-N-(prop-2-yn-1-yl)-7,9-dioxa-12,13-dithia-8-silapentacosan-1-amine |
| 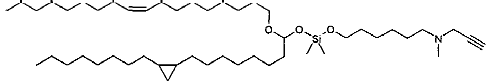 | (Z)-N,8,8-trimethyl-10-(7-(2-octylcyclopropyl)heptyl)-N-(prop-2-yn-1-yl)-7,9,11-trioxa-8-silanonacos-20-en-1-amine |
| 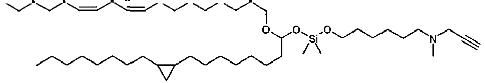 | (20Z,23Z)-N,8,8-trimethyl-10-(7-(2-octylcyclopropyl)heptyl)-N-(prop-2-yn-1-yl)-7,9,11-trioxa-8-silanonacosa-20,23-dien-1-amine |
| 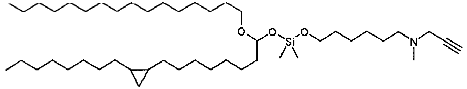 | N,8,8-trimethyl-10-(7-(2-octylcyclopropyl)heptyl)-N-(prop-2-yn-1-yl)-7,9,11-trioxa-8-silaheptacosan-1-amine |
| 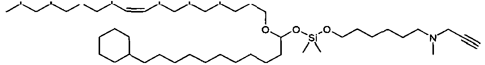 | (Z)-10-(10-cyclohexyldecyl)-N,8,8-trimethyl-N-(prop-2-yn-1-yl)-7,9,11-trioxa-8-silanonacos-20-en-1-amine |
| 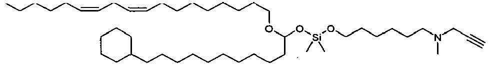 | (20Z,23Z)-10-(10-cyclohexyldecyl)-N,8,8-trimethyl-N-(prop-2-yn-1-yl)-7,9,11-trioxa-8-silanonacosa-20,23-dien-1-amine |
| 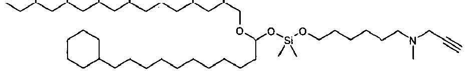 | 10-(10-cyclohexyldecyl)-N,8,8-trimethyl-N-(prop-2-yn-1-yl)-7,9,11-trioxa-8-silaheptacosan-1-amine |
| 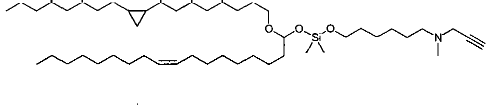 | (Z)-10-(heptadec-8-en-1-yl)-N,8,8-trimethyl-19-(2-octylcyclopropyl)-N-(prop-2-yn-1-yl)-7,9,11-trioxa-8-silanonadecan-1-amine |
| 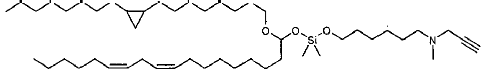 | 10-((8Z,11Z)-heptadeca-8,11-dien-1-yl)-N,8,8-trimethyl-19-(2-octylcyclopropyl)-N-(prop-2-yn-1-yl)-7,9,11-trioxa-8-silanonadecan-1-amine |
| 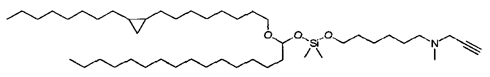 | N,8,8-trimethyl-19-(2-octylcyclopropyl)-10-pentadecyl-N-(prop-2-yn-1-yl)-7,9,11 - trioxa-8-silanonadecan-1-amine |
| 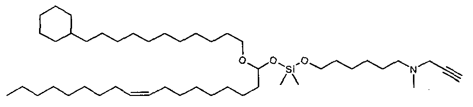 | (Z)-22-cyclohexyl-10-(heptadec-8-en-1-yl)-N,8,8-trimethyl-N-(prop-2-yn-1-yl)-7,9,11-trioxa-8-siladocosan-1-amine |
| 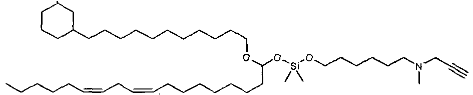 | 22-cyclohexyl-10-((8Z,11Z)-heptadeca-8,11-dien-1-yl)-N,8,8-trimethyl-N-(prop-2-yn-1-yl)-7,9,11-trioxa-8-siladocosan-1-amine |
| 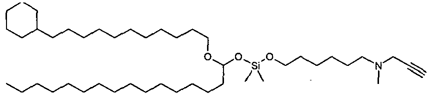 | 22-cyclohexyl-N,8,8-trimethyl-10-pentadecyl-N-(prop-2-yn--yl)-7,9,11-trioxa-8-siladocosan-1-amine |
| 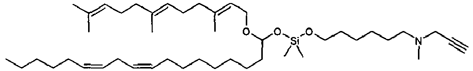 | (13E,17E)-10-((8Z,11Z)-heptadeca-8,11 - dien-1-yl)-N,8,8,14,18,22-hexamethyl-N-(prop-2-yn-1-yl)-7,9,11-trioxa-8-silatricosa-13,17,21-trien-1-amine |
| 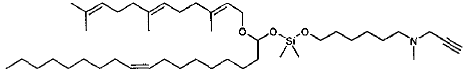 | (13E,17E)-10-((Z)-heptadec-8-en-1-yl)-N,8,8,14,18,22-hexamethyl-N-(prop-2-yn-1-yl)-7,9,11-trioxa-8-silatricosa-13,17,21-trien-1-amine |
| 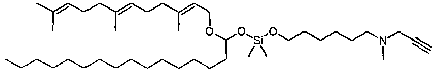 | (13E,17E)-N,8,8,14,18,22-hexamethyl-10-pentadecyl-N-(prop-2-yn-1-yl)-7,9,11- trioxa-8-silatricosa-13,17,21-trien-1-amine |
| 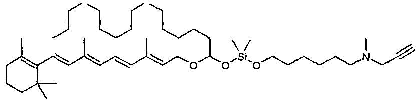 | (13E,15E,17E,19E)-N,8,8,14,18-pentamethyl-10-pentadecyl-N-(prop-2-yn-1-yl)-20-(2,6,6-trimethylcyclohex-1-en-1-yl)-7,9,11-trioxa-8-silaicosa-13,15,17,19-tetraen-1-amine |
| 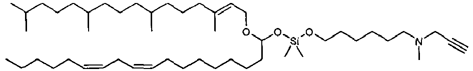 | (E)-10-((8Z,111Z)-heptadeca-8,11-dien-1-yl)-N,8,8,14,18,22,26-heptamethyl-N-(prop-2-yn-1-yl)-7,9,11-trioxa-8-silaheptacos-13-en-l-amine |
| 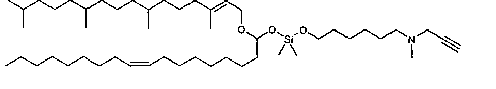 | (E)-10-((Z)-heptadec-8-en-1-yl)-N,8,8,14,18,22,26-heptamethyl-N-(prop-2-yn-1-yl)-7,9,11-trioxa-8-silaheptacos-13-en-1-amine |
| 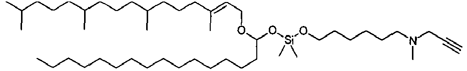 | (E)-N,8,8,14,18,22,26-heptamethyl-10-pentadecyl-N-(prop-2-yn-1-yl)-7,9,11-trioxa-8-silaheptacos-13-en-1-amine |
| 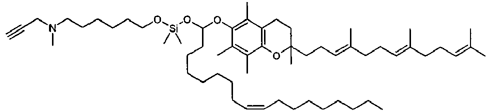 | 6-((dimethyl(((Z)-1-((2,5,7,8-tetramethyl-2-((3E,7E)-4,8,12-trimethyltrideca-3,7,11- trien-1-yl)chroman-6-yl)oxy)octadec-9-en-1-yl)oxy)silyl)oxy)-N-methyl-N-(prop-2-yn-1-yl)hexan-1-amine |
| 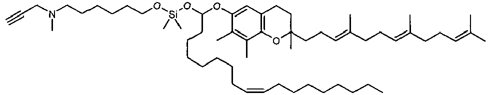 | 6-((dimethyl(((Z)-1-((2,7,8-trimethyl-2-((3E,7E)-4,8,12-trimethyltrideca-3,7,11-trien-1-yl)chroman-6-yl)oxy)octadec-9-en-1-yl)oxy)silyl)oxy)-N-methyl-N-(prop-2-yn-1-yl)hexan-1-amine |
| 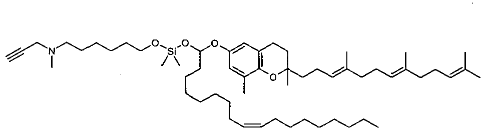 | 6-(((((Z)-1-((2,8-dimethyl-2-((3E,7E)-4,8,12-trimethyltrideca-3,7,11-trien-1-yl)chroman-6-yl)oxy)octadec-9-en-1-yl)oxy)dimethylsilyl)oxy)-N-methyl-N-(prop-2-yn-1-yl)hexan-1-amine |
| 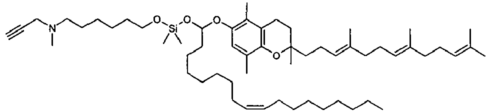 | 6-((dimethyl(((Z)-1-((2,5,8-trimethyl-2-((3E,7E)-4,8,12-trimethyltrideca-3,7,11-trien-1-yl)chroman-6-yl)oxy)octadec-9-en-1-yl)oxy)silyl)oxy)-N-methyl-N-(prop-2-yn-1-yl)hexan-1-amine |
| 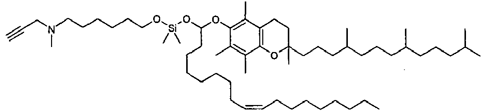 | (Z)-6-((dimethyl((1-((2,5,7,8-tetramethyl-2-(4,8,12-trimethyltridecyl)chroman-6-yl)oxy)octadec-9-en-1-yl)oxy)silyl)oxy)-N-methyl-N-(prop-2-yn-1-yl)hexan-1-amine |
| 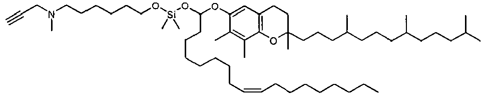 | (Z)-6-((dimethyl((1-((2,7,8-trimethyl-2-(4,8,12-trimethyltridecyl)chroman-6-yl)oxy)octadec-9-en-1-yl)oxy)silyl)oxy)-N-methyl-N-(prop-2-yn-1-yl)hexan-1-amine |
| 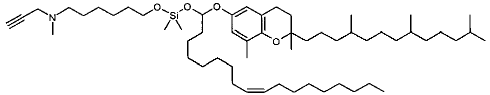 | (Z)-6-((((1-((2,8-dimethyl-2-(4,8,12-trimethyltridecyl)chroman-6-yl)oxy)octadec-9-en-1-yl)oxy)dimethylsilyl)oxy)-N-methyl-N-(prop-2-yn-1-yl)hexan-1-amine |
| 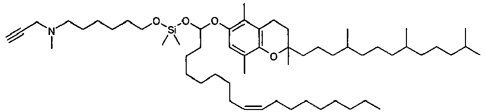 | (Z)-6-((dimethyl((1-((2,5,8-trimethyl-2-(4,8,12-trimethyltridecyl)chroman-6-yl)oxy)octadec-9-en-1-yl)oxy)silyl)oxy)-N-methyl-N-(prop-2-yn-1-yl)hexan-1-amine |
| 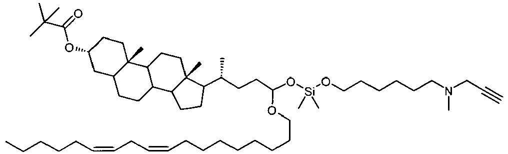 | (3R,10S,13R)-10,13-dimethyl-17-((17R)-4,12,12-trimethyl-14-(((9Z,12Z)-octadeca-9,12-dien-1-yl)oxy)-11,13-dioxa-4-aza-12-silaoctadec-1-yn-17-yl)hexadecahydro-1H-cyclopenta[a]phenanthren-3-yl pivalate |
| 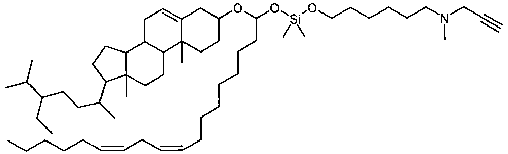 | 6-(((((9Z,12Z)-1-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)octadeca-9,12-dien-1-yl)oxy)dimethylsilyl)oxy)-N-methyl-N-(prop-2-yn-1-yl)hexan-1-amine |
| 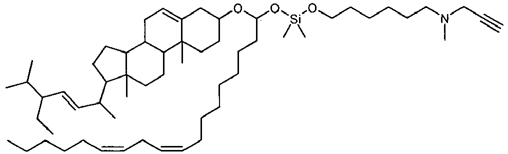 | 6-(((((9Z,12Z)-1-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)octadeca-9,12-dien-1-yl)oxy)dimethylsilyl)oxy)-N-methyl-N-(prop-2-yn-1-yl)hexan-1-amine |
| 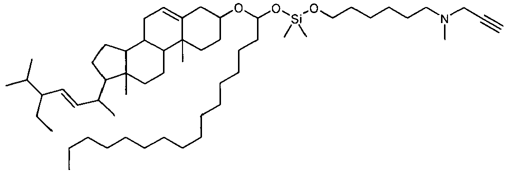 | (E)-6-((((l-((17-(5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)hexadecyl)oxy)dimethylsilyl)oxy)-N-methyl-N-(prop-2-yn-1-yl)hexan-1-amine |
| 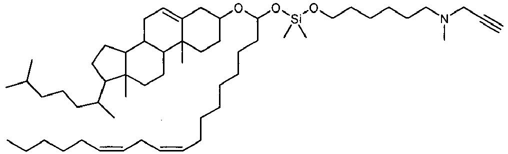 | 6-(((((9Z,12Z)-1-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)octadeca-9,12-dien-1-yl)oxy)dimethylsilyl)oxy)-N-methyl-N-(prop-2-yn-1-yl)hexan-1-amine |
| 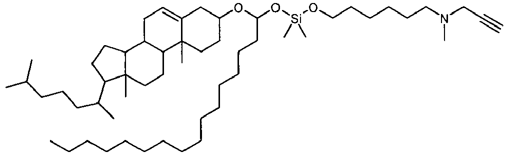 | 6-((((1-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)hexadecyl)oxy)dimethylsilyl)oxy)-N-methyl-N-(prop-2-yn-1-yl)hexan-1-amine |
| 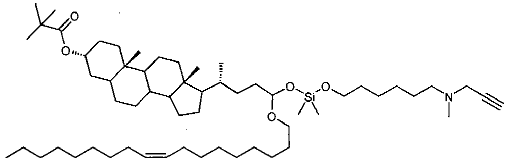 | (3R,10S,13R)-10,13-dimethyl-17-((17R)-4,12,12-trimethyl-14-(((Z)-octadec-9-en-1-yl)oxy)-11,13-dioxa-4-aza-12-silaoctadec-1-yn-17-yl)hexadecahydro-1H-cyclopenta[a]phenanthren-3-yl pivalate |
| 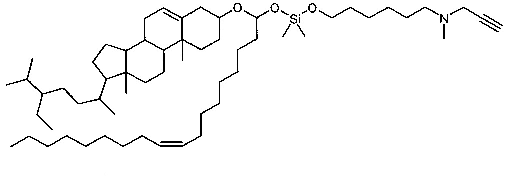 | (Z)-6-((((1-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)octadec-9-en-1-yl)oxy)dimethylsilyl)oxy)-N-methyl-N-(prop-2-yn-1-yl)hexan-1-amine |
| 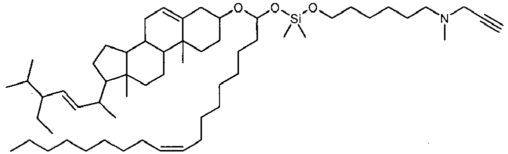 | 6-((Z)-1-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)octadec-9-en-1- yl)oxy)dimethylsilyl)oxy)-N-methyl-N-(prop-2-yn-1-yl)hexan-1-amine |
| 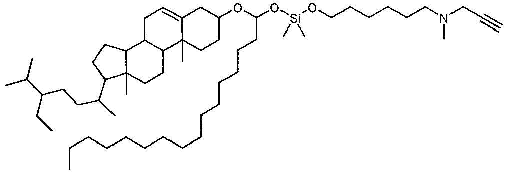 | 6-((((1-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)hexadecyl)oxy)dimethylsilyl)oxy)-N-methyl-N-(prop-2-yn-1-yl)hexan-1-amine |
| 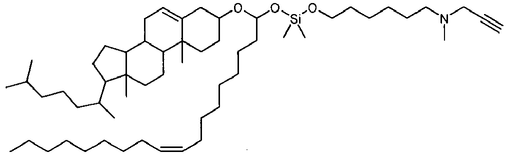 | (Z)-6-((((1-((10,13-dimethyl-17-(6-methylheptan-2-yl)-2,3,4,7,8,9,10,11,12,13,14,15,16,17-tetradecahydro-1H-cyclopenta[a]phenanthren-3-yl)oxy)octadec-9-en-1-yl)oxy)dimethylsilyl)oxy)-N-methyl-N-(prop-2-yn-1-yl)hexan-1-amine |
| 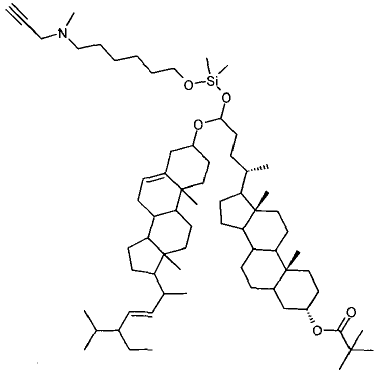 | (3S,10R,13S)-17-((17S)-14-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)-4,12,12-trimethyl-11,13-dioxa-4-aza-12-silaoctadec-1-yn-17-yl)-10,13-dimethylhexadecahydro-1H-cyclopenta[a]phenanthren-3-yl pivalate |
| 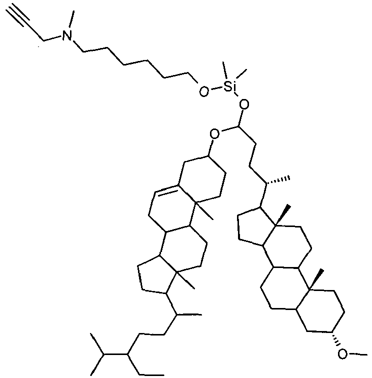 | 6-(((((4S)-1-((17-(5-ethyl-6-methylheptan-2-yl)-10,13-dimethyl-2,3,4,7,8,9,10,11,12,13,14,15,16,17-tetradecahydro- 1H-cyclopenta[a]phenanthren-3-yl)oxy)-4-((3S,10R,13S)-3-methoxy-10,13-dimethylhexadecahydro-1H-cyclopenta[a]phenanthren-17-yl)pentyl)oxy)dimethylsilyl)oxy)-N-methyl-N-(prop-2-yn-1-yl)hexan-1-amine |
| 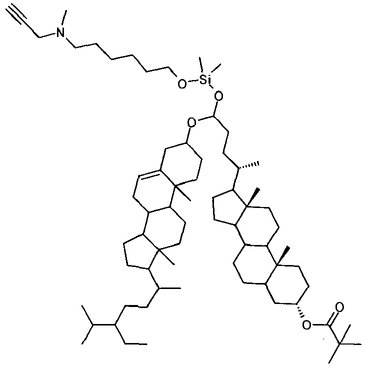 | (3S,10R,13S)-17-((17S)-14-((17-(5-ethyl-6-methylheptan-2-yl)-10,13-dimethyl-2,3,4,7,8,9,10,11,12,13,14,15,16,17-tetradecahydro-1H-cyclopenta[a]phenanthren-3-yl)oxy)-4,12,12-trimethyl-11,13-dioxa-4-aza-12-silaoctadec-1-yn-17-yl)-10,13-dimethylhexadecahydro-1H-cyclopenta[a]phenanthren-3-yl pivalate |
| 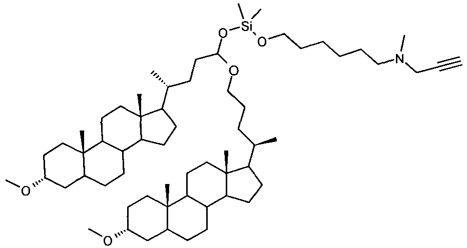 | (2R)-2-((3R,10S,13R)-3-methoxy-10,13- dimethylhexadecahydro-1H-cyclopenta[a]phenanthren-17-yl)-7-((3R)-3-((3R,10S,13R)-3-methoxy-10,13-dimethylhexadecahydro-1H-cyclopenta[a]phenanthren-17-yl)butyl)-N,9,9-trimethyl-N-(prop-2-yn-l-yl)-6,8,10-trioxa-9-silahexadecan-16-amine |
| 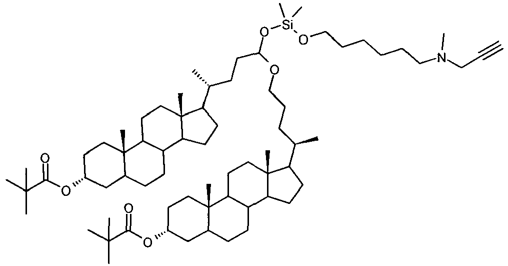 | (3R,10S,13R)-17-((19R)-14-((3R)-3-((3R,10S,13R)-10,13-dimethyl-3-(pivaloyloxy)hexadecahydro-1H-cyclopenta[a]phenanthren-17-yl)butyl)-4,12,12-trimethyl-11,13,15-trioxa-4-aza-12-silaicos-1-yn-19-yl)-10,13-dimethylhexadecahydro-1H-cyclopenta[a]phenanthren-3-yl pivalate |
| 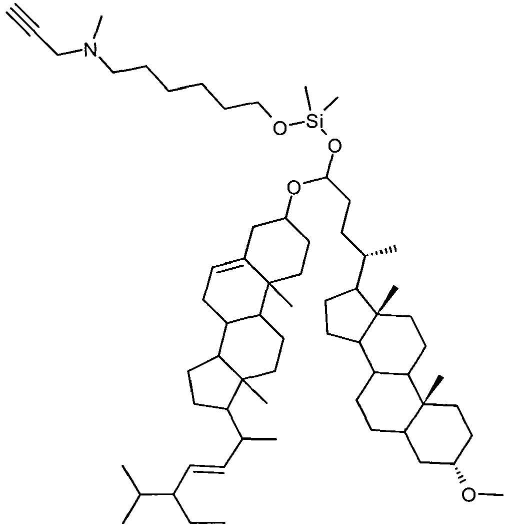 | 6-(((((4S)-1-((17-((E)-5-ethyl-6-methylhept-3-en-2-yl)-10,13-dimethyl-2,3,4,7,8,9,10,11,12,13,14,15,16,17-tetradecahydro-1H-cyclopenta[a]phenanthren-3-yl)oxy)-4-((3S,10R,13S)-3-methoxy-10,13-dimethylhexadecahydro-1H-cyclopenta[a]phenanthren-17-yl)pentyl)oxy)dimethylsilyl)oxy)-N-methyl-N-(prop-2-yn-1-yl)hexan-1-amine |
